(19) Europäisches Patentamt
European Patent Office
Office européen des brevets

(11) EP 1 346 041 B1

(12) # EUROPEAN PATENT SPECIFICATION

(45) Date of publication and mention of the grant of the patent:
**28.02.2007 Bulletin 2007/09**

(21) Application number: **01995259.7**

(22) Date of filing: **27.11.2001**

(51) Int Cl.:
*C12N 15/12* (2006.01) *C12N 15/13* (2006.01)
*C12N 15/85* (2006.01) *C12N 5/10* (2006.01)
*C07K 14/00* (2006.01) *A61K 38/17* (2006.01)
*C07K 16/00* (2006.01)

(86) International application number:
**PCT/US2001/044581**

(87) International publication number:
**WO 2002/042462 (30.05.2002 Gazette 2002/22)**

(54) **THERAPEUTIC AGENTS AND METHODS OF USE THEREOF FOR TREATING AN AMYLOIDOGENIC DISEASE**

THERAPEUTISCHE AGENTIEN UND METHODEN IHRER VERWENDUNG ZUR BEHANDLUNG VON AMYLOIDOGENEN ERKRANKUNGEN

AGENTS THERAPEUTIQUES ET METHODES D'UTILISATION DE CES AGENTS THERAPEUTIQUES POUR TRAITER UNE MALADIE AMYLOIDOGENIQUE

(84) Designated Contracting States:
**AT BE CH CY DE DK ES FI FR GB GR IE IT LI LU MC NL PT SE TR**

(30) Priority: **27.11.2000 US 253302 P**
**29.11.2000 US 250198 P**
**20.12.2000 US 257186 P**

(43) Date of publication of application:
**24.09.2003 Bulletin 2003/39**

(73) Proprietor: **Praecis Pharmaceuticals Incorporated**
**Waltham, MA 02451 (US)**

(72) Inventors:
- **GEFTER, Malcolm, L.**
**Lincoln, MA 01773 (US)**
- **ISRAEL, David, I.**
**Concord, MA 01742 (US)**
- **JOYAL, John, L.**
**Melrose, MA 02176 (US)**
- **GOSSELIN, Michael**
**Melrose, MA 02176 (US)**

(74) Representative: **Hermann, Bettina Celia et al**
**Frohwitter**
**Patent- und Rechtsanwälte**
**Possartstraße 20**
**81679 München (DE)**

(56) References cited:
**WO-A-01/83526**
**WO-A-93/04194**
**WO-A-96/28471**

- **PALLITTO, MONICA M. ET AL: "Recognition sequence design for peptidyl modulators of.beta.- amyloid aggregation and toxicity" BIOCHEMISTRY (1999), 38(12), 3570-3578 , XP002152181**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 346 041 B1

SEQUENCE LISTING

<110> Praecis Pharmaceuticals, Inc

<120> THERAPEUTIC AGENTS AND METHODS OF USE THEREOF FOR TREATING AN AMYLOIDOGENIC DISEASE

<130> PPI-105PC

<140>
<141>

<150> 60/253,302
<151> 2000-11-27

<150> 60/250,198
<151> 2000-11-29

<150> 60/257,186
<151> 2000-12-20

<160> 13

<170> PatentIn Ver. 2.0

<210> 1
<211> 43
<212> PRT
<213> Homo sapiens

<400> 1

```
Asp Ala Glu Phe Arg His Asp Ser Gly Tyr Glu Val His His Gln Lys
  1               5                  10                  15

Leu Val Phe Phe Ala Glu Asp Val Gly Ser Asn Lys Gly Ala Ile Ile
             20                  25                  30

Gly Leu Met Val Gly Gly Val Val Ile Ala Thr
         35                  40
```

<210> 2
<211> 4
<212> PRT
<213> Homo sapiens

<400> 2

```
Leu Val Phe Phe
  1
```

<210> 3
<211> 5
<212> PRT
<213> Homo sapiens

<400> 3

Leu Val Phe Phe Ala
1 5

<210> 4
<211> 8
<212> PRT
<213> Homo sapiens

<400> 4

Ala Gly Ala Ala Ala Ala Gly Ala
1 5

<210> 5
<211> 5
<212> PRT
<213> Homo sapiens

<400> 5

Ala Ile Leu Ser Ser
1 5

<210> 6
<211> 36
<212> DNA
<213> Artificial Sequence

<220>
<223> Description of Artificial Sequence:primers

<400> 6
ctggttccgc gtggatccgt gcccagggat tgtggt 36

<210> 7
<211> 33
<212> DNA
<213> Artificial Sequence

<220>
<223> Description of Artificial Sequence:primers

<400> 7
attaagcatt ctagatcatt taccaggaga gtg 33

<210> 8
<211> 22
<212> PRT
<213> Homo sapiens

<400> 8

Met Asp Ala Met Lys Arg Gly Leu Cys Cys Val Leu Leu Leu Cys Gly
1 5 10 15

Ala Val Phe Val Ser Pro
20

<210> 9
<211> 15
<212> PRT
<213> Homo sapiens

<400> 9

Lys Leu Val Phe Phe Ala Glu Asp Val Gly Ser Asn Lys Gly Ala
1 5 10 15

<210> 10
<211> 232
<212> PRT
<213> Homo sapiens

<400> 10

```
Glu Pro Lys Ser Cys Asp Lys Thr His Thr Cys Pro Pro Cys Pro Ala
 1               5                  10                  15

Pro Glu Leu Leu Gly Gly Pro Ser Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Asp Glu Leu Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro Gly Lys
225                 230
```

<210> 11
<211> 804
<212> DNA
<213> Artificial Sequence

<220>
<223> Description of Artificial Sequence:alpha-beta(16-30)Fc

<400> 11

```
atggatgcaa tgaagagagg gctctgctgt gtgctgctgc tgtgtggagc agtcttcgtt 60
aagcttgtat tcttcgcaga agacgtcgga tcgaacaaag gtgccgagcc caaatcttgt 120
gacaaaactc acacatgccc accgtgccca gcacctgaac tcctgggggg accgtcagtc 180
ttcctcttcc ccccaaaacc caaggacacc ctcatgatat cccggacccc tgaggtcaca 240
tgcgtggtgg tggacgtgag ccacgaagac cctgaggtca agttcaactg gtacgtggac 300
ggcgtggagg tgcataatgc caagacaaag ccgcgggagg agcagtacaa cagcacgtac 360
cgggtggtca gcgtcctcac cgtcctgcac caggactggc tgaatggcaa ggagtacaag 420
tgcaaggtct ccaacaaagc cctcccagcc cccatcgaga aaaccatctc caaagccaaa 480
gggcagcccc gagaaccaca ggtgtacacc ctgcccccat cccgggatga gctgaccaag 540
aaccaggtca gcctgacctg cctggtcaaa ggcttctatc ccagcgacat cgccgtggag 600
tgggagagca atgggcagcc ggagaacaac tacaagacca cgcctcccgt gctggactcc 660
gacggctcct tcttcctcta cagcaagctc accgtggaca agagcaggtg gcagcagggg 720
aacgtcttct catgctccgt gatgcatgag gctctgcaca accactacac gcagaagagc 780
ctctccctgt ctccgggtaa atga                                        804
```

<210> 12
<211> 267
<212> PRT
<213> Artificial Sequence

<220>
<223> Description of Artificial Sequence:alpha-beta(16-30)Fc

<400> 12

```
Met Asp Ala Met Lys Arg Gly Leu Cys Cys Val Leu Leu Leu Cys Gly
1               5                   10                  15

Ala Val Phe Val Lys Leu Val Phe Phe Ala Glu Asp Val Gly Ser Asn
            20                  25                  30

Lys Gly Ala Glu Pro Lys Ser Cys Asp Lys Thr His Thr Cys Pro Pro
        35                  40                  45

Cys Pro Ala Pro Glu Leu Leu Gly Gly Pro Ser Val Phe Leu Phe Pro
    50                  55                  60

Pro Lys Pro Lys Asp Thr Leu Met Ile Ser Arg Thr Pro Glu Val Thr
65                  70                  75                  80

Cys Val Val Val Asp Val Ser His Glu Asp Pro Glu Val Lys Phe Asn
                85                  90                  95

Trp Tyr Val Asp Gly Val Glu Val His Asn Ala Lys Thr Lys Pro Arg
            100                 105                 110

Glu Glu Gln Tyr Asn Ser Thr Tyr Arg Val Val Ser Val Leu Thr Val
        115                 120                 125

Leu His Gln Asp Trp Leu Asn Gly Lys Glu Tyr Lys Cys Lys Val Ser
    130                 135                 140

Asn Lys Ala Leu Pro Ala Pro Ile Glu Lys Thr Ile Ser Lys Ala Lys
145                 150                 155                 160

Gly Gln Pro Arg Glu Pro Gln Val Tyr Thr Leu Pro Pro Ser Arg Asp
                165                 170                 175
```

```
Glu Leu Thr Lys Asn Gln Val Ser Leu Thr Cys Leu Val Lys Gly Phe
            180                 185                 190

Tyr Pro Ser Asp Ile Ala Val Glu Trp Glu Ser Asn Gly Gln Pro Glu
        195                 200                 205

Asn Asn Tyr Lys Thr Thr Pro Pro Val Leu Asp Ser Asp Gly Ser Phe
    210                 215                 220

Phe Leu Tyr Ser Lys Leu Thr Val Asp Lys Ser Arg Trp Gln Gln Gly
225                 230                 235                 240

Asn Val Phe Ser Cys Ser Val Met His Glu Ala Leu His Asn His Tyr
                245                 250                 255

Thr Gln Lys Ser Leu Ser Leu Ser Pro Gly Lys
            260                 265
```

<210> 13
<211> 247
<212> PRT
<213> Homo sapiens

<400> 13

```
Lys Leu Val Phe Phe Ala Glu Asp Val Gly Ser Asn Lys Gly Ala Glu
 1               5                  10                  15

Pro Lys Ser Cys Asp Lys Thr His Thr Cys Pro Pro Cys Pro Ala Pro
            20                  25                  30

Glu Leu Leu Gly Gly Pro Ser Val Phe Leu Phe Pro Pro Lys Pro Lys
        35                  40                  45

Asp Thr Leu Met Ile Ser Arg Thr Pro Glu Val Thr Cys Val Val Val
    50                  55                  60

Asp Val Ser His Glu Asp Pro Glu Val Lys Phe Asn Trp Tyr Val Asp
65                  70                  75                  80

Gly Val Glu Val His Asn Ala Lys Thr Lys Pro Arg Glu Glu Gln Tyr
                85                  90                  95

Asn Ser Thr Tyr Arg Val Val Ser Val Leu Thr Val Leu His Gln Asp
            100                 105                 110

Trp Leu Asn Gly Lys Glu Tyr Lys Cys Lys Val Ser Asn Lys Ala Leu
        115                 120                 125

Pro Ala Pro Ile Glu Lys Thr Ile Ser Lys Ala Lys Gly Gln Pro Arg
    130                 135                 140

Glu Pro Gln Val Tyr Thr Leu Pro Pro Ser Arg Asp Glu Leu Thr Lys
145                 150                 155                 160

Asn Gln Val Ser Leu Thr Cys Leu Val Lys Gly Phe Tyr Pro Ser Asp
                165                 170                 175

Ile Ala Val Glu Trp Glu Ser Asn Gly Gln Pro Glu Asn Asn Tyr Lys
            180                 185                 190

Thr Thr Pro Pro Val Leu Asp Ser Asp Gly Ser Phe Phe Leu Tyr Ser
        195                 200                 205

Lys Leu Thr Val Asp Lys Ser Arg Trp Gln Gln Gly Asn Val Phe Ser
    210                 215                 220

Cys Ser Val Met His Glu Ala Leu His Asn His Tyr Thr Gln Lys Ser
225                 230                 235                 240

Leu Ser Leu Ser Pro Gly Lys
                245
```

## Description

### Related Applications

This application claims priority to U.S. Provisional Patent Application Serial No. 60/253,302 filed November 27, 2000; U.S. Provisional Patent Application Serial No. 60/250,198 filed November 29, 2000; and U.S. Provisional Patent Application Serial No. 60/257,186 filed December 20, 2000, the entire contents of each of which are incorporated herein by reference.

### Background of the Invention

Mononuclear phagocytes are closely associated with diseases of the central nervous system. Microglia found in normal adult brain are highly ramified, quiescent cells that retract processes and become reactive during CNS injury (Rio-Hortega (1932). Reactive microglia (activated brain mononuclear phagocytes) have been identified with Alzheimer Disease (AD) neuritic plaques (Bolsi, 1927; McGeer et al., 1987; Rogers et al., 1988; Giulian, 1992; Perlmutter et al., 1992; Giulian et al., 1995a). As a result, beta amyloid (Aβ)-induced neuron damage is thought to involve inflammatory cells. In Alzheimer Disease, quantitative histopathology has determined that >80% of core plaques are associated with clusters of reactive microglia while fewer than 2% of diffuse Aβ deposits show such an association (Giulian et al., 1995a). These observations suggest that brain inflammatory responses may be directed specifically against the constituents of neuritic and core plaques. As the principal immune effector cells of the brain, activated microglia are capable of releasing such cytotoxic agents as proteolytic enzymes, cytokines, complement proteins, reactive oxygen intermediates, NMDA-like toxins, and nitric oxide (Thery et al., 1991; Giulian, 1992; Rogers et al., 1992; Lees, 1993, Banati, R. B., 1993).

Alzheimer's disease (AD), first described by the Bavarian psychiatrist Alois Alzheimer in 1907, is a progressive neurological disorder that begins with short term memory loss and proceeds to disorientation, impairment of judgment and reasoning and, ultimately, dementia. The course of the disease usually leads to death in a severely debilitated, immobile state between four and 12 years after onset. AD has been estimated to afflict 5 to 11 percent of the population over age 65 and as much as 47 percent of the population over age 85. The societal cost for managing AD is upwards of 80 billion dollars annually, primarily due to the extensive custodial care required for AD patients. Moreover, as adults bom during the population boom of the 1940's and 1950's approach the age when AD becomes more prevalent, the control and treatment of AD will become an even more significant health care problem. Currently, there is no treatment that significantly retards the progression of the disease. For reviews on AD, see Selkoe, D.J. *Sci. Amer.,* November 1991, pp. 68-78; and Yankner, B.A. *et al.* (1991) *N. Eng. J. Med.* 325:1849-1857.

It has been reported (Games *et al.* (1995) *Nature* 373:523-527) that an Alzheimer-type neuropathology has been created in transgenic mice. The transgenic mice express high levels of human mutant amyloid precursor protein and progressively develop many of the pathological conditions associated with AD.

Pathologically, AD is characterized by the presence of distinctive lesions in the victim's brain. These brain lesions include abnormal intracellular filaments called neurofibrillary tangles (NTFs) and extracellular deposits of amyloidogenic proteins in senile, or amyloid, plaques. Amyloid deposits are also present in the walls of cerebral blood vessels of AD patients. The major protein constituent of amyloid plaques has been identified as a 4 kilodalton peptide called β-amyloid peptide (β-AP)(Glenner, G.G. and Wong, C.W. (1984) *Biochem. Biophys. Res. Commun.* 120:885-890; Masters, C. *et al.* (1985) *Proc. Natl. Acad. Sci. USA* 82:4245-4249). Diffuse deposits of β-AP are frequently observed in normal adult brains, whereas AD brain tissue is characterized by more compacted, dense-core β-amyloid plaques. (See *e.g.*, Davies, L. *et al.* (1988) *Neurology* 38:1688-1693) These observations suggest that β-AP deposition precedes, and contributes to, the destruction of neurons that occurs in AD. In further support of a direct pathogenic role for β-AP, β-amyloid has been shown to be toxic to mature neurons, both in culture and *in vivo.* Yankner, B.A. *et al.* (1989) *Science* 245:417-420; Yankner, B.A. *et al.* (1990) *Proc. Natl. Acad. Sci. USA* 87:9020-9023; Roher, A.E. *et al.* (1991) *Biochem. Biophys. Res. Commun.* 174:572-579; Kowall,N.W. *et al.* (1991) *Proc. Natl. Acad. Sci. USA* 88:7247-7251. Furthermore, patients with hereditary cerebral hemorrhage with amyloidosis-Dutch-type (HCHWA-D), which is characterized by diffuse β-amyloid deposits within the cerebral cortex and cerebrovasculature, have been shown to have a point mutation that leads to an amino acid substitution within β-AP. Levy, E. *et al.* (1990) *Science* 248:1124-1126. This observation demonstrates that a specific alteration of the β-AP sequence can cause β-amyloid to be deposited.

Natural β-AP is derived by proteolysis from a much larger protein called the amyloid precursor protein (APP). Kang, J. *et al.* (1987) *Nature* 325:733; Goldgaber, D. *et al.* (1987) *Science* 235:877; Robakis, N.K. *et al.* (1987) *Proc. Natl. Acad Sci. USA* 84:4190; Tanzi, R.E. *et al.* (1987) *Science* 235:880. The APP gene maps to chromosome 21, thereby providing an explanation for the β-amyloid deposition seen at an early age in individuals with Down's syndrome, which is caused by trisomy of chromosome 21. Mann, D.M. *et al.* (1989) *Neuropathol. Appl. Neurobiol.* 15:317; Rumble, B. *et al.* (1989) *N. Eng. J. Med.* 320:1446. APP contains a single membrane spanning domain, with a long amino terminal region (about two-thirds of the protein) extending into the extracellular environment and a shorter carboxy-terminal region

projecting into the cytoplasm. Differential splicing of the APP messenger RNA leads to at least five forms of APP, composed of either 563 amino acids (APP-563), 695 amino acids (APP-695), 714 amino acids (APP-714), 751 amino acids (APP-751) or 770 amino acids (APP-770).

Within APP, naturally-occurring β amyloid peptide begins at an aspartic acid residue at amino acid position 672 of APP-770. Naturally-occurring β-AP derived from proteolysis of APP is 39 to 43 amino acid residues in length, depending on the carboxy-terminal end point, which exhibits heterogeneity. The predominant circulating form of β-AP in the blood and cerebrospinal fluid of both AD patients and normal adults is β1-40 ("short β"). Seubert, P. *et al.* (1992) *Nature* 359:325; Shoji, M. *et al.* (1992) *Science* 258:126. However, β1-42 and β1-43 ("long β") also are forms in β-amyloid plaques. Masters, C. *et al.* (1985) *Proc. Natl. Acad Sci. USA* 82:4245; Miller, D. *et al.* (1993) *Arch. Biochem. Biophys.* 301:41; Mori, H. *et al.* (1992) *J. Biol. Chem.* 267:17082. Although the precise molecular mechanism leading to β-APP aggregation and deposition is unknown, the process has been likened to that of nucleation-dependent polymerizations, such as protein crystallization, microtubule formation and actin polymerization. See *e.g.*, Jarrett, J.T. and Lansbury, P.T. (1993) *Cell* 73:1055-1058. In such processes, polymerization of monomer components does not occur until nucleus formation. Thus, these processes are characterized by a lag time before aggregation occurs, followed by rapid polymerization after nucleation. Nucleation can be accelerated by the addition of a "seed" or preformed nucleus, which results in rapid polymerization. The long β forms of β-AP have been shown to act as seeds, thereby accelerating polymerization of both long and short β-AP forms. Jarrett, J.T. *et al.* (1993) *Biochemistry* 32:4693.

In one study, in which amino acid substitutions were made in β-AP, two mutant β peptides were reported to interfere with polymerization of non-mutated β-AP when the mutant and non-mutant forms of peptide were mixed. Hilbich, C. *et al.* (1992) *J. Mol. Biol.* 228:460-473. Equimolar amounts of the mutant and non-mutant (*i.e.,* natural) β amyloid peptides were used to see this effect and the mutant peptides were reported to be unsuitable for use *in vivo.* Hilbich, C. *et al.* (1992), *supra.*

WO 96/28471 describes an amyloid modulator compound comprising an amyloidogenic protein, or peptide fragment thereof, coupled directly or indirectly to at least one modifying group such that the compound modulates the aggregation of natural amyloid proteins or peptides when contacted with the natural amyloidogenic proteins or peptides. Compounds, recombinant cells, pharmaceutical compositions, method for the preparation thereof and their use according to the invention are not disclosed nor suggested by WO 96/28471.

Pallitto, M. *et al.* (*Biochemistry,* 1999, 38, 3570-3578) describes a modular strategy for generating compounds that inhibit Aβ toxicity, based on linking a recognition element for Aβ to a disrupting element designed to interfere with Aβ aggregation. One such compound, with the 15-25 sequence of Aβ as the recognition element and a lysine hexamer as the disrupting element, altered Aβ aggregation kinetics and protected cells from Aβ toxicity. Optimization experiments with recognition elements consisting of 4-8 residues revealed that none of the recognition peptides altered Aβ aggregation kinetics and only two, KLVFF and KLVF, had any protective effect against Aβ toxicity. The hybrid peptide KLVFF-KKKKKK dramatically altered Aβ aggregation kinetics and aggregate morphology and provided significantly improved protection against Aβ toxicity compared to the recognition peptide alone. The scrambled sequence VLFKF was nearly as effective a recognition domain as KLVFF, suggesting the hydrophobic characteristics of the recognition sequence as critical. Compounds, recombinant cells, pharmaceutical compositions, method for the preparation thereof and their use according to the invention are not disclosed nor suggested by Pallitto, M. *et al.* (*Biochemistry,* 1999, 38, 3570-3578).

## Summary of the Invention

The present invention provides therapeutic agents, pharmaceutical compositions thereof, and methods of use thereof for treating an amyloidogenic disease. The therapeutic agents of the invention include compounds comprising the formula I-L-P, where I is an immunoglobulin, *e.g.*, IgG, IgA, IgM, IgD or IgE, heavy chain constant region or fragment thereof; L is a linker group or a direct bond; and P is a peptide capable of binding an amyloidogenic protein, *e.g.*, β-amyloid, transthyretin (TTR), prion protein (PrP), islet amyloid polypeptide (IAPP), atrial natriuretic factor (ANF), kappa light chain, lambda light chain, amyloid A, procalcitonin, cystatin C, β2 microglobulin, ApoA-I, gelsolin, calcitonin, fibrinogen, lysozyme, Huntington, or α-synuclein. In one embodiment, I may comprise the amino acid sequence set forth in SEQ ID NO:10. In another embodiment, I comprises an amino acid sequence having at least 80%, 85%, 90%, 95%, 98%, or more identity with the amino acid sequence set forth in SEQ ID NO:10. I may be about 1-100, 1-90, 1-80, 1-70, 1-60, 1-50, 1-40, 1-30, 1-20, or 1-10 amino acids.

P may comprise about 4, 5, 6, 7, 8, 9, 10, 11, 12, 13, 14, 15, 16, 17, 18, 19, or 20 amino acids, and preferably about 50, 40, 30, 20, or 10 amino acids. In some embodiments P may comprise at least one non-naturally occurring or at least one D amino acid. In a preferred embodiment, P may comprise a subregion of an amyloidogenic protein such as β-amyloid peptide, transthyretin (TTR), prion protein (PrP), islet amyloid polypeptide (IAPP), atrial natriuretic factor (ANF), kappa light chain, lambda light chain, amyloid A, procalcitonin, cystatin C, β2 microglobulin, ApoA-I, gelsolin, calcitonin, fibrinogen or lysozyme. In a preferred embodiment, P may comprise the amino acid sequence set forth in SEQ ID NO:1 or fragments thereof. In another embodiment, P comprises an amino acid sequence having at least 80%,

85%, 90%, 95%, 98%, or more identity with the amino acid sequence set forth in SEQ ID NO:1.

In another embodiment, P is a peptide comprised entirely of D-amino acids and having at least three amino acid residues independently selected from the group consisting of a D-leucine structure, a D-phenylalanine structure, a D-tyrosine structure, a D-iodotyrosine structure and a D-alanine structure. In a preferred embodiment, P is a peptide comprising the structure

$$(Y\text{-}Xaa_1\text{-}Xaa_2\text{-}Xaa_3\text{-}Xaa_4\text{-}Z)$$

wherein $Xaa_1$, $Xaa_2$, $Xaa_3$ and $Xaa_4$ are each D-amino acid structures and at least two of $Xaa_1$, $Xaa_2$, $Xaa_3$ and $Xaa_4$ are, independently, selected from the group consisting of a D-leucine structure, a D-phenylalanine structure and a D-valine structure; Y, which may or may not be present, is a structure having the formula $(Xaa)_a$, wherein Xaa is any D-amino acid structure and a is an integer from 1 to 15; and Z, which may or may not be present, is a structure having the formula $(Xaa)_b$, wherein Xaa is any D-amino acid structure and b is an integer from 1 to 15.

In a particularly preferred embodiment, P is a peptide selected from the group consisting of: D-Leu-D-Val-D-Phe-D-Phe-D-Leu, D-Leu-D-Val-D-Phe-D-Phe-D-Ala, D-Leu-D-Val-D-Phe-D-Phe, D-Leu-D-Val-D-Phe-phenethylamide, D-Leu-D-Val-D-Tyr-D-Phe, D-Leu-D-Val-D-IodoTyr-D-Phe, D-Leu-D-Val-D-Phe-D-Tyr, D-Leu-D-Val-D-Phe-D-IodoTyr, D-Leu-D-Val-D-Phe-D-Ala, D-Leu-D-Val-D-Phe-D-Phe-D-Ala, D-Ala-D-Val-D-Phe-D-Phe-D-Leu, D-Leu-D-Val-D-Tyr-D-Phe-D-Ala, D-Leu-D-Val-D-IodoTyr-D-Phe-D-Ala, D-Leu-D-Val-D-Phe-D-Tyr-D-Ala, D-Leu-D-Val-D-Phe-D-IodoTyr-D-Ala, D-Phe-D-Phe-D-Val-D-Leu, D-Ala-D-Phe-D-Phe-D-Val, D-Ala-D-Phe-D-Phe-D-Val-D-Leu, D-Ala-D-Phe-D-Phe-D-Leu-D-Leu, D-Leu-D-Phe-D-Phe-D-Val-D-Leu, D-Phe-D-Phe-D-Phe-D-Val-D-Leu, D-Phe-D-Phe-D-Phe-D-Leu-D-Val, D-Phe-D-Phe-D-Phe-D-Phe-D-Leu and D-Ala-D-Phe-D-Phe-D-Phe-D-Leu.

In another aspect, the present invention features dimers or other multimers of the compounds of the invention.

In a further aspect, the invention features a pharmaceutical composition comprising a therapeutically effective amount of a compound of the invention and a pharmaceutically acceptable carrier.

In another aspect, the present invention provides methods for clearing an amyloidogenic protein from a subject by contacting the amyloidogenic protein with a compound of the invention such that the amyloidogenic protein is cleared from the subject.

In yet another aspect, the invention features methods for treating a subject suffering from an amyloidogenic disorder, *e.g.*, Alzheimer's disease or spongifirm encephalopathy, by administering to the subject a therapeutically effective amount of a compound of the invention, thereby treating the subject suffering from an amyloidogenic disorder.

In another embodiment, the present invention provides a method of preparing a therapeutic agent comprising the formula I-L-P', where I is an immunoglobulin, *e.g.*, IgG, IgA, IgM, IgD or IgE, heavy chain constant region or fragment thereof; L is a linker group or a direct bond; and P' is a peptide capable of binding a target protein. The method comprises (1) screening a peptide library to identify one or more peptides which bind to the target protein; (2) determining the amino acid sequence of at least one peptide which binds to the target protein; and (3) producing a therapeutic agent comprising a peptide having the amino acid sequence identified in step (2) and an immunoglobulin heavy chain constant region or fragment thereof, linked via a linker group, L, or a direct bond.

## Brief Description of the Drawings

Figure 1 depicts a Western blot analysis of COS cell lysates and medium harvested from COS cells expressing the Fc region of mouse IgG1 fused to amino acid residues 1-40, 1-42, 10-25, 16-30, 17-21, or 17-21 (A21L) of β-amyloid with or without an N-terminal triple glycine cap.

Figure 2 depicts an immunohistochemistry analysis of coronal brain sections from 20-22 week mice transgenic for both the Swedish mutation of amyloid precursor protein and presenilin of mouse IgG1 fused to various segments of β-amyloid, medium from nontransfected COS cells, or anti-β-amyloid polyclonal antibody.

Figure 3 depicts the synthetic oligonucleotides that were used to assemble the synthetic APP/IgG gene. These oligonucleotides contain unique restriction endonuclease sites needed for the assembly.

Figure 4 is a schematic representation of the pTIg expression vector.

Figure 5 is a schematic representation of the assembly of synthetic Aβ1-40 and Aβ1-42, with and without a triple Gly linker group between the tPA propeptide and the β-amyloid peptide.

Figure 6 depicts the DNA sequence, amino acid composition, and restriction endonuclease recognition sites of the synthetic β-amyloid gene.

Figure 7A depicts the sequence of the oligonucleotides used to assemble subfragments of the synthetic β-amyloid gene and a compilation of the chimeric β-amyloid/IgG1 constructs that were made.

Figure 7B depicts the sequence of the oligonucleotides used to assemble subfragments of the synthetic β-amyloid

gene and a compilation of the chimeric β-amyloid/IgG1 constructs that were made.

Figure 8 is a graph demonstrating that Fc receptor-mediated fibril uptake by cells occurs in the presence of either the Aβ(16-30)-Fc fusion protein or the α-β-amyloid antibody.

Figure 9 is a graph demonstrating that the Aβ(16-30)-Fc fusion protein interferes with the binding of soluble β-amyloid peptide to amyloid fibrils.

Figure 10 is brain section stained with Thioflavin S, demonstrating that treatment of an Alzheimer's disease model transgenic mouse with the Aβ(16-30)-Fc fusion protein results in a decrease in plaque at the site of administration.

Figure 11 depicts the coding region of the tPAΔpro/16-30/Fc cDNA synthetic gene synthetic gene (SEQ ID NO:11).

Figure 12 depicts the amino acid sequence of the tPAΔpro/16-30/Fc fusion protein (SEQ ID NO:12). Annotated functional elements are also shown. The Aβ(16-30)-Fc protein is set forth herein as SEQ ID NO:13

## Detailed Description of the Invention

The present invention provides therapeutic agents and methods of use thereof for treating an amyloidogenic disease. The therapeutic agents of the invention include compounds comprising the formula I-L-P, wherein I is an immunoglobulin heavy chain constant region or fragment thereof (*e.g.*, comprising the Fc region); L is a linker group or a direct bond; and P is a peptide capable of binding an amyloidogenic protein.

Without intending to be limited by theory it is believed that the P portion of the compounds of the invention will serve to bind an amyloidogenic protein, *e.g.*, an amyloidogenic protein within an amyloid plaque, and the I portion of the compounds of the invention will serve to direct microglia to the amyloidogenic protein, which microglia may then internalize and degrade the amyloidogenic protein and the amyloid plaque.

As used interchangeably herein, the terms "I" and "immunoglobulin heavy chain constant region" are intended to include the constant region of any immunoglobulin heavy chain, *e.g.*, $\gamma_1$, $\gamma_2$, $\gamma_3$, $\gamma_4$, μ, $\alpha_1$, $\alpha_2$, δ, or ε heavy chain, or a fragment thereof. The immunoglobulin heavy chain constant region or fragment thereof may be monoclonally or polyclonally derived, has no epitopic specificity and, preferably, contains an Fc region (*i.e.,* retains the ability to bind an Fc receptor, *e.g.,* an Fc receptor on a microglial cell such as an Fcγ receptor). In preferred embodiments, I will include the Fc region of an immunoglobulin heavy chain constant region. For example, I preferably includes the CH2 and the CH3 domains and the hinge region of an immunoglobulin heavy chain constant region.

Immunoglobulin heavy chain constant regions are known in the art. For example, the mouse IgG sequence may be found in GenBank Accession No. M60428, the human IgG1 sequence may be found in GenBank Accession No. J00228, the human IgG2 sequence may be found in GenBank Accession No. J00230, the human IgG3 sequence may be found in GenBank Accession No. AJ390267, and the human IgG4 sequence may be found in GenBank Accession No. K01316, the contents of all of which are incorporated herein by reference. Preferred sequences to be used include the mouse IgG sequence starting at residue 98 and ending at the C-terminus of the molecule, the human IgG1 sequence starting at residue 97 and ending at the C-terminus of the molecule, the human IgG2 sequence starting at residue 97 and ending at the C-terminus of the molecule, the human IgG3 sequence starting at residue 98 and ending at the C-terminus of the molecule, and the human IgG4 sequence starting at residue 97 and ending at the C-terminus of the molecule. In one embodiment, I may comprise the amino acid sequence set forth in SEQ ID NO:10 or fragments thereof.

An "Fc receptor," as used herein, is a protein expressed on the surface of a cell, *e.g.*, a microglial cell, that recognizes and binds to the non-specific, constant heavy chain region of circulating immunoglobulins, *e.g.*, IgG, IgA, IgM, IgD or IgE.

An "Fc region" as used herein, includes the part of an immunoglobulin heavy chain constant region that is required for binding an Fc receptor.

As used interchangeably herein, the terms "P" and "peptide capable of binding an amyloidogenic protein" are intended to include compounds comprising four or more amino acid residues linked by amide bonds that have the ability to bind an amyloidogenic protein. Such compounds can be natural peptide biomolecules, amino acid sequence variants of a natural peptide biomolecule, or synthetic peptides. In one embodiment, the peptide includes any or all of the twenty natural L-amino acids. The peptide can also include one or more D-amino acid residues and/or one or more non-natural amino acid residues.

As used herein, the term "amyloidogenic protein" includes any protein that is capable of, or is involved in forming an amyloid deposit, *e.g.*, an extracellular protein deposit characteristic of a number of different diseases. Though diverse in their occurrence, all amyloid deposits have common morphological properties, stain with specific dyes (*e.g.*, Congo red), and have a characteristic red-green birefringent appearance in polarized light after staining. They also share common ultrastructural features and common x-ray diffraction and infrared spectra. Examples of amyloidogenic proteins include transthyretin (TTR), prion protein (PrP), islet amyloid polypeptide (IAPP), atrial natriuretic factor (ANF), kappa light chain, lambda light chain, amyloid A, procalcitonin, cystatin C, β2 microglobulin, ApoA-I, gelsolin, calcitonin, fibrinogen, lysozyme, Huntington, and α-synuclein.

As used interchangeably herein, the terms "L" and "linker" include a direct bond or any agent that can be used

to link the immunoglobulin heavy chain constant region or fragment thereof and the peptide capable of binding an amyloidogenic protein.

Preferred linkers include peptidic linkers as well as heterobifunctional cross-linkers, which can be used to link proteins in a stepwise manner. A wide variety of heterobifunctional cross-linkers are known in the art, including succinimidyl 4-(N-maleimidomethyl) cyclohexane- 1-carboxylate (SMCC), m-Maleimidobenzoyl-N-hydroxysuccinimide ester (MBS); N-succinimidyl (4-iodoacetyl) aminobenzoate (SIAB), succinimidyl 4-(p-maleimidophenyl) butyrate (SMPB), 1-ethyl-3-(3-dimethylaminopropyl) carbodiimide hydrochloride (EDC); 4-succinimidyl-oxycarbonyl-a-methyl-a-(2-pyridyldithio)-toluene (SMPT), N-succinimidyl 3-(2-pyridyldithio) propionate (SPDP), succinimidyl 6-[3-(2-pyridyldithio) propionate] hexanoate (LC-SPDP).

In one embodiment, the linker is an amino acid residue or a sequence of amino acid residues. Preferably, the linker is about 1-20, about 1-15, about 1-10, or about 1-5 amino acid residues. Most preferably, the linker comprises amino acid residues with small side chains, *e.g.*, alanine or glycine. Most preferably, the linker is $Ala_N$ or $Gly_N$, where N is about 1-10 residues.

The present invention also provides methods for treating a subject suffering from an amyloidogenic disorder. The methods include administering to the subject a therapeutically effective amount of a compound of the invention, thereby treating the subject suffering from the amyloidogenic disorder.

As used herein, the term "amyloidogenic disorder" includes any disease, disorder or condition caused or characterized by deposits of an amyloidogenic protein. Non limiting examples of amyloidogenic disorders include those caused or characterized by deposits of Transthyretin (TTR),*e.g.*, familial amyloid polyneuropathy (Portuguese, Japanese and Swedish types), familial amyloid cardiomyopathy (Danish type), isolated cardiac amyloid and systemic senile amyloidosis; those caused or characterized by deposits of Prion Protein (PrP), *e.g.*, spongiform encephalopathies, including scrapie in sheep, bovine spongiform encephalopathy in cows and Creutzfeldt-Jakob disease (CJ) and Gerstmann-Straussler-Scheinker syndrome (GSS) in humans; those caused or characterized by deposits of Islet Amyloid Polypeptide (IAPP, also known as amylin), *e.g.*, adult onset diabetes and insulinoma; those caused or characterized by deposits of Atrial Natriuretic Factor (ANF), *e.g.,* isolated atrial amyloid; those caused or characterized by deposits of Kappa or Lambda Light Chain, *e.g.,* idiopathic (primary) amyloidosis, myeloma or macroglobulinemia-associated amyloidosis, and primary localized cutaneous nodular amyloidosis associated with Sjogren's syndrome; those caused or characterized by deposits of Amyloid A, *e.g.*, reactive (secondary) amyloidosis (see *e.g.*, Liepnieks, J.J., *et al.* (1995) *Biochim. Biophys. Acta* 1270:81-86), familial Mediterranean Fever and familial amyloid nephropathy with urticaria and deafness (Muckle-Wells syndrome); those caused or characterized by deposits of Cystatin C, *e.g.*, hereditary cerebral hemorrhage with amyloidosis of Icelandic type; those caused or characterized by deposits of β2 microglobulin (β2M), *e.g.,* complications associated with long term hemodialysis; those caused or characterized by deposits of Apolipoprotein A-I (ApoA-I), *e.g.*, hereditary non-neuropathic systemic amyloidosis (familial amyloid polyneuropathy III); those caused or characterized by deposits of Gelsolin, *e.g.*, familial amyloidosis of Finnish type; those caused or characterized by deposits of Procalcitonin or calcitonin, *e.g.*, amyloid fibrils associated with medullary carcinoma of the thyroid; those caused or characterized by deposits of Fibrinogen, *e.g.*, hereditary renal amyloidosis; and those caused or characterized by deposits of Lysozyme, *e.g.*, hereditary systemic amyloidosis. Other examples of amyloidogenic disorders include Huntington's disease and inclusion body myocytis.

As used herein, the term "subject" includes warm-blooded animals, preferably mammals, including humans. In a preferred embodiment, the subject is a primate. In an even more preferred embodiment, the primate is a human.

As used herein, the term "administering" to a subject includes dispensing, delivering or applying a compound, *e.g.*, a compound in a pharmaceutical formulation (as described herein), to a subject by any suitable route for delivery of the compound to the desired location in the subject, including delivery by either the parenteral or oral route, intramuscular injection, subcutaneous/intradermal injection, intravenous injection, buccal administration, transdermal delivery and administration by the rectal, colonic, vaginal, intranasal or respiratory tract route (*e.g.,* by inhalation).

As used herein, the term "effective amount" includes an amount effective, at dosages and for periods of time necessary, to achieve the desired result, *e.g.*, sufficient to treat an amyloidogenic disorder in a subject. An effective amount of a compound of the invention, as defined herein may vary according to factors such as the disease state, age, and weight of the subject, and the ability of the compound to elicit a desired response in the subject. Dosage regimens may be adjusted to provide the optimum therapeutic response. An effective amount is also one in which any toxic or detrimental effects (*e.g.*, side effects) of the compound are outweighed by the therapeutically beneficial effects.

A therapeutically effective amount of a compound of the invention (*i.e.*, an effective dosage) may range from about 0.001 to 30 mg/kg body weight, preferably about 0.01 to 25 mg/kg body weight, more preferably about 0.1 to 20 mg/kg body weight, and even more preferably about 1 to 10 mg/kg, 2 to 9 mg/kg, 3 to 8 mg/kg, 4 to 7 mg/kg, or 5 to 6 mg/kg body weight. The skilled artisan will appreciate that certain factors may influence the dosage required to effectively treat a subject, including but not limited to the severity of the disease or disorder, previous treatments, the general health and/or age of the subject, and other diseases present. Moreover, treatment of a subject with a therapeutically effective amount of a compound of the invention can include a single treatment or, preferably, can include a series of treatments.

In one example, a subject is treated with a compound of the invention in the range of between about 0.1 to 20 mg/kg body weight, one time per week for between about 1 to 10 weeks, preferably between 2 to 8 weeks, more preferably between about 3 to 7 weeks, and even more preferably for about 4, 5, or 6 weeks. It will also be appreciated that the effective dosage of a compound of the invention used for treatment may increase or decrease over the course of a particular treatment.

Various additional aspects of the present invention are described in further detail in the following subsections.

I. Immunoglobulin Heavy Chain Constant Region

The immunoglobulin heavy chain constant region used in the compounds of the invention may be obtained using a variety of art known techniques. For example, polyclonal immunoglobulin preparations (that may be cleaved as described below to generate immunoglobulin heavy chain constant regions or fragments thereof) can be derived directly from the blood of the desired animal species. Thus, in the case of humans, polyclonal immunoglobulin preparations can be prepared from outdated units of blood utilizing protocols known or readily ascertainable to those of skill in the art. Such products are commercially available (Sandoz Limited; Cutter Laboratories; Hyland Laboratories) and are routinely used for the preparation of immunoglobulins.

In addition, if desired, polyclonal immunoglobulin preparations may be prepared from the blood of immunized subjects of the desired species following immunization with any of a variety of antigens, followed by harvesting of the blood and processing it according to known techniques. A distinctive advantage of non-specific, immunoglobulin preparations is that by preparing immunoglobulin from the same species into which it will be administered, immune reactions across species barriers are prevented and repeated administrations of the same product are less likely to cause side-effects. It should be emphasized that cross-species administrations may be done. However, their use might increase the incidence of untoward reactions such as anaphylactic reactions, febrile reactions, and/or the generation of an immune response to the foreign immunoglobulin protein that will block its effective use, as well as endanger the health of the subject. The avoidance of such reactions adds greatly to the appeal of using an immunoglobulin preparation which is from the same species as that being treated.

Monoclonal immunoglobulins (that may be cleaved as described below to generate immunoglobulin heavy chain constant regions or fragments thereof) can be prepared using well known techniques such as the hybridoma technique originally described by Kohler and Milstein (1975) *Nature* 256:495-497) (see also, Brown *et al.* (1981) *J. Immunol.* 127:539-46; Brown *et al.* (1980) *J. Biol. Chem.* 255:4980-83; Yeh *et al.* (1976) *Proc. Natl. Acad. Sci. USA* 76: 2927-31; and Yeh *et al.* (1982) *Int. J. Cancer* 29:269-75); the more recent human B cell hybridoma technique (Kozbor *et al.* (1983) *Immunol Today* 4:72); or the EBV-hybridoma technique (Cole *et al.* (1985), *Monoclonal Antibodies and Cancer Therapy,* Alan R. Liss, Inc., pp. 77-96). The technology for producing monoclonal antibody hybridomas is well known (see generally R. H. Kenneth, in *Monoclonal Antibodies: A New Dimension In Biological Analyses,* Plenum Publishing Corp., New York, New York (1980); E. A. Lerner (1981) *Yale J. Biol. Med.,* 54:387-402; M. L. Gefter *et al.* (1977) *Somatic Cell Genet.* 3:231-36).

Any of the many well known protocols used for fusing lymphocytes and immortalized cell lines can be applied for the purpose of generating a monoclonal antibody (see, *e.g.*, G. Galfre *et al.* (1977) *Nature* 266:55052; Gefter *et al. Somatic Cell Genet.,* cited *supra*; Lerner, *Yale J. Biol. Med.,* cited *supra*; Kenneth, *Monoclonal Antibodies,* cited *supra*). Moreover, the ordinarily skilled worker will appreciate that there are many variations of such methods which also would be useful. Typically, the immortal cell line (*e.g.*, a myeloma cell line) is derived from the same mammalian species as the lymphocytes. For example, murine hybridomas can be made by fusing lymphocytes from a mouse immunized with an immunogenic preparation of the present invention with an immortalized mouse cell line. Preferred immortal cell lines are mouse myeloma cell lines that are sensitive to culture medium containing hypoxanthine, aminopterin and thymidine ("HAT medium"). Any of a number of myeloma cell lines can be used as a fusion partner according to standard techniques, *e.g.*, the P3-NS1/1-Ag4-1, P3-x63-Ag8.653 or Sp2/O-Ag14 myeloma lines. These myeloma lines are available from ATCC. Typically, HAT-sensitive mouse myeloma cells are fused to mouse splenocytes using polyethylene glycol ("PEG"). Hybridoma cells resulting from the fusion are then selected using HAT medium, which kills unfused and unproductively fused myeloma cells (unfused splenocytes die after several days because they are not transformed).

Alternative to preparing monoclonal antibody-secreting hybridomas, a monoclonal antibody (that may be cleaved as described below to generate immunoglobulin heavy chain constant regions or fragments thereof) can be isolated from a recombinant combinatorial immunoglobulin library (*e.g.*, an antibody phage display library). Kits for generating and screening phage display libraries are commercially available (*e.g.,* the Pharmacia *Recombinant Phage Antibody System,* Catalog No. 27-9400-01; and the Stratagene *SurfZAP*™ *Phage Display Kit,* Catalog No. 240612). Additionally, examples of methods and reagents particularly amenable for use in generating and screening antibody display library can be found in, for example, Ladner *et al.* U.S. Patent No. 5,223,409; Kang *et al.* PCT International Publication No. WO 92/18619; Dower *et al.* PCT International Publication No. WO 91/17271; Winter *et al.* PCT International Publication WO 92/20791; Markland *et al.* PCT International Publication No. WO 92/15679; Breitling *et al.* PCT

International Publication WO 93/01288; McCafferty *et al.* PCT International Publication No. WO 92/01047; Garrard *et al.* PCT International Publication No. WO 92/09690; Ladner *et al.* PCT International Publication No. WO 90/02809; Fuchs *et al.* (1991) *Bio/Technology* 9:1370-1372; Hay *et al.* (1992) *Hum. Antibod. Hybridomas* 3:81-85; Huse *et al.* (1989) *Science* 246:1275-1281; Griffiths *et al.* (1993) *EMBO J* 12:725-734; Hawkins *et al.* (1992) *J. Mol. Biol.* 226:889-896; Clarkson *et al.* (1991) *Nature* 352:624-628; Gram *et al.* (1992) *Proc. Natl. Acad. Sci. USA* 89:3576-3580; Garrad *et al.* (1991) *Bio/Technology* 9:1373-1377; Hoogenboom *et al.* (1991) *Nuc. Acid Res.* 19:4133-4137; Barbas *et al.* (1991) *Proc. Natl. Acad. Sci. USA* 88:7978-7982; and McCafferty *et al. Nature* (1990) 348:552-554.

Additionally, recombinant immunoglobulins, such as chimeric and humanized immunoglobulins, comprising both human and non-human portions, which can be made using standard recombinant DNA techniques, can also be used to generate immunoglobulin heavy chain constant regions or fragments thereof. Such chimeric and humanized monoclonal immunoglobulins/antibodies can be produced by recombinant DNA techniques known in the art, for example using methods described in Robinson *et al.* International Application No. PCT/US86/02269; Akira, *et al.* European Patent Application 184,187; Taniguchi, M., European Patent Application 171,496; Morrison *et al.* European Patent Application 173,494; Neuberger *et al.* PCT International Publication No. WO 86/01533; Cabilly *et al.* U.S. Patent No. 4,816,567; Cabilly *et al.* European Patent Application 125,023; Better *et al.* (1988) *Science* 240:1041-1043; Liu *et al.* (1987) *Proc. Natl. Acad. Sci. USA* 84:3439-3443; Liu *et al.* (1987) *J. Immunol.* 139:3521-3526; Sun *et al.* (1987) *Proc. Natl. Acad. Sci. USA* 84:214-218; Nishimura *et al.* (1987) *Canc. Res.* 47:999-1005; Wood *et al.* (1985) *Nature* 314:446-449; and Shaw *et al.* (1988) *J. Natl. Cancer Inst.* 80:1553-1559); Morrison, S. L. (1985) *Science* 229:1202-1207; Oi *et al.* (1986) *BioTechniques* 4:214; Winter U.S. Patent 5,225,539; Jones *et al.* (1986) *Nature* 321:552-525; Verhoeyan *et al.* (1988) *Science* 239:1534; and Beidler *et al.* (1988) *J. Immunol.* 141:4053-4060.

Antibodies/immunoglobulins prepared by any of the foregoing techniques may then be cleaved, *e.g.*, using known enzymes such as papain, pepsin and subtilisin, to generate immunoglobulin heavy chain constant regions or fragments thereof.

Moreover, the compounds of the invention (comprising an immunoglobulin heavy chain constant region or a fragment thereof, *e.g.,* a fragment comprising the Fc region) may be generated as fusion proteins using standard recombinant DNA techniques, as described in further detail below.

II. <u>Peptides Capable Of Binding An Amyloidogenic Protein</u>

The "P" component of the compounds of the invention may be any molecule comprising four or more amino acid residues linked by amide bonds that has the ability to bind an amyloidogenic protein.

In one embodiment, the "P" component of the compounds of the invention is designed based upon the amino acid sequence of the natural β-AP. The terms "natural β-AP," "natural β-amyloid peptide," and "natural Aβ peptide", used interchangeably herein, are intended to encompass naturally occurring proteolytic cleavage products of the β amyloid precursor protein (APP) which are involved in β-AP aggregation and β-amyloidosis. These natural peptides include β-amyloid peptides having 39-43 amino acids (*i.e.,* $A\beta_{1-39}$, $A\beta_{1-40}$, $A\beta_{1-41}$, $A\beta_{1-42}$ and $A\beta_{1-43}$). The amino-terminal amino acid residue of natural β-AP corresponds to the aspartic acid residue at position 672 of the 770 amino acid residue form of the amyloid precursor protein ("APP-770"). The 43 amino acid long form of natural β-AP has the amino acid sequence DAEFRHDSGYEVHHQKLVFFAEDVGSNKGAIIGLMVGGVVIAT (SEQ ID NO:1), whereas the shorter forms have 1-4 amino acid residues deleted from the carboxy-terminal end. Any fragment of the natural β-AP that is capable of binding an amyloidogenic protein may be used as the "P" component in the compounds of the invention. Preferably, the "P" component in the compounds of the invention may comprise at least 4, 5, 6, 7, 8, 9, 10, 11, 12, 13, 14, 15, 16, 17, 18, 19, or 20 contiguous amino acids of a natural Aβ peptide.

In a preferred embodiment, the "P" component in the compounds of the invention is designed based upon the amino acid sequence of an "Aβ aggregation core domain" (ACD). As used herein, the term "Aβ aggregation core domain" refers to a subregion of a natural β-amyloid peptide that is sufficient to modulate aggregation of natural β-APs when this subregion, in its L-amino acid form, is appropriately modified (*e.g.*, modified at the amino-terminus), as described in detail in U.S. patent application Serial No. 08/548,998 and U.S. patent application Serial No. 08/616,081, the entire contents of each of which are expressly incorporated herein by reference. Preferably, the "P" component in the compounds of the invention is or is modeled after a subregion of natural β-AP that is less than 15 amino acids in length and more preferably is between 4-10 amino acids in length. In various embodiments, the "P" component in the compounds of the invention is, or is modeled after, a subregion of β-AP that is 4, 5, 6, 7, 8, 9, 10, 11, 12, 13, 14, 15, or more amino acids in length.

In one embodiment, the subregion of β-AP upon which the "P" component in the compounds of the invention is modeled is an internal or carboxy-terminal region of β-AP (*i.e.*, downstream of the amino-terminus at amino acid position 1). P can, thus, be a fragment of Aβ that includes 35 or fewer, 30 or fewer, 25 or fewer, 20 or fewer or 15 or fewer amino acid residues. In another embodiment, the "P" component in the compounds of the invention is, or is modeled after, a subregion of β-AP that is hydrophobic. Preferred "P" components encompass amino acid residues 16-30, 17-20,

17-21, 16-25, or 1-25 of natural β-AP ($A\beta_{16-30}$, $A\beta_{17-20}$, $A\beta_{17-21}$, $A\beta_{16-25}$, or $A\beta_{1-25}$, respectively). The amino acid sequences of $A\beta_{17-20}$ and $A\beta_{17-21}$ are Leu-Val-Phe-Phe (SEQ ID NO:2) and Leu-Val-Phe-Phe-Ala (SEQ ID NO:3), respectively,

The "P" component in the compounds of the invention may comprise a D-amino acid sequence corresponding to the L-amino acid sequence of $A\beta_{17-20}$, $A\beta_{17-21}$, $A\beta_{16-25}$, or $A\beta_{1-25}$, a D-amino acid sequence which is a retro-inverso isomer of the L-amino acid sequence of $A\beta_{17-20}$, $A\beta_{17-21}$, $A\beta_{16-25}$, or $A\beta_{1-25}$, or a D-amino acid sequence that is a scrambled or substituted version of the L-amino acid sequence of $A\beta_{17-20}$, $A\beta_{17-21}$, $A\beta_{16-25}$, or $A\beta_{1-25}$. The structures of effective "P" components are generally hydrophobic and are characterized by the presence of at least two D-amino acid structures independently selected from the group consisting of a D-leucine structure, a D-phenylalanine structure and a D-valine structure. As used herein, the term a "D-amino acid structure" (such as a "D-leucine structure", a "D-phenylalanine structure" or a "D-valine structure") is intended to include the D-amino acid, as well as analogues, derivatives and mimetics of the D-amino acid that maintain the ability of the "P" component to bind an amyloidogenic protein. For example, the term "D-phenylalanine structure" is intended to include D-phenylalanine as well as D-pyridylalanine and D-homophenylalanine. The term "D-leucine structure" is intended to include D-leucine, as well as substitution with D-valine or other natural or non-natural amino acids having an aliphatic side chain, such as D-norleucine. The term "D-valine structure" is intended to include D-valine, as well as substitution with D-leucine or other natural or non-natural amino acids having an aliphatic side chain.

In other embodiments, the peptidic structure of the "P" component in the compounds of the invention comprises at least two D-amino acid structures independently selected from the group consisting of a D-leucine structure, a D-phenylalanine structure, a D-valine structure, a D-alanine structure, a D-tyrosine structure and a D-iodotyrosine structure. In another embodiment, the peptidic structure of the "P" component in the compounds of the invention is comprised of at least three D-amino acid structures independently selected from the group consisting of a D-leucine structure, a D-phenylalanine structure and a D-valine structure. In yet another embodiment, the peptidic structure of the "P" component in the compounds of the invention is comprised of at least three D-amino acid structures independently selected from the group consisting of a D-leucine structure, a D-phenylalanine structure, a D-valine structure, a D-alanine structure, a D-tyrosine structure and a D-iodotyrosine structure. In yet another embodiment, the peptidic structure of the "P" component in the compounds of the invention comprises at least four D-amino acid structures independently selected from the group consisting of a D-leucine structure, a D-phenylalanine structure and a D-valine structure. In yet another embodiment, the peptidic structure of the "P" component in the compounds of the invention is comprised of at least four D-amino acid structures independently selected from the group consisting of a D-leucine structure, a D-phenylalanine structure and a D-valine structure. In a preferred embodiment, the peptidic structure of the "P" component in the compounds of the invention includes a D-amino acid dipeptide selected from the group consisting of D-Phe-D-Phe, D-Phe-D-Tyr, D-Tyr-D-Phe, D-Phe-D-IodoTyr and D-IodoTyr-D-Phe.

In one embodiment, the "P" component in the compounds of the invention comprises a formula (I):

$$(\ Y\text{-}Xaa_1\text{-}Xaa_2\text{-}Xaa_3\text{-}Xaa_4\text{-}Z\ ) \diagup An \qquad \text{(I)}$$

wherein $Xaa_1$, $Xaa_2$, $Xaa_3$ and $Xaa_4$ are each D-amino acid structures and at least two of $Xaa_1$, $Xaa_2$, $Xaa_3$ and $Xaa_4$ are, independently, selected from the group consisting of a D-leucine structure, a D-phenylalanine structure and a D-valine structure;

Y, which may or may not be present, is a structure having the formula $(Xaa)_a$, wherein Xaa is any D-amino acid structure and a is an integer from 1 to 15;

Z, which may or may not be present, is a structure having the formula $(Xaa)_b$, wherein Xaa is any D-amino acid structure and b is an integer from 1 to 15;

A, which may or may not be present, is a modifying group attached directly or indirectly to the "P" component; and

n is an integer from 1 to 15;

wherein $Xaa_1$, $Xaa_2$, $Xaa_3$, $Xaa_4$, Y, Z, A and n are selected such that the "P" component binds to an amyloidogenic protein.

In a sub-embodiment of this formula, a fifth amino acid residue, $Xaa_5$, is specified C-terminal to $Xaa_4$ and Z, which may or may not be present, is a structure having the formula $(Xaa)_b$, wherein Xaa is any D-amino acid structure and b is an integer from 1 to 14. Accordingly, the "P" component in the compounds of the invention may comprise a formula (II):

$$(Y\text{-}Xaa_1\text{-}Xaa_2\text{-}Xaa_3\text{-}Xaa_4\text{-}Xaa_5\text{-}Z) A_n \qquad (II)$$

wherein b is an integer from 1 to 14.

In a preferred embodiment, $Xaa_1$, $Xaa_2$, $Xaa_3$, $Xaa_4$ of formula (I) are selected based on the sequence of $A\beta_{17-20}$, or acceptable substitutions thereof. Accordingly, in preferred embodiments, $Xaa_1$ is a D-alanine structure or a D-leucine structure, $Xaa_2$ is a D-valine structure, $Xaa_3$ is a D-phenylalanine structure, a D-tyrosine structure or a D-iodotyrosine structure and $Xaa_4$ is a D-phenylalanine structure, a D-tyrosine structure or a D-iodotyrosine structure.

In another preferred embodiment, $Xaa_1$, $Xaa_2$, $Xaa_3$, $Xaa_4$ and $Xaa_5$ of formula (II) are selected based on the sequence of $A\beta_{17-21}$, or acceptable substitutions thereof. Accordingly, in preferred embodiments, $Xaa_1$ is a D-alanine structure or a D-leucine structure, $Xaa_2$ is a D-valine structure, $Xaa_3$ is a D-phenylalanine structure, a D-tyrosine structure or a D-iodotyrosine structure, $Xaa_4$ is a D-phenylalanine structure, a D-tyrosine structure or a D-iodotyrosine structure, and $Xaa_5$ is a D-alanine structure or a D-leucine structure.

In another preferred embodiment, $Xaa_1$, $Xaa_2$, $Xaa_3$ and $Xaa_4$ of formula (I) are selected based on the retro-inverso isomer of $A\beta_{17-20}$, or acceptable substitutions thereof. Accordingly, in preferred embodiments, $Xaa_1$ is a D-alanine structure, a D-leucine structure or a D-phenylalanine structure, $Xaa_2$ is a D-phenylalanine structure, a D-tyrosine structure or a D-iodotyrosine structure, $Xaa_3$ is a D-phenylalanine structure, a D-tyrosine structure or a D-iodotyrosine structure and $Xaa_4$ is a D-valine structure or a D-leucine structure.

In another preferred embodiment, $Xaa_1$, $Xaa_2$, $Xaa_3$, $Xaa_4$ and $Xaa_5$ of formula (II) are selected based on the retroinverso isomer of $A\beta_{17-21}$, or acceptable substitutions thereof. Accordingly, in preferred embodiments, $Xaa_1$ is a D-alanine structure, a D-leucine structure or a D-phenylalanine structure, $Xaa_2$ is a D-phenylalanine structure, a D-tyrosine structure or a D-iodotyrosine structure, $Xaa_3$ is a D-phenylalanine structure, a D-tyrosine structure or a D-iodotyrosine structure, $Xaa_4$ is a D-valine structure or a D-leucine structure and $Xaa_5$ is a D-leucine structure.

In another embodiment, the "P" component in the compounds of the invention may comprise a formula (III):

$$A\text{-}(Y)\text{-}Xaa_1\text{-}Xaa_2\text{-}Xaa_3\text{-}Xaa_4\text{-}(Z)\text{-}B \qquad (III)$$

wherein $Xaa_1$, $Xaa_2$, $Xaa_3$ and $Xaa_4$ are each D-amino acid structures and at least two of $Xaa_1$, $Xaa_2$, $Xaa_3$ and $Xaa_4$ are, independently, selected from the group consisting of a D-leucine structure, a D-phenylalanine structure and a D-valine structure;

Y, which may or may not be present, is a peptidic structure having the formula $(Xaa)_a$, wherein Xaa is any amino acid structure and a is an integer from 1 to 15;

Z, which may or may not be present, is a peptidic structure having the formula $(Xaa)_b$, wherein Xaa is any amino acid structure and b is an integer from 1 to 15; and

A, which may or may not be present, is a modifying group attached directly or indirectly to the amino terminus of the "P" component; and

B, which may or may not be present, is a modifying group attached directly or indirectly to the carboxy terminus of the "P" component;

$Xaa_1$, $Xaa_2$, $Xaa_3$, $Xaa_4$, Y, Z, A and B being selected such that the compound binds to an amyloidogenic protein.

In a sub-embodiment of formula (III), a fifth amino acid residue, $Xaa_5$, is specified C-terminal to $Xaa_4$ and Z, which may or may not be present, is a structure having the formula $(Xaa)_b$, wherein Xaa is any D-amino acid structure and b is an integer from 1 to 14. Accordingly, the "P" component in the compounds of the invention may comprise a formula (IV):

$$A\text{-}(Y)\text{-}Xaa_1\text{-}Xaa_2\text{-}Xaa_3\text{-}Xaa_4\text{-}Xaa_5\text{-}(Z)\text{-}B \qquad (IV)$$

wherein b is an integer from 1 to 14.

In a preferred embodiment, $Xaa_1$, $Xaa_2$, $Xaa_3$, $Xaa_4$ of formula (III) are selected based on the sequence of $A\beta_{17-20}$, or acceptable substitutions thereof. Accordingly, in preferred embodiments, $Xaa_1$ is a D-alanine structure or a D-leucine structure, $Xaa_2$ is a D-valine structure, $Xaa_3$ is a D-phenylalanine structure, a D-tyrosine structure or a D-iodotyrosine structure and $Xaa_4$ is a D-phenylalanine structure, a D-tyrosine structure or a D-iodotyrosine structure.

In another preferred embodiment, $Xaa_1$, $Xaa_2$, $Xaa_3$, $Xaa_4$ and $Xaa_5$ of formula (IV) are selected based on the sequence of $A\beta_{17-21}$, or acceptable substitutions thereof. Accordingly, in preferred embodiments, $Xaa_1$ is a D-alanine structure or a D-leucine structure, $Xaa_2$ is a D-valine structure, $Xaa_3$ is a D-phenylalanine structure, a D-tyrosine structure or a D-iodotyrosine structure, $Xaa_4$ is a D-phenylalanine structure, a D-tyrosine structure or a D-iodotyrosine structure,

and $Xaa_5$ is a D-alanine structure or a D-leucine structure.

In another preferred embodiment, $Xaa_1$, $Xaa_2$, $Xaa_3$ and $Xaa_4$ of formula (III) are selected based on the retro-inverso isomer of $A\beta_{17\text{-}20}$, or acceptable substitutions thereof. Accordingly, in preferred embodiments, $Xaa_1$ is a D-alanine structure, a D-leucine structure or a D-phenylalanine structure, $Xaa_2$ is a D-phenylalanine structure, a D-tyrosine structure or a D-iodotyrosine structure, $Xaa_3$ is a D-phenylalanine structure, a D-tyrosine structure or a D-iodotyrosine structure and $Xaa_4$ is a D-valine structure or a D-leucine structure.

In another preferred embodiment, $Xaa_1$, $Xaa_2$, $Xaa_3$, $Xaa_4$ and $Xaa_5$ of formula (IV) are selected based on the retroinverso isomer of $A\beta_{17\text{-}21}$, or acceptable substitutions thereof. Accordingly, in preferred embodiments, $Xaa_1$ is a D-alanine structure, a D-leucine structure or a D-phenylalanine structure, $Xaa_2$ is a D-phenylalanine structure, a D-tyrosine structure or a D-iodotyrosine structure, $Xaa_3$ is a D-phenylalanine structure, a D-tyrosine structure or a D-iodotyrosine structure, $Xaa_4$ is a D-valine structure or a D-leucine structure and $Xaa_5$ is a D-leucine structure.

In preferred embodiments, the "P" component in the compounds of the invention may comprise a peptidic structure selected from the group consisting of D-Leu-D-Val-D-Phe-D-Phe, D-Leu-D-Val-D-Phe-phenethylamide, D-Leu-D-Val-D-Tyr-D-Phe, D-Leu-D-Val-D-IodoTyr-D-Phe, D-Leu-D-Val-D-Phe-D-Tyr, D-Leu-D-Val-D-Phe-D-IodoTyr, D-Leu-D-Val-D-Phe-D-Ala, D-Leu-D-Val-D-Phe-D-Phe-D-Ala, D-Ala-D-Val-D-Phe-D-Phe-D-Leu, D-Leu-D-Val-D-Tyr-D-Phe-D-Ala, D-Leu-D-Val-D-IodoTyr-D-Phe-D-Ala, D-Leu-D-Val-D-Phe-D-Tyr-D-Ala, D-Leu-D-Val-D-Phe-D-IodoTyr-D-Ala, D-Phe-D-Phe-D-Val-D-Leu, D-Ala-D-Phe-D-Phe-D-Val, D-Ala-D-Phe-D-Phe-D-Val-D-Leu, D-Ala-D-Phe-D-Phe-D-Leu-D-Leu, D-Leu-D-Phe-D-Phe-D-Val-D-Leu, D-Phe-D-Phe-D-Phe-D-Val-D-Leu, D-Phe-D-Phe-D-Phe-D-Leu-D-Val, D-Phe-D-Phe-D-Phe-D-Phe-D-Leu, D-Ala-D-Phe-D-Phe-D-Phe-D-Leu, and D-Leu-D-Val-D-Phe-D-Phe-D-Leu.

Preferred "P" components may comprise D-amino acid peptide amides designed based on the foregoing retro-inverso isomers of $A\beta_{17\text{-}21}$, or acceptable substitutions thereof.

The D-amino acid peptidic structures of the "P" components in the compounds of the invention are further intended to include other peptide modifications, including analogues, derivatives and mimetics, that retain the ability of the "P" component to bind an amyloidogenic protein. For example, a D-amino acid peptidic structure of a "P" component may be further modified to increase its stability, bioavailability, or solubility. The terms "analogue", "derivative" and "mimetic" as used herein are intended to include molecules which mimic the chemical structure of a D-peptidic structure and retain the functional properties of the D-peptidic structure. Approaches to designing peptide analogs, derivatives and mimetics are known in the art. For example, see Farmer, P.S. in Drug Design (E.J. Ariens, ed.) Academic Press, New York, 1980, vol. 10, pp. 119-143; Ball. J.B. and Alewood, P.F. (1990) *J. Mol. Recognition* 3:55; Morgan, B.A. and Gainor, J.A. (1989) *Ann. Rep. Med. Chem.* 24:243; and Freidinger, R.M. (1989) *Trends Pharmacol. Sci.* 10:270. See also Sawyer, T.K. (1995) "Peptidomimetic Design and Chemical Approaches to Peptide Metabolism" in Taylor, M.D. and Amidon, G.L. (eds.) *Peptide-Based Drug Design: Controlling Transport and Metabolism,* Chapter 17; Smith, A.B. 3rd, *et al.* (1995) *J. Am. Chem. Soc.* 117:11113-11123; Smith, A.B. 3rd, *et al.* (1994) *J. Am. Chem. Soc.* 116:9947-9962; and Hirschman, R., *et al.* (1993) *J. Am. Chem. Soc.* 115:12550-12568.

As used herein, a "derivative" of a compound X (*e.g.,* a peptide or amino acid) refers to a form of X in which one or more reaction groups on the compound have been derivatized with a substituent group. Examples of peptide derivatives include peptides in which an amino acid side chain, the peptide backbone, or the amino- or carboxy-terminus has been derivatized (*e.g.,* peptidic compounds with methylated amide linkages). As used herein an "analogue" of a compound X refers to a compound which retains chemical structures of X necessary for functional activity of X yet which also contains certain chemical structures which differ from X. An examples of an analogue of a naturally-occurring peptide is a peptide which includes one or more non-naturally-occurring amino acids. As used herein, a "mimetic" of a compound X refers to a compound in which chemical structures of X necessary for functional activity of X have been replaced with other chemical structures which mimic the conformation of X. Examples of peptidomimetics include peptidic compounds in which the peptide backbone is substituted with one or more benzodiazepine molecules (see *e.g.*, James, G.L. *et al.* (1993) *Science* 260:1937-1942).

Analogues of the "P" component are intended to include compounds in which one or more D-amino acids of the peptidic structure are substituted with a homologous amino acid such that the properties of the original "P" component are maintained. Preferably conservative amino acid substitutions are made at one or more amino acid residues. A "conservative amino acid substitution" is one in which the amino acid residue is replaced with an amino acid residue having a similar side chain. Families of amino acid residues having similar side chains have been defined in the art, including basic side chains (*e.g.*, lysine, arginine, histidine), acidic side chains (*e.g.*, aspartic acid, glutamic acid), uncharged polar side chains (*e.g.*, glycine, asparagine, glutamine, serine, threonine, tyrosine, cysteine), nonpolar side chains (*e.g.*, alanine, valine, leucine, isoleucine, proline, phenylalanine, methionine, tryptophan), β-branched side chains (*e.g.*, threonine, valine, isoleucine) and aromatic side chains (*e.g.*, tyrosine, phenylalanine, tryptophan, histidine). Non-limiting examples of homologous substitutions that can be made in the peptidic structures of the "P" component include substitution of D-phenylalanine with D-tyrosine, D-pyridylalanine or D-homophenylalanine, substitution of D-leucine with D-valine or other natural or non-natural amino acid having an aliphatic side chain and/or substitution of D-valine with D-leucine or other natural or non-natural amino acid having an aliphatic side chain.

The term mimetic, and in particular, peptidomimetic, is intended to include isosteres. The term "isostere" as used herein is intended to include a chemical structure that can be substituted for a second chemical structure because the steric conformation of the first structure fits a binding site specific for the second structure. The term specifically includes peptide back-bone modifications (*i.e.*, amide bond mimetics) well known to those skilled in the art. Such modifications include modifications of the amide nitrogen, the α-carbon, amide carbonyl, complete replacement of the amide bond, extensions, deletions or backbone crosslinks. Several peptide backbone modifications are known, including ψ[$CH_2S$], ψ[$CH_2NH$], ψ[$CSNH_2$], ψ[NHCO], ψ[$COCH_2$], and ψ [(E) or (Z) CH=CH]. In the nomenclature used above, ψ indicates the absence of an amide bond. The structure that replaces the amide group is specified within the brackets.

Other possible modifications include an N-alkyl (or aryl) substitution (ψ [CONR]), or backbone crosslinking to construct lactams and other cyclic structures. Other derivatives of the "P" component include C-terminal hydroxymethyl derivatives, *O*-modified derivatives (*e.g.*, C-terminal hydroxymethyl benzyl ether), N-terminally modified derivatives including substituted amides such as alkylamides and hydrazides and "P" components in which a C-terminal phenylalanine residue is replaced with a phenethylamide analogue (*e.g.*, Val-Phe-phenethylamide as an analogue of the tripeptide Val-Phe-Phe).

A "P" component may also be modified with a modifying group. The term "modifying group" is intended to include structures that are directly attached to the peptidic structure of the "P" component (*e.g.*, by covalent coupling), as well as those that are indirectly attached to the peptidic structure (*e.g.*, by a stable non-covalent association or by covalent coupling to additional amino acid residues, or mimetics, analogues or derivatives thereof, which may flank the peptidic structure). For example, the modifying group can be coupled to the amino-terminus or carboxy-terminus of the "P" component. Alternatively, the modifying group can be coupled to a side chain of at least one L- or D-amino acid residue of the "P" component (*e.g.*, through the epsilon amino group of a lysyl residue(s), through the carboxyl group of an aspartic acid residue(s) or a glutamic acid residue(s), through a hydroxy group of a tyrosyl residue(s), a serine residue (s) or a threonine residue(s) or other suitable reactive group on an amino acid side chain). Modifying groups covalently coupled to the "P" component can be attached by means and using methods well known in the art for linking chemical structures, including, for example, amide, alkylamino, carbamate, urea or ester bonds.

A "P" component of the compounds of the invention (as well as an "L" or an "I" component of the compounds of the invention) can be further modified to label the "P" component (or the "L" or the "I" component) with a detectable substance. Suitable detectable substances include various enzymes, prosthetic groups, fluorescent materials, luminescent materials and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; and examples of suitable radioactive material include $^{14}C$, $^{123}I$, $^{124}I$, $^{125}I$, $^{131}I$, $^{99m}Tc$, $^{35}S$ or $^{3}H$. In a preferred embodiment, a "P" component (or an "L" or an "I" component) is radioactively labeled with $^{14}C$, either by incorporation of $^{14}C$ into the modifying group or one or more amino acid structures in the "P" component (or the "L" or the "I" component). Labeled compounds of the invention can be used to assess the *in vivo* pharmacokinetics of the compounds of the invention, as well as to detect amyloidogenic protein aggregation, for example for diagnostic purposes. Amyloidogenic protein aggregation can be detected either *in vivo* or in an *in vitro* sample derived from a subject.

Preferably, for use as an *in vivo* diagnostic agent, a compound of the invention is labeled (via the "P" or the "L" or the "I" component) with radioactive technetium, *e.g.*, $^{99m}Tc$, or iodine. Methods for labeling peptide compounds with technetium are known in the art (see *e.g.,* U.S. Patent Nos. 5,443,815, 5,225,180 and 5,405,597, all by Dean *et al.*; Stepniak-Biniakiewicz, D., *et al.* (1992) *J. Med. Chem.* 35:274-279; Fritzberg, A.R., *et al.* (1988) *Proc. Natl. Acad Sci. USA* 85:4025-4029; Baidoo, K.E., *et al.* (1990) *Cancer Res. Suppl.* 50:799s-803s; and Regan, L. and Smith, C.K. (1995) *Science* 270:980-982). A modifying group can be chosen that provides a site at which a chelation group for $^{99m}Tc$ can be introduced, such as the Aic derivative of cholic acid, which has a free amino group. In another embodiment, the "P" component (or the "L" or the "I" component) may be labeled with radioactive iodine. For example, a phenylalanine residue within the "P" component (such as $Phe_{19}$ or $Phe_{20}$ within the Aβ sequence) can be substituted with radioactive iodotyrosyl. Any of the various isotopes of radioactive iodine can be incorporated to create a diagnostic agent. Preferably, $^{123}I$ (half-life = 13.2 hours) is used for whole body scintigraphy, $^{124}I$ (half life = 4 days) is used for positron emission tomography (PET), $^{125}I$ (half life = 60 days) is used for metabolic turnover studies and $^{131}I$ (half life = 8 days) is used for whole body counting and delayed low resolution imaging studies.

In one embodiment, a "P" component is prepared in a "prodrug" form, wherein the "P" component itself does not bind an amyloidogenic protein, but rather is capable of being transformed, upon metabolism *in vivo,* into a peptide capable of binding an amyloidogenic protein, as defined herein. A variety of strategies are known in the art for preparing peptide prodrugs that limit metabolism in order to optimize delivery of the active form of the peptide-based drug (see *e.g.,* Moss, J. (1995) in *Peptide-Based Drug Design: Controlling Transport and Metabolism,* Taylor, M.D. and Amidon, G.L. (eds), Chapter 18. Additionally strategies have been specifically tailored to achieving CNS delivery based on "sequential metabolism" (see *e.g.*, Bodor, N., *et al.* (1992) *Science* 257:1698-1700; Prokai, L., *et al.* (1994) *J. Am. Chem.*

*Soc.* 116:2643-2644; Bodor, N. and Prokai, L. (1995) in *Peptide-Based Drug Design: Controlling Transport and Metabolism,* Taylor, M.D. and Amidon, G.L. (eds), Chapter 14. In one embodiment of a prodrug form of a "P" component, the modifying group comprises an alkyl ester to facilitate blood-brain barrier permeability.

Peptides capable of binding an amyloidogenic protein (the "P" component in the compounds of the invention) can be prepared by standard techniques known in the art, such as those described in Bodansky, M. *Principles of Peptide Synthesis,* Springer Verlag, Berlin (1993) and Grant, G.A (ed.). *Synthetic Peptides: A User's Guide,* W.H. Freeman and Company, New York (1992). Automated peptide synthesizers are commercially available (*e.g.*, Advanced ChemTech Model 396; Milligen/ Biosearch 9600). Additionally, one or more modulating groups can be attached to the "P" component by standard methods, for example using methods for reaction through an amino group (*e.g.*, the alpha-amino group at the amino-terminus of a peptide), a carboxyl group (*e.g.,* at the carboxy terminus of a peptide), a hydroxyl group (*e.g.,* on a tyrosine, serine or threonine residue) or other suitable reactive group on an amino acid side chain (see *e.g.*, Greene, T.W and Wuts, P.G.M. *Protective Groups in Organic Synthesis,* John Wiley and Sons, Inc., New York (1991).

Moreover, the compounds of the invention comprising a "P" component may be generated as fusion proteins using standard recombinant DNA techniques, as described in further detail below.

III. Methods for Preparing the Compounds of the Invention

The compounds of the invention may be prepared by any of the well known methods to those of skill in the art. For example, the immunoglobulin heavy chain constant region or a fragment thereof, *e.g.*, a fragment comprising the Fc region, ("I") and the peptide capable of binding an amyloidogenic protein ("P") may be prepared as described in the foregoing sections and chemically crosslinked via a linker group. Numerous chemical crosslinking agents are known in the art (*e.g.*, those commercially available from Pierce, Rockford IL). A crosslinking agent can be chosen which allows for high yield coupling of the immunoglobulin heavy chain constant region to the peptide capable of binding an amyloidogenic protein.

Alternatively, the compounds of the invention may be prepared as fusion proteins using standard recombinant DNA techniques as described in, for example, Sambrook, J., Fritsh, E. F., and Maniatis, T. *Molecular Cloning: A Laboratory Manual. 2nd, ed., Cold Spring Harbor Laboratory,* Cold Spring Harbor Laboratory Press, Cold Spring Harbor, NY, 1989. Briefly, a construct may be generated, in an appropriate expression vector, in which a nucleic acid molecule encoding an immunoglobulin heavy chain constant region or a fragment thereof, *e.g.*, a fragment comprising the Fc region, ("I") is operatively linked to a nucleic acid molecule encoding a peptide capable of binding an amyloidogenic protein ("P"). The term "operatively linked" is intended to indicate that the encoded immunoglobulin heavy chain constant region polypeptide and the peptide capable of binding an amyloidogenic protein are fused in-frame to each other. The immunoglobulin heavy chain constant region polypeptide can be fused to the N-terminus or C-terminus of the peptide capable of binding an amyloidogenic protein, as long as the activity of the resulting compound is retained. For example, the DNA fragments coding for the two polypeptide sequences are ligated together in-frame in accordance with conventional techniques, for example, by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation.

PCR amplification of gene fragments can also be carried out using anchor primers which give rise to complementary overhangs between two consecutive gene fragments which can subsequently be annealed and re-amplified to generate a chimeric gene sequence (see, for example, *Current Protocols in Molecular Biology,* eds. Ausubel *et al.* John Wiley & Sons: 1992).

The resulting expression vector may be introduced into an appropriate host cell to thereby produce the fusion protein. The recombinant expression vectors used can be designed for expression of the fusion protein in prokaryotic or eukaryotic cells. For example, the foregoing fusion proteins can be expressed in bacterial cells such as *E. coli,* insect cells (using baculovirus expression vectors) yeast cells or mammalian cells. Suitable host cells are discussed further in Goeddel, *Gene Expression Technology: Methods in Enzymology* 185, Academic Press, San Diego, CA (1990). Alternatively, the recombinant expression vector can be transcribed and translated *in vitro,* for example using T7 promoter regulatory sequences and T7 polymerase.

The precise site at which the fusion is made is not critical; particular sites are well known and may be selected in order to optimize the biological activity and binding characteristics of the "P" and "I" components of the compounds of the invention. Typically, such fusions retain at least a functionally active CH1, CH2, CH3, and/or hinge domain of the heavy chain constant region of an immunoglobulin heavy chain. In preferred embodiments, the fusion proteins retain a functionally active CH2 domain of the heavy chain constant region of an immunoglobulin heavy chain. Fusions may be made to the C-terminus of the CH3 domain of the heavy chain constant region, or immediately N-terminal to the CH1 domain of the heavy chain constant region. Preferably, P is fused, optionally via L, to the N-terminus of I.

Preferably, I includes the CH2 and CH3 domains and the hinge domain or a portion thereof. In a particularly preferred embodiment, I includes the CH2 and CH3 domains and the hinge domain or a portion thereof, but does not

include the CH1 domain. In this embodiment, P is fused directly or via a linker to the N-terminus of I.

The expression of the fusion proteins of the invention can be achieved using a variety of constructs. Constructs containing a leader sequence either with or without a propeptide express and secrete the fusion proteins directly into the supernatant. In addition, the fusion proteins of the invention can be fused to an extracellular domain of a membrane protein, such as the extracellular domain of the FGF receptor, the TNF receptor or gp 120, for example, with a protease cleavage site in between the two fusions. In a preferred embodiment, the protease cleavage site is an enterokinase site. In this embodiment, the large fusion protein including the extracellular domain is expressed and secreted into the supernatant and the desired fusion protein of the invention can be separated from the extracellular domain by specific cleavage with enterokinase.

In some embodiments the compounds of the invention are assembled as monomers, hetero- or homo-dimers, or as multimers. A basic four chain structural unit is the form in which IgG, IgD, and IgE exist. A four chain unit is repeated in the higher molecular weight immunoglobulins; IgM generally exists as a pentamer of a basic four-chain unit held together by disulfide bonds. IgA globulin, and occasionally IgG globulin, may also exist in a multimeric form in serum. In the case of multimers, each four chain unit may be the same or different.

Preferably, the compounds of the invention are prepared as dimers with two monomeric units linked via disulfide bonds between the two heavy chain constant regions or fragments thereof, *e.g.*, fragments comprising the Fc region. The compounds of the invention may be prepared as homodimers or as heterodimers, *e.g.,* as heterodimers of compounds which contain different "P" components.

## IV. Pharmaceutical Compositions

Another aspect of the invention pertains to pharmaceutical compositions of the compounds of the invention. In one embodiment, the composition includes a compound of the invention in a therapeutically or prophylactically effective amount sufficient to bind an amyloidogenic protein and direct it to a microglial cell, and a pharmaceutically acceptable carrier. A "therapeutically effective amount" refers to an amount effective, at dosages and for periods of time necessary, to achieve the desired therapeutic result, such as reduction of amyloidogenic protein deposition and/or reduction or reversal of amyloidogenic protein related neurotoxicity. A therapeutically effective amount of a compound of the invention may vary according to factors such as the disease state, age, sex, and weight of the subject, and the ability of the compound to elicit a desired response in the subject. Dosage regimens may be adjusted to provide the optimum therapeutic response. A therapeutically effective amount is also one in which any toxic or detrimental effects of the compound are outweighed by the therapeutically beneficial effects. The potential neurotoxicity of the compounds of the invention can be assayed using the assays described herein and a therapeutically effective compound can be selected which does not exhibit significant neurotoxicity. In a preferred embodiment, a therapeutically effective amount of a compound of the invention is sufficient to alter, and preferably inhibit, aggregation of a molar excess amount of amyloidogenic proteins. A "prophylactically effective amount" refers to an amount effective, at dosages and for periods of time necessary, to achieve the desired prophylactic result, such as preventing or inhibiting the rate of amyloidogenic protein deposition and/or amyloidogenic protein associated neurotoxicity in a subject predisposed to amyloidogenic protein deposition. A prophylactically effective amount can be determined as described above for the therapeutically effective amount. Typically, since a prophylactic dose is used in subjects prior to or at an earlier stage of disease, the prophylactically effective amount will be less than the therapeutically effective amount.

One factor that may be considered when determining a therapeutically or prophylactically effective amount of a compound of the invention is the concentration of an amyloidogenic protein, *e.g.*, natural β-AP, in a biological sample obtained from a subject, such as in the cerebrospinal fluid (CSF) of the subject. The concentration of natural β-AP in the CSF has been estimated at 3 nM (Schwartzman, (1994) *Proc. Natl. Acad. Sci. USA* 91:8368-8372). A non-limiting range for a therapeutically or prophylactically effective amount of a compound of the invention is 0.01 nM-10 μM.

It is to be noted that dosage values may vary with the severity of the condition to be alleviated. It is to be further understood that for any particular subject, specific dosage regimens should be adjusted over time according to the individual need and the professional judgment of the person administering or supervising the administration of the compounds, and that dosage ranges set forth herein are exemplary only and are not intended to limit the scope or practice of the claimed composition.

The amount of compound in the composition may vary according to factors such as the disease state, age, sex, and weight of the subject, each of which may affect the amount of amyloidogenic protein in the subject. Dosage regimens may be adjusted to provide the optimum therapeutic response. For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the

required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

As used herein "pharmaceutically acceptable carrier" includes any and all solvents, dispersion media, coatings, antibacterial and antifungal agents, isotonic and absorption delaying agents, and the like that are physiologically compatible. In one embodiment, the carrier is suitable for parenteral administration or for administration via inhalation. Preferably, the carrier is suitable for administration into the central nervous system (*e.g.*, intraspinally or intracerebrally). Alternatively, the carrier can be suitable for intravenous, intraperitoneal or intramuscular administration. In another embodiment, the carrier is suitable for oral administration. Pharmaceutically acceptable carriers include sterile aqueous solutions or dispersions and sterile powders for the extemporaneous preparation of sterile injectable solutions or dispersion. The use of such media and agents for pharmaceutically active substances is well known in the art. Except insofar as any conventional media or agent is incompatible with the active compound, use thereof in the pharmaceutical compositions of the invention is contemplated. Supplementary active compounds can also be incorporated into the compositions.

Therapeutic compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as manitol, sorbitol, or sodium chloride in the composition. Prolonged absorption of the injectable compositions can be brought about by including in the composition an agent which delays absorption, for example, monostearate salts and gelatin. Moreover, the compounds of the invention can be administered in a time release formulation, for example in a composition which includes a slow release polymer. Time release formulations are described in U.S. Patent No. 5,968,895, incorporated herein in its entirety by reference. The active compounds can be prepared with carriers that will protect the compound against rapid release, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, polylactic acid and polylactic, polyglycolic copolymers (PLG). Many methods for the preparation of such formulations are patented or generally known to those skilled in the art.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof

A compound of the invention can be formulated with one or more additional compounds that enhance the solubility of the compound. Preferred compounds to be added to formulations to enhance the solubility of the compounds of the invention are cyclodextrin derivatives, preferably hydroxypropyl-γ-cyclodextrin. Drug delivery vehicles containing a cyclodextrin derivative for delivery of peptides to the central nervous system are described in Bodor, N., *et al.* (1992) *Science* 257:1698-1700. For the compounds described herein, inclusion in the formulation of hydroxypropyl-γ-cyclodextrin at a concentration 50-200 mM may increase the aqueous solubility of the compounds. In addition to increased solubility, inclusion of a cyclodextrin derivative in the formulation may have other beneficial effects, since β-cyclodextrin itself has been reported to interact with an amyloidogenic protein, *e.g.*, the Aβ peptide, and inhibit fibril formation *in vitro* (Camilleri, P., *et al.* (1994) *FEBS Letters* 341:256-258. Accordingly, use of a compound of the invention in combination with a cyclodextrin derivative may result in greater inhibition of Aβ aggregation than use of the compound alone. Chemical modifications of cyclodextrins are known in the art (Hanessian, S., *et al.* (1995) J. Org. Chem. 60:4786-4797).

Another preferred formulation for the compounds of the invention that helps to enhance brain uptake comprises the detergent Tween-80, polyethylene glycol (PEG) and ethanol in a saline solution. A non-limiting example of such a preferred formulation is 0.16% Tween-80, 1.3% PEG-3000 and 2% ethanol in saline.

In another embodiment, a pharmaceutical composition comprising a compound of the invention is formulated such that the compound is transported across the blood-brain barrier (BBB). Various strategies known in the art for increasing transport across the BBB can be adapted to the compounds of the invention to thereby enhance transport of the compounds across the BBB (for reviews of such strategies, see *e.g.*, Pardridge, W.M. (1994) *Trends in Biotechnol.* 12:239-245; Van Bree, J.B. *et al.* (1993) *Pharm. World Sci.* 15:2-9; and Pardridge, W.M. *et al.* (1992) *Pharmacol. Toxicol.* 71:3-10). In one approach, the compound is chemically modified to form a prodrug with enhanced transmembrane

transport. Suitable chemical modifications include covalent linking of a fatty acid to the compound through an amide or ester linkage (see *e.g.*, U.S. Patent 4,933,324 and PCT Publication WO 89/07938, both by Shashoua; U.S. Patent 5,284,876 by Hesse *et al.;* Toth, I. *et al.* (1994) *J. Drug Target.* 2:217-239; and Shashoua, V.E. *et al.* (1984) *J. Med. Chem.* 27:659-664) and glycating the compound (see *e.g.,* U.S. Patent 5,260,308 by Poduslo *et al.*). Also, N-acylamino acid derivatives may be used in a compound to form a "lipidic" prodrug (see *e.g.,* 5,112,863 by Hashimoto *et al.*).

In another approach for enhancing transport across the BBB, a compound is conjugated to a second peptide or protein, thereby forming a chimeric protein, wherein the second peptide or protein undergoes absorptive-mediated or receptor-mediated transcytosis through the BBB. Accordingly, by coupling the compound to this second peptide or protein, the chimeric protein is transported across the BBB. The second peptide or protein can be a ligand for a brain capillary endothelial cell receptor ligand. For example, a preferred ligand is a monoclonal antibody that specifically binds to the transferrin receptor on brain capillary endothelial cells (see *e.g.*, U.S. Patents 5,182,107 and 5,154,924 and PCT Publications WO 93/10819 and WO 95/02421, all by Friden *et al.*). Other suitable peptides or proteins that can mediate transport across the BBB include histones (see *e.g.*, U.S. Patent 4,902,505 by Pardridge and Schimmel) and ligands such as biotin, folate, niacin, pantothenic acid, riboflavin, thiamin, pryridoxal and ascorbic acid (see *e.g.*, U.S. Patents 5,416,016 and 5,108,921, both by Heinstein). Additionally, the glucose transporter GLUT-1 has been reported to transport glycopeptides (L-serinyl-β-D-glucoside analogues of [Met5]enkephalin) across the BBB (Polt, R. *et al.* (1994) *Proc. Natl. Acad Sci. USA* 91:7114-1778). Accordingly, a compound of the invention can be coupled to such a glycopeptide to target the compound to the GLUT-1 glucose transporter. Chimeric proteins can be formed by recombinant DNA methods (*e.g.*, by formation of a chimeric gene encoding a fusion protein) or by chemical crosslinking of the compound to the second peptide or protein to form a chimeric protein, as described above.

In yet another approach for enhancing transport across the BBB, the compound of the invention is encapsulated in a carrier vector which mediates transport across the BBB. For example, the compound can be encapsulated in a liposome, such as a positively charged unilamellar liposome (see *e.g.*, PCT Publications WO 88/07851 and WO 88/07852, both by Faden) or in polymeric microspheres (see *e.g.*, U.S. Patent 5,413,797 by Khan *et al.,* U.S. Patent 5,271,961 by Mathiowitz *et al.* and 5,019,400 by Gombotz *et al.*). Moreover, the carrier can be modified to target it for transport across the BBB. For example, the carrier (*e.g.*, liposome) can be covalently modified with a molecule which is actively transported across the BBB or with a ligand for brain endothelial cell receptors, such as a monoclonal antibody that specifically binds to transferrin receptors (see *e.g.,* PCT Publications WO 91/04014 by Collins *et al.* and WO 94/02178 by Greig *et al.*).

In still another approach to enhancing transport of the compound across the BBB, the compound may be formulated with another agent which functions to permeabilize the BBB. Examples of such BBB "permeabilizers" include bradykinin and bradykinin agonists (see *e.g.*, U.S. Patent 5,112,596 by Malfroy-Camine) and peptidic compounds disclosed in U.S. Patent 5,268,164 by Kozarich *et al.*

Assays that measure the *in vitro* stability of the compounds in cerebrospinal fluid (CSF) and the degree of brain uptake of the compounds in animal models can be used as predictors of *in vivo* efficacy of the compounds. Suitable assays for measuring CSF stability and brain uptake are described in herein.

A compound of the invention can be formulated into a pharmaceutical composition wherein the compound of the invention is the only active compound or, alternatively, the pharmaceutical composition can contain additional active compounds. For example, two or more compounds may be used in combination. Moreover, a compound of the invention can be combined with one or more other agents that have anti-amyloidogenic properties. For example, a compound of the invention can be combined with the non-specific cholinesterase inhibitor tacrine (COGNEX®, Parke-Davis) or with Aricept™, secretase inhibitors, or other agents known to treat a neurological condition.

In another embodiment, a pharmaceutical composition of the invention is provided as a packaged formulation. The packaged formulation may include a pharmaceutical composition of the invention in a container and printed instructions for administration of the composition for treating a subject having an amyloidogenic disorder, *e.g.* Alzheimer's disease.

## V. Methods of Using The Compounds of the Invention

Another aspect of the invention pertains to methods for treating a subject suffering from an amyloidogenic disorder by administering to the subject a therapeutically effective amount of a compound of the invention, thereby treating the subject suffering from an amyloidogenic disorder.

An "amyloidogenic disorder" includes any disease, disorder or condition caused or characterized by deposits of amyloidogenic proteins. Non-limiting examples of amyloidogenic proteins or peptides, and their associated amyloidogenic disorders, include:

Transthyretin (TTR) - Amyloids containing transthyretin occur in familial amyloid polyneuropathy (Portuguese, Japanese and Swedish types), familial amyloid cardiomyopathy (Danish type), isolated cardiac amyloid and systemic senile amyloidosis. Peptide fragments of transthyretin have been shown to form amyloid fibrils *in vitro.* For example,

TTR 10-20 and TTR 105-115 form amyloid-like fibrils in 20-30% acetonitrile/water at room temperature (Jarvis, J.A., *et al.*(1994) *Int. J. Pept. Protein Res.* 44:388-398). Moreover, familial cardiomyopathy (Danish type) is associated with mutation of Leu at position 111 to Met, and an analogue of TTR 105-115 in which the wildtype Leu at position 111 has been substituted with Met (TTR 105-115Met111) also forms amyloid-like fibrils *in vitro* (see *e.g.*, Hermansen, L.F., *et al.* (1995) *Eur. J. Biochem.* 227:772-779; Jarvis *et al. supra*). Peptide fragments of TTR that form amyloid fibrils *in vitro* are also described in Jarvis, J.A., *et al.* (1993) *Biochem. Biophys. Res. Commun.* 192:991-998 and Gustavsson, A., *et al.* (1991) *Biochem. Biophys. Res. Commun.* 175:1159-1164. A peptide fragment of wildtype or mutated transthyretin that forms amyloid fibrils can be used as described herein to create a compound that can be used in the detection or treatment of familial amyloid polyneuropathy (Portuguese, Japanese and Swedish types), familial amyloid cardiomyopathy (Danish type), isolated cardiac amyloid or systemic senile amyloidosis.

Prion Protein (PrP) - Amyloids in a number of spongiform encephalopathies, including scrapie in sheep, bovine spongiform encephalopathy in cows and Creutzfeldt-Jakob disease (CJ) and Gerstmann-Straussler-Scheinker syndrome (GSS) in humans, contain PrP. Limited proteolysis of PrPSc (the prion protein associated with scrapie) leads to a 27-30 kDa fragment (PrP27-30) that polymerizes into rod-shaped amyloids (see *e.g.,* Pan, K.M., *et al.* (1993) *Proc. Natl. Acad. Sci. USA* 90:10962-10966; Gasset, M., *et al.* (1993) *Proc. Natl. Acad. Sci. USA* 90:1-5). Peptide fragments of PrP from humans and other mammals have been shown to form amyloid fibrils *in vitro*. For example, polypeptides corresponding to sequences encoded by normal and mutant alleles of the PRNP gene (encoding the precursor of the prion protein involved in CJ), in the regions of codon 178 and codon 200, spontaneously form amyloid fibrils *in vitro* (see *e.g.*, Goldfarb, L.G., *et al.* (1993) *Proc. Natl. Acad. Sci. USA* 90:4451-4454). A peptide encompassing residues 106-126 of human PrP has been reported to form straight fibrils similar to those extracted from GSS brains, whereas a peptide encompassing residues 127-147 of human PrP has been reported to form twisted fibrils resembling scrapie-associated fibrils (Tagliavini, F., *et al.* (1993) *Proc. Natl. Acad. Sci. USA* 90: 9678-9682). Peptides of Syrian hamster PrP encompassing residues 109-122, 113-127, 113-120, 178-191 or 202-218 have been reported to form amyloid fibrils, with the most amyloidogenic peptide being Ala-Gly-Ala-Ala-Ala-Ala-Gly-Ala (SEQ ID NO:4), which corresponds to residues 113-120 of Syrian hamster PrP but which is also conserved in PrP from other species (Gasset, M., *et al.* (1992) *Proc. Natl. Acad. Sci. USA* 89:10940-10944). A peptide fragment of PrP that forms amyloid fibrils can be used as described herein to create a compound that can be used in the detection or treatment of scrapie, bovine spongiform encephalopathy, Creutzfeldt-Jakob disease or Gerstmann-Straussler-Scheinker syndrome.

Islet Amyloid Polypeptide (IAPP, also known as amylin) - Amyloids containing IAPP occur in adult onset diabetes and insulinoma. IAPP is a 37 amino acid polypeptide formed from an 89 amino acid precursor protein (see *e.g.*, Betsholtz, C., *et al.* (1989) *Exp. Cell. Res.* 183:484-493; Westermark, P., *et al.* (1987) *Proc. Natl. Acad. Sci. USA* 84:3881-3885). A peptide corresponding to IAPP residues 20-29 has been reported to form amyloid-like fibrils *in vitro,* with residues 25-29, having the sequence Ala-Ile-Leu-Ser-Ser (SEQ ID NO:5), being strongly amyloidogenic (Westermark, P., *et al.* (1990) *Proc. Natl. Acad. Sci. USA* 87:5036-5040; Glenner, G.G., *et al.* (1988) *Biochem. Biophys. Res. Commun.* 155:608-614). A peptide fragment of IAPP that forms amyloid fibrils can be used as described herein to create a compound that can be used in the detection or treatment of adult onset diabetes or insulinoma.

Atrial Natriuretic Factor (ANF) - Amyloids containing ANF are associated with isolated atrial amyloid (see *e.g.*, Johansson, B., *et al.* (1987) *Biochem. Biophys. Res. Commun.* 148:1087-1092). ANF corresponds to amino acid residues 99-126 (proANF99-126) of the ANF prohormone (proANP1-126) (Pucci, A., *et al.* (1991) J. Pathol. 165: 235-241). ANF, or a fragment thereof, that forms amyloid fibrils can be used as described herein to create a compound that can be used in the detection or treatment of isolated atrial amyloid.

Kappa or Lambda Light Chain - Amyloids containing kappa or lambda light chains are associated idiopathic (primary) amyloidosis, myeloma or macroglobulinemia-associated amyloidosis, and primary localized cutaneous nodular amyloidosis associated with Sjogren's syndrome. The structure of amyloidogenic kappa and lambda light chains, including amino acid sequence analysis, has been characterized (see *e.g.*, Buxbaum, J.N., *et al.* (1990) *Ann. Intern. Med.* 112:455-464; Schormann, N., *et al.* (1995) *Proc. Natl. Acad. Sci. USA* 92:9490-9494; Hurle, M.R., *et al.* (1994) *Proc. Natl. Acad. Sci. USA* 91:5446-5450; Liepnieks, J.J., *et al.* (1990) *Mol. Immunol.* 27:481-485; Gertz, M.A., *et al.* (1985) *Scand. J. Immunol.* 22:245-250; Inazumi, T., *et al.* (1994) *Dermatology* 189:125-128). Kappa or lambda light chains, or a peptide fragment thereof that forms amyloid fibrils, can be used as described herein to create a compound that can be used in the detection or treatment of idiopathic (primary) amyloidosis, myeloma or macroglobulinemia-associated amyloidosis or primary localized cutaneous nodular amyloidosis associated with Sjogren's syndrome.

Amyloid A - Amyloids containing the amyloid A protein (AA protein), derived from serum amyloid A, are associated with reactive (secondary) amyloidosis (see *e.g.,* Liepnieks, J.J., *et al.* (1995) *Biochim. Biophys. Acta* 1270:81-86), familial Mediterranean Fever and familial amyloid nephropathy with urticaria and deafness (Muckle-Wells syndrome) (see *e.g.*, Linke, R.P., *et al.* (1983) *Lab. Invest.* 48:698-704). Recombinant human serum amyloid A forms amyloid-like *fibrils in vitro* (Yamada, T., *et al.* (1994) *Biochim. Biophys. Acta* 1226:323-329) and circular dichroism studies revealed a predominant β sheet/turn structure (McCubbin, W.D., *et al.* (1988) *Biochem J.* 256:775-783). Serum amyloid A, amyloid A protein or a fragment thereof that forms amyloid fibrils can be used as described herein to create a compound that can be used in the detection or treatment of reactive (secondary) amyloidosis, familial Mediterranean Fever and familial amyloid nephropathy with urticaria and deafness (Muckle-Wells syndrome).

Cystatin C - Amyloids containing a variant of cystatin C are associated with hereditary cerebral hemorrhage with amyloidosis of Icelandic type. The disease is associated with a leucine to glycine mutation at position 68 and cystatin C containing this mutation aggregates *in vitro* (Abrahamson, M. and Grubb, A. (1994) *Proc. Natl. Acad. Sci. USA* 91:1416-1420). Cystatin C or a peptide fragment thereof that forms amyloid fibrils can be used as described herein to create a compound that can be used in the detection or treatment of hereditary cerebral hemorrhage with amyloidosis of Icelandic type.

β2 microglobulin - Amyloids containing β2 microglobulin (β2M) are a major complication of long term hemodialysis (see *e.g.,* Stein, *G., et al.* (1994) Nephrol. Dial. Transplant. 9:48-50; Floege, J., *et al.* (1992) Kidney Int. Suppl. 38: S78-S85; Maury, C.P. (1990) Rheumatol. Int. 10:1-8). The native β2M protein has been shown to form amyloid fibrils *in vitro* (Connors, L.H., *et al.* (1985) Biochem. Biophys. Res. Commun. 131:1063-1068; Ono, K., *et al.* (1994) Nephron 66:404-407). β2M, or a peptide fragment thereof that forms amyloid fibrils, can be used as described herein to create a compound that can be used in the detection or treatment of amyloidosis associated with long term hemodialysis.

Apolipoprotein A-I (ApoA-I) - Amyloids containing variant forms of ApoA-I have been found in hereditary non-neuropathic systemic amyloidosis (familial amyloid polyneuropathy III). For example, N-terminal fragments (residues 1-86, 1-92 and 1-93) of an ApoA-I variant having a Trp to Arg mutation at position 50 have been detected in amyloids (Booth, D.R., *et al.* (1995) *QJM* 88:695-702). In another family, a leucine to arginine mutation at position 60 was found (Soutar, A.K., *et al.* (1992) *Proc. Natl. Acad. Sci. USA* 89:7389-7393). ApoA-I or a peptide fragment thereof that forms amyloid fibrils can be used as described herein to create a compound that can be used in the detection or treatment of hereditary non-neuropathic systemic amyloidosis.

Gelsolin - Amyloids containing variants of gelsolin are associated with familial amyloidosis of Finnish type. Synthetic gelsolin peptides that have sequence homology to wildtype or mutant gelsolins and that form amyloid fibrils *in vitro* are reported in Maury, C.P. *et al.* (1994) *Lab. Invest.* 70:558-564. A nine residue segment surrounding residue 187 (which is mutated in familial gelsolin amyloidosis) was defined as an amyloidogenic region (Maury, *et al., supra*; see also Maury, C.P., *et al.* (1992) *Biochem. Biophys. Res. Commun.* 183:227-231; Maury, C.P. (1991) *J. Clin. Invest.* 87:1195-1199). Gelsolin or a peptide fragment thereof that forms amyloid fibrils can be used as described herein to create a compound that can be used in the detection or treatment of familial amyloidosis of Finnish type.

Procalcitonin or calcitonin - Amyloids containing procalcitonin, calcitonin or calcitonin-like immunoreactivity have been detected in amyloid fibrils associated with medullary carcinoma of the thyroid (see *e.g.*, Butler, M. and Khan, S. (1986) Arch. Pathol. Lab. Med. 110:647-649; Sletten, K., *et al.* (1976) J. Exp. Med. 143:993-998). Calcitonin has been shown to form a nonbranching fibrillar structure *in vitro* (Kedar, I., *et al.* (1976) Isr. J. Med. Sci. 12:1137-1140). Procalcitonin, calcitonin or a fragment thereof that forms amyloid fibrils can be used as described herein to create a compound that can be used in the detection or treatment of amyloidosis associated with medullary carcinoma of the thyroid.

Fibrinogen - Amyloids containing a variant form of fibrinogen alpha-chain have been found in hereditary renal amyloidosis. An arginine to leucine mutation at position 554 has been reported in amyloid fibril protein isolated from postmortem kidney of an affected individual (Benson, M.D., *et al.* (1993) *Nature Genetics* 3:252-255). Fibrinogen alpha-chain or a peptide fragment thereof that forms amyloid fibrils can be used as described herein to create a compound that can be used in the detection or treatment of fibrinogen-associated hereditary renal amyloidosis.

Lysozyme - Amyloids containing a variant form of lysozyme have been found in hereditary systemic amyloidosis. In one family the disease was associated with a threonine to isoleucine mutation at position 56, whereas in another family the disease was associated with a histidine to aspartic acid mutation at position 67 (Pepys, M.B., *et al.* (1993) *Nature* 362:553-557). Lysozyme or a peptide fragment thereof that forms amyloid fibrils can be used as described herein to create a compound that can be used in the detection or treatment of lysozyme-associated hereditary systemic amyloidosis.

The methods of the invention can also be used prophylactically or therapeutically to treat other clinical occurrences of amyloidogenic protein deposition, such as in Down's syndrome individuals and in patients with hereditary cerebral hemorrhage with amyloidosis-Dutch-type (HCHWA-D). Additionally, abnormal accumulation of amyloidogenic proteins, *e.g.*, β-amyloid precursor protein, in muscle fibers has been implicated in the pathology of sporadic inclusion body myositis (IBM) (Askana, V. et al. (1996) *Proc. Natl. Acad. Sci. USA* 93:1314-1319; Askanas, V. et al. (1995) *Current Opinion in Rheumatology* 7:486-496). Accordingly, the compounds of the invention can be used prophylactically or therapeutically in the treatment of disorders in which amyloidogenic proteins are abnormally deposited at non-neurological

locations, such as treatment of IBM by delivery of the compounds to muscle fibers.

The compounds of the invention may be administered to a subject by any suitable route effective for inhibiting amyloidogenic protein aggregation in the subject, although in a particularly preferred embodiment, the compound is administered parenterally, most preferably to the central nervous system of the subject. Possible routes of CNS administration include intraspinal administration and intracerebral administration (*e.g.*, intracerebrovascular administration). Alternatively, the compound can be administered, for example, orally, intraperitoneally, intravenously or intramuscularly. For non-CNS administration routes, the compound can be administered in a formulation which allows for transport across the BBB. Certain compounds may be transported across the BBB without any additional further modification whereas others may need further modification as described above in subsection IV.

Suitable modes and devices for delivery of compounds of the invention to the CNS of a subject are known in the art, including cerebrovascular reservoirs (*e.g.*, Ommaya or Rikker reservoirs; see *e.g.,* Raney, J.P. *et al.* (1988) *J. Neurosci. Nurs.* 20:23-29; Sundaresan, N. *et al.* (1989) *Oncology* 3:15-22), catheters for intrathecal delivery (*e.g.,* Port-a-Cath, Y-catheters and the like; see *e.g.,* Plummer, J.L. (1991) *Pain* 44:215-220; Yaksh, T.L. *et al.* (1986) *Pharmacol. Biochem. Behav.* 25:483-485), injectable intrathecal reservoirs (*e.g.*, Spinalgesic; see *e.g.*, Brazenor, G.A. (1987) *Neurosurgery* 21:484-491), implantable infusion pump systems (*e.g.*, Infusaid; see *e.g.,* Zierski, J. *et al.* (1988) *Acta Neurochem. Suppl.* 43:94-99; Kanoff, R.B. (1994) *J. Am. Osteopath. Assoc.* 94:487-493) and osmotic pumps (sold by Alza Corporation). A particularly preferred mode of administration is via an implantable, externally programmable infusion pump. Suitable infusion pump systems and reservoir systems are also described in U.S. Patent No. 5, 368,562 by Blomquist and U.S. Patent No. 4,731,058 by Doan, developed by Pharmacia Deltec Inc.

The methods of the invention further include administering to a subject a therapeutically effective amount of a compound of the invention in combination with another pharmaceutically active compound known to inhibit amyloidogenic protein deposition, *e.g.*, a cyclodextrin derivative, or in combination with an agent which functions to permeabilize the blood brain barrier (BBB). Examples of such BBB "permeabilizers" include bradykinin and bradykinin agonists (see *e.g.*, U.S. Patent 5,112,596 by Malfroy-Camine) and peptidic compounds disclosed in U.S. Patent 5,268,164 by Kozarich *et al.* Other pharmaceutically active compounds that may be used in the methods of the invention can be found in Harrison's Principles of Internal Medicine, Thirteenth Edition, Eds. T.R. Harrison et al. McGraw-Hill N.Y., NY; and the Physicians Desk Reference 50th Edition 1997, Oradell New Jersey, Medical Economics Co., the complete contents of which are expressly incorporated herein by reference. The compounds of the invention and the additional pharmaceutically active compound may be administered to the subject in the same pharmaceutical composition or in different pharmaceutical compositions (at the same time or at different times).

In yet another embodiment, the present invention provides methods for treating a subject suffering from an amyloidogenic disorder by administering to the subject a recombinant expression vector encoding a compound of the invention such that the compound is synthesized in the subject, thereby treating the subject suffering from an amyloidogenic disorder.

The constructs encoding the compounds of the invention can be inserted into vectors suitable for use as gene therapy vectors. Gene therapy vectors can be delivered to a subject by, for example, intravenous injection, local administration (see U.S. Patent 5,328,470) or by stereotactic injection (see *e.g.,* Chen *et al.* (1994) *Proc. Natl. Acad. Sci. USA* 91:3054-3057). A pharmaceutical preparation of the gene therapy vector may also be administered to a subject. The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e.g.*, retroviral vectors, a pharmaceutical preparation including one or more cells which produce the gene delivery system may be administered to the subject.

In another embodiment, a compound of the invention may be used *in vivo* to detect, and, if desired, quantitate, amyloidogenic protein deposition in a subject to, for example, aid in the diagnosis of an amyloidogenic disorder in the subject. To aid in detection, the compound can be modified with a detectable substance, preferably $^{99m}$Tc or radioactive iodine (described above), which can be detected *in vivo* in a subject. The labeled compound is administered to the subject and, after sufficient time to allow accumulation of the compound at sites of amyloid deposition, the labeled compound is detected by standard imaging techniques. The radioactive signal generated by the labeled compound can be directly detected (*e.g.*, whole body counting), or alternatively, the radioactive signal can be converted into an image on an autoradiograph or on a computer screen to allow for imaging of amyloid deposits in the subject. Methods for imaging amyloidosis using radiolabeled proteins are known in the art. For example, serum amyloid P component (SAP), radiolabeled with either $^{123}$I or $^{99m}$Tc, has been used to image systemic amyloidosis (see *e.g.*, Hawkins, P.N. and Pepys, M.B. (1995) *Eur. J. Nucl. Med.* 22:595-599). Of the various isotypes of radioactive iodine, preferably $^{123}$I (half-life =13.2 hours) is used for whole body scintigraphy, $^{124}$I (half life = 4 days) is used for positron emission tomography (PET), $^{125}$I (half life = 60 days) is used for metabolic turnover studies and $^{131}$I (half life = 8 days) is used for whole body counting and delayed low resolution imaging studies. Analogous to studies using radiolabeled SAP, a labeled compound of the invention can be delivered to a subject by an appropriate route (*e.g.*, intravenously, intraspinally, intracerebrally) in a single bolus, for example containing 100 μg of labeled compound carrying approximately 180 MBq of radioactivity.

VI. Therapeutic Agents Comprising the Formula I-L-P'

In another embodiment, the present invention provides a method of preparing a therapeutic agent comprising the formula I-L-P', where I is an immunoglobulin, *e.g.,* IgG, IgA, IgM, IgD or IgE, heavy chain constant region or fragment thereof; L is a linker group or a direct bond; and P' is a peptide capable of binding a target protein. The method comprises (1) screening a peptide library to identify one or more peptides which bind to the target protein; (2) determining the amino acid sequence of at least one peptide which binds to the target protein; and (3) producing a therapeutic agent comprising a peptide having the amino acid sequence identified in step (2) and an immunoglobulin heavy chain constant region or fragment thereof, linked via a linker group, L, or a direct bond.
In one embodiment, the therapeutic agent is prepared by synthesizing the amino acid sequence identified in step two and conjugating this sequence to an amino acid sequence comprising the Fc region of an immunoglobulin heavy chain constant region using a peptidic linker or a non-peptidic linker, as described above. In this embodiment, the amino acid sequence which binds to the target protein can comprise L-amino acid residues, D-amino acid residues and/or non-natural amino acid residues.
The therapeutic agent comprising the Fc region of an immunoglobulin heavy chain and an amino acid sequence which binds to a target protein can also be a fusion protein, as discussed above. For example, an immunoglobulin heavy chain constant region, or the Fc region thereof, can be directly fused to the amino acid sequence which binds to the target protein, or indirectly fused to the amino acid sequence which binds to the target protein via one or more linking amino acid residues. Such fusion proteins can be prepared as discussed above, for example, via expression of a nucleic acid molecule encoding the fusion protein in a suitable host.
The peptide library screened in step (1) can be a library of L-amino acid peptides, for example, a phage library, a phagemid library or a peptide library produced via synthetic methods, such as those known in the art. A synthetic peptide library can also include peptides comprising D-amino acid residues and non-natural amino acid residues. In one embodiment, the amino acid sequence which binds to the target protein is identified using the methods described in WO 97/22617, the entire contents of which are hereby incorporated by reference. Preferably, the amino acid sequence which binds to the target protein comprises 50 or less, 40 or less, 30 or less, or 25 or less amino acid residues. In a preferred embodiment, the amino acid sequence which binds to the target protein comprises 20 or less, 15 or less, 10 or less, or 7 or less amino acid residues.
The present invention further provides the therapeutic agents prepared using the foregoing method. Preferred therapeutic agents of this type include those in which the amino acid sequence which binds to the target protein is not significantly homologous to a series of contiguous amino acid residues in a protein or peptide which is a naturally occurring ligand of the target protein. As used herein, the term "not significantly homologous" signifies that the degree of homology, or identity between two amino acid sequences is less than 50%.
To determine the percent identity of two amino acid sequences, the sequences are aligned for optimal comparison purposes (*e.g.*, gaps can be introduced in one or both of a first and a second amino acid for optimal alignment and non-identical sequences can be disregarded for comparison purposes). The amino acid residues at corresponding amino acid positions are then compared. When a position in the first sequence is occupied by the same amino acid residue as the corresponding position in the second sequence, then the molecules are identical at that position (as used herein amino acid "identity" is equivalent to amino acid "homology"). The percent identity between the two sequences is a function of the number of identical positions shared by the sequences, taking into account the number of gaps, and the length of each gap, which need to be introduced for optimal alignment of the two sequences.
The comparison of sequences and determination of percent identity between two sequences can be accomplished using a mathematical algorithm. In a preferred embodiment, the percent identity between two amino acid sequences is determined using the Needleman and Wunsch (*J. Mol. Biol.* (48):444-453 (1970)) algorithm which has been incorporated into the GAP program in the GCG software package (available at www.gcg.com), using either a Blosum 62 matrix or a PAM250 matrix, and a gap weight of 16, 14, 12, 10, 8, 6, or 4 and a length weight of 1, 2, 3, 4, 5, or 6. In another embodiment, the percent identity between two amino acid sequences is determined using the algorithm of E. Meyers and W. Miller (*Comput. Appl. Biosci.,* 4:11-17 (1988)) which has been incorporated into the ALIGN program (version 2.0 or version 2.0U), using a PAM120 weight residue table, a gap length penalty of 12 and a gap penalty of 4.
Other preferred therapeutic agents of this type include those in which the peptide which binds to the target protein (P') is a variant of a naturally occurring ligand of the target protein, *e.g.*, a variant in which one or more amino acid residues have been replaced with a non-natural amino acid residue or a D-amino acid residue.
The present invention also features nucleic acid molecules which encode the aforementioned fusion proteins, and recombinant cells which contain a heterologous gene which encodes the aforementioned fusion proteins.
In one embodiment, the amino acid sequence which binds to a target is a naturally occurring sequence, such as a fragment of a ligand which is known to bind the target *in vivo* or *in vitro.* Preferably, the amino acid sequence which binds to the target is derived from the same species as the immunoglobulin heavy chain constant region sequence or fragment thereof. More preferably, both sequences are derived from the species to be treated with the protein. In one

embodiment, both the target binding sequence and the immunoglobulin heavy chain constant region or fragment thereof, such as the Fc region, are both derived from human proteins, that is, both sequences are encoded by the human genome. Preferably the amino acid sequence which binds to a target consists of less than 100 amino acid residues, more preferably less than 50 amino acid residues and, most preferably, about 20 amino acid residues or less.

The target protein can be any protein or peptide. In one embodiment, the protein is associated with a pathogenic organism, and is, preferably, an external or membrane-associated protein, such as a viral coat protein or a bacterial membrane protein. The protein can also be a surface protein of an aberrant cell, such as a cancer cell or other cell which exhibits an unregulated proliferation. Upon administration of a therapeutic agent of the invention to a subject infected with such a pathogenic organism or having such cells which exhibit an unregulated proliferation, the therapeutic agent binds to the target protein and cellular and non-cellular components of the immune system are recruited to the Fc region of the immunoglobulin heavy chain, assisting in the clearance of the pathogenic organism by the subject's immune system.

In another embodiment, the target protein can be a protein that is associated with a disease state, such as a toxin molecule, for example, a bacterial or viral toxin, or a protein which accumulates inappropriately as part of the disease process. Examples of toxin molecules include bacterial endotoxins, such as the *C. difficile* toxins A and B and toxins produced by pathogenic *E. coli* strains.

This invention is further illustrated by the following examples which should not be construed as limiting. The contents of all references, patents and published patent applications cited throughout this application, as well as the figures and the Sequence Listing are hereby incorporated by reference.

## EXAMPLES

### EXAMPLE 1:PREPARATION OF THE COMPOUNDS OF THE INVENTION

Method A:

The peptide capable of binding an amyloidogenic protein and the immunoglobulin heavy chain constant region can be prepared on an Advanced ChemTech Model 396 multiple peptide synthesizer using an automated protocol established by the manufacturer for 0.025 mmole scale synthesis. Double couplings are performed on all cycles using 2-(1H-benzotriazol-1-yl)-1,1,3,3-tetramethyluronium hexafluorophosphate (HBTU)/N,N-diisopropylethylamine (DIEA)/HOBt/FMOC-amino acid in four-fold excess for 30 minutes followed by DIC/HOBt/FMOC-amino acid in four-fold excess for 45 minutes. The peptides are deprotected and removed from the resin by treatment with TFA/water (95 %/5 %) for three hours and precipitated with ether. The pellet is resuspended in 10 % acetic acid and lyophilized. The material is purified by a preparative HPLC using 15 %-40 % acetonitrile over 80 minutes on a Vydac C18 column (21 x 250 mm).

The peptide capable of binding an amyloidogenic protein and the immunoglobulin heavy chain constant region are then linked using a linker group, *e.g.*, a heterobifunctional cross-linker.

Method B:

Expression plasmids encoding the peptide capable of binding an amyloidogenic protein fused to the Fc region of murine IgG2a are constructed by ligating a cDNA sequence encoding the peptide capable of binding an amyloidogenic protein to a genomic DNA segment encoding the hinge-CH2-CH3 domains for an IgG2a.

For production of the fusion protein, the reconstructed sequences are inserted into the pHTOP expression vector (Kaufman, R. J. *et al.* (1991) *Nucleic Acids Res.* 19:4485-4490). The recombinant plasmids are transfected into the CHO cell line and amplified by standard techniques (Kaufman, R. J. *et al.* (1991) *Nucleic Acids Res.* 19:4485-4490). CHO cells expressing the fusion protein are grown in DME/F12 (Life Technologies, Inc.) supplemented with 10% FCS, 0.02 μM methotrexate (Kaufman, R. J. *et al.* (1991) *Nucleic Acids Res.* 19:4485-4490), and 1 mg/ml G418 (Geneticin; Life Technologies, Inc.). At confluence, growth media are discarded, the cells are washed with PBS, and serum-free medium is added. Culture supernatants are collected at 24 hours, clarified by sequential passage through 5.0 and 0.22 μm filters, and concentrated using a 30-kDa tangential flow cartridge filter. The concentrate is loaded onto a protein A-Sepharose Fast Flow column (Pharmacia Biotech), washed with PBS, and eluted with 20 mM citrate (pH 3.0). Elution fractions containing the fusion protein are neutralized with 1 M Tris (pH 8.0; Sigma, St. Louis, MO), and the material is formulated in PBS (pH 7.2) by buffer exchange using a stirred cell with YM30 membrane (Amicon, Beverly, MA). Protein is depyrogenated by chromatography on Poros PI (PerSeptive Biosystems, Framingham, MA). Protein concentration is calculated using absorbance at 280 nm.

### EXAMPLE 2: ASSAY OF COMPOUND STABILITY IN CEREBROSPINAL FLUID

The stability of a compound of the invention in cerebrospinal fluid (CSF) can be assayed in an *in vitro* assay

as follows. A CSF solution is prepared containing 75% Rhesus monkey CSF (commercially available from Northern Biomedical Research), 23% sterile phosphate buffered saline and 2% dimethylsulfoxide (v/v) (Aldrich Chemical Co., Catalog No. 27,685-5). Test compounds are added to the CSF solution to a final concentration of 40 μM or 15 μM. All sample handling is carried out in a laminar flow hood and test solutions are maintained at 37 °C during the assay. After 24 hours, enzymatic activity in the solutions is quenched by adding acetonitrile to produce a final concentration of 25% (v/v). Samples (at the 0 time point and the 24 hour time point) are analyzed at room temperature using reverse-phase HPLC. A microbore column is used to maximize sensitivity. The parameters for analytical HPLC are as follows:

Solvent System

A: 0.1% Trifluoroacetic acid (TFA) in water (v/v)
B: 0.085% TFA/Acetonitrile, 1% $H_2O$ (v/v)

Injection and Gradient

Inject: 100-250 μL of test sample
Run: 10% for B for 5 min., then 10-70% B over 60 min.

Chromatographic analysis is performed using a Hewlett Packard 1090 series II HPLC. The column used for separation is a C4, 5 μm, 1 x 250 mm (Vydac #214TP51). The flow rate is 50 μL/min and the elution profile of the test compounds is monitored at 214, 230, 260 and 280 nm.

**EXAMPLE 3: BRAIN UPTAKE ASSAY**

Brain uptake of test compounds is measured using the technique of Oldendorf (*Brain Research* (1970) 24: 372-376). In this established model, the brain uptake index (BUI) is an estimate of the relative ability of a particular compound to cross the blood-brain barrier, expressed as a percentage of that observed by the freely diffusable reference, water. Radiolabeled compounds are administered to a test animal as a rapid bolus (200 μl) into the left common carotid artery (with the left external carotid artery ligated). The animal is sacrificed 15 seconds later and the amount of radioactivity within the ipsilateral forebrain is determined. The BUI is computed using the equation below:

$$\text{Brain Uptake Index (BUI)} = \frac{\text{(dpm of test compound in brain)/(dpm of water in brain)}}{\text{(dpm of test compound in injectate)/(dpm of water in injectate)}}$$

The vehicle used for the test compounds may be 50 mM cyclodextrin in 75% phosphate buffered saline. Water may be used as the freely diffusable reference, and sucrose may be used as a negative control.

**EXAMPLE 4: Synthesis Of Synthetic Genes Encoding A Fusion Between Fragments Of Human Amyloid Precursor Protein And Immunoglobulin Heavy Chain And Cloning Into A Mammalian Cell Expression Vector**

A fragment of mouse IgG1 was amplified by PCR using a 5' primer (5'-CTGGTTCCGCGTGGATCCGTGCCCAGGGATTGTGGT-3' (SEQ ID NO:6)) and 3' primer (5-ATTAAGCATTCTAGATCATTTACCAGGAGAGTG-3' (SEQ ID NO:7)). This fragment encodes the hinge, CH2 and CH3 regions of mouse IgG1 and is flanked by an in frame BamHI site on its 5' end, and an XbaI site after the termination codon. This fragment was cloned into several common mammalian cell expression vectors, including pCMV4 and pED.

A second gene fragment encoding the leader sequence and pro-peptide from human tissue plasminogen activator (tPA) was assembled by PCR. A conservative mutation was introduced into this fragment to create a unique BssHII restriction site. This fragment was cloned into the pED expression vector.

A new vector was then constructed by a 3-way ligation of the KpnI-BssHII fragment from the tPA vector, a BamHI to KpnI fragment from the IgGI expression vector, and the double stranded synthetic oligonucleotides shown in Figure 3. This vector contains regulatory elements for high level expression in many mammalian cells, including COS, CHO, and 293 cells, a secretary leader sequence from the tPA gene, a fragment encoding the Fc region of mouse IgG1, a unique BssHII within the tPA sequence, and a unique SpeI site between the tPA sequence and IgG1 region. The vector is shown in Figure 4 and is designated pTIg.

A synthetic DNA fragment encoding the β-amyloid portion of the human amyloid precursor protein gene was

assembled using an overlap PCR strategy as indicated in Figure 5. A BssHII site on the 5' end and a SpeI site on the 3' end flanked this fragment. The synthetic β-amyloid fragment was assembled with and without a gly-gly-gly flanking sequence at the 5' end, and terminated either at amino acid 40 of β-amyloid, or amino acid 42 of β-amyloid. The conditions used for PCR assembly and cloning of the synthetic β-amyloid fragment were as follows: A Klenow or PCR amplification was performed for each pair of oligos at an approximately 42°C annealing temperature for 25 cycles, 15" extension for each cycle. For the sequential PCR synthesis, the 217/218 products were mixed with the 219/220 products, or the 219/220 products were mixed with the 221/222 products, and the PCR reaction was repeated with outer primers. These products were then mixed (or one PCR product from this step was mixed with one product from step one) and the PCR reaction was repeated with outer primers for final assembly. The PCR reactions were cleaned up by gel purification if many side products were present or by column purification if the PCR reaction mixture was relatively clean.

The PCR products and the pTIg vector were then digested with BssHII and SpeI, and the two fragments were ligated. The sequence of the assembled synthetic β-amyloid gene was confirmed and contains the codons and restriction endonuclease recognition sites shown in Figure 6.

The synthetic β-amyloid gene was used as template for PCR to generate smaller fragments of the β-amyloid. The PCR gene fragments were cloned as BssHII-SpeI digestion products (as described above for the 1-40 and 1-42 fragments). The fragments encoding pentapeptides of β-amyloid with or without the gly-gly-gly linkers were assembled using complimentary oligonucleotides with BssHII and SpeI overhanging termini. The fragments of the synthetic β-amyloid gene and oligonucleotides used to synthesize those fragments are shown in Figures 7A and 7B. All constructs were confirmed by DNA sequencing.

## EXAMPLE 5:EXPRESSION AND CHARACTERIZATION OF FUSION PROTEINS

The following methods were used in these experiments.

Cell Transfection and Protein Analysis

COS cells were plated on 100 mm dishes in Dulbecco's Modified Eagles Medium (Gibco-BRL) supplemented with 10% fetal bovine serum (Sigma), 4 mM L-glutamine, 50 μg gentamycin, and transfected with HDNA encoding various segments of β-amyloid flanked by the mouse IgG1 Fc region when the cells reached approximately 50% confluency. The transfection reagent was prepared by adding 12 μl of Fugene (Roche) followed by 5 μg plasmid DNA to 0.3 ml of serum-free medium. After incubating for 15 minutes at room temperature, the transfection reagent was added to the medium bathing the cells. The dishes were swirled gently to distribute the reagent. After 24 hours, the medium was removed and the cells were washed once in Dulbecco's Modified Eagles Medium/F12 (Gibco-BRL) supplemented with 4 mM L-glutamine, 0.8 mM L-serine, 0.3 mM L-asparagine, 10 μg/ml insulin, 1.5 μM ferrous sulfate, 100 nM hydrocortisone, 10 mM putrescine, and 28 nM sodium selenite then incubated in 6 ml of the same medium. After 24 hours, the conditioned medium was collected. An aliquot of the medium was added to 6.25X gel loading buffer (final concentration, 50 mM Tris pH 6.8, % SDS, 0.1% bromophenol blue, and 10% glycerol). Cells were lysed in gel loading buffer and collected. Samples were heated to 100°C for 2 minutes and resolved on a 10% SDS-polyacrylamine gel and transferred to polyvinylidine difluoride membrane (Millipore). Membranes were blocked for 1 hour in 5% nonfat dry mil, 1% bovine serum albumin, 0.02% sodium azide in phosphate-buffered saline (PBS), washed three times in PBS containing 0.05% Tween-20 (PBS-T), and incubated for two hours in horse radish peroxidase-conjugated anti-mouse antibody raised in sheep (Amersham) diluted 1/5000 in 1% nonfat dry milk in PBS-T. Blots were visualized by enhanced chemiluminescent using a kit from Roche.

Immunohistochemistry

Twenty micron serial coronal brain sections were prepared from 20-22 week mice transgenic for both the Swedish mutation of amyloid precursor protein and presenilin M146L (Ref.). All incubations were performed at room temperature. Sections were washed once in 100 mM Tris, pH 7.4 (Tris buffer), incubated in 70% formic acid for 3 minutes, then washed three times in Tris buffer. Endogenous peroxidase activity was blocked by incubating for 20 minutes in 10% methanol, 3% hydrogen peroxide in 40 mM Tris, pH 7.4. Sections were washed three times in Tris buffer then incubated in 0.85% NaCl, 0.1% Triton X-100, 100 mM Tris pH 7.4 (Tris buffer A) for 5 minutes followed by buffer B) for 15 minutes. Sections were incubated overnight in conditioned medium from cells expressing secreted fusion proteins or anti-β-amyloid polyclonal antibody diluted 1/1000 in Tris buffer B. Sections were washed twice in Tris buffer A and once in Tris buffer B then incubated with biotin-conjugated anti-mouse antibody raised in donkey from Jackson Laboratories diluted 1/500 in Tris buffer A for 1 hour. Sections were washed twice in Tris buffer A and once in Tris buffer B then incubated with avidin-conjugated horse radish peroxidase using the ABC Immunohistochemistry kit from Vector Laboratories. Sections were washed three times in Tris buffer and stained for 5 minutes with diaminobenzidine hydrochloride from Vector Laboratories. Sections were dehydrated by successive passage through 50% ethanol, 70% ethanol, twice in 95% ethanol, twice in 100% ethanol, and twice in xylene. After applying a coverslip, slides were viewed using an Olympus BX-60 microscope and images were captured with Bioquant software.

Results

Medium harvested from COS cells expressing the Fc Region of mouse IgG1 fused to amino acid residues 1-40, 1-42, 10-25, 16-30, 17-21, or 17-21 (A21L) of β-amyloid with or without an N-terminal triple glycine cap were resolved by SDS polyacrylamide gel electrophoresis in the absence of a reducing agent and examined by Western blot analysis. Probing blots with an anti-mouse antibody demonstrated that each construct yielded a protein that migrated at approximately 75 kDa (Figure 1). The migration of the bands was changed to approximately 35 kDa if β-mercaptoethanol were added to the samples, confirming that the proteins formed dimers via disulfide linkages (data not shown). Higher molecular weight species were detected from some of the fusion proteins and likely represent aggregates not dispersed by SDS. A reduced amount of secreted protein was detected in the medium from cells expressing the Fc domain fused to residues 1-40, 1-42, or 10-25 of β-amyloid.

The medium harvested from COS cells secreting the Fc region of mouse IgG1 fused to various segments of β-amyloid was incubated with coronal brain sections from 20-22 week mice transgenic for both the Swedish mutation of amyloid precursor protein and presenilin M146L and developed with the anti-mouse antibody. Tissue sections incubated with medium from cells expressing segments of β-amyloid fused to the Fc distribution of plaques is consistent with the distribution of amyloid plaques. The intensity of the staining was greatest when residues 17-21 or 16-30 of β-amyloid attached to the Fc region of IgG1 were examined. No amyloid plaques were detected when conditioned medium from nontransfected cells or form cells expressing the Fc domain without a β-amyloid fragment were tested (Figure 2). In addition, plaques were not observed when sections were incubated with purified IgG1. Co-localization of the fusion proteins with amyloid plaques was confirmed by staining adjacent brain sections with either an anti-β-amyloid polyclonal antibody or thioflavin.

**EXAMPLE 6: FIBRIL UPTAKE BY CELLS**

Binding of the fusion protein of Aβ(16-30) and mouse IgG1 Fc (Aβ(16-30)-Fc) to Fc receptors on J774 macrophages and to MCF7 breast cancer cells lacking the Fc receptor was determined by a modification of the procedure by Webster, S.D. *et al.* (2201) *J. Immunol.,* 166, 7496-7503. Briefly, J774 macrophages in Dulbecco's Modified Eagle Medium (Gibco #11960-051) supplemented with 10% fetal bovine serum (Sigma #2442) were plated in 24-well plates at a density of 3 x $10^5$ cells/well and maintained at 37 °C for 24 hours prior to the assay. Aggregated fluorescent β-amyloid$_{1-40}$ (FAβ$_{1-40}$) was prepared by incubating 500 μM Aβ$_{1-40}$ (California Peptide Research, Inc. #641-10) with 30 μM fluorescein-conjugated Aβ$_{1-40}$ (AnaSpec #23513) in 10 mM Hepes (pH 7.4) while stirring overnight at room temperature. FAβ$_{1-40}$ was diluted to 50 μM in phosphate buffered saline (PBS). Test proteins were bound to FAβ$_{1-40}$ by incubating 100 μg of either Aβ(16-30)-Fc, mouse IgG1 Fc only, or αβ-amyloid antibody (Biosource International #44-352), or mouse IgG$_1$ (Sigma #M9269) with 1 ml of FAβ$_{1-40}$ at 37°C for 30 minutes. Protein-bound FAβ$_{1-40}$ was pelleted by centrifugation at 14,000 x g for 5 minutes, washed twice in PBS, and resuspended to 500 μM in PBS. Immediately prior to the assay, the J774 cell medium was replaced with 0.5 ml Dulbecco's Modified Eagle Medium containing 1% BSA. Fucoidan was added to a final concentration of 100 μg/ml and the cells were incubated for 30 minutes at 37°C. In some experiments, 25 μg of αFc receptor antibody (Pharmingen #01241D) was added and the cells were incubated at 37°C for an additional 15 minutes. Cells were moved to 4°C for 30 minutes. Protein-bound FAβ$_{1-40}$ was added at a final concentration of 10 μM and incubated at 4°C for 45 minutes. The solution was removed and cells were washed twice with cold Hepes buffered saline (HBS). Cold trypsin (Gibco #25200-056) was added for 20 minutes at 4°C to dislodge the cells. Cells were resuspended in cold HBS containing 0.1% BSA and analyzed on a Becton Dickinson FACScan analyzer.

The results of this experiment are presented in Figure 8, which presents a graph showing relative cell fluoresence in the presence of each of the proteins, and in the presence and absence of the anti-Fc receptor antibody. The cell fluoresence is equated with fibril uptake by the cells and shows that in the absence of anti-Fc receptor antibody, both the Aβ(16-30)-Fc fusion protein and the α-β-amyloid antibody caused cellular uptake by the J774 macrophage cells, but in the absence of additional protein of the control protein, no fibril uptake was observed. Fibril uptake in the presence of both the Aβ(16-30)-Fc fusion protein and the α-β-amyloid antibody was inhibited by the α-Fc receptor antibody. In contrast, there was no fibril uptake by the MCF7 cells, which lack Fc receptor, under any of the conditions examined. The foregoing results indicate that Fc receptor-mediated fibril uptake occurs in the presence of either the Aβ(16-30)-Fc fusion protein or the α-β-amyloid antibody.

**EXAMPLE 7: FIBRIL BINDING ASSAY**

The ability of a compound of the invention to modulate (*e.g.*, inhibit or promote) the aggregation of natural β-AP when combined with the natural β-AP was examined using the Fibril binding assay. Natural β-AP (β-$AP_{1-40}$) for use in the aggregation assays is commercially available from Bachem (Torrance, CA).

The following materials are needed for the Fibril binding assay: Millipore multifilter apparatus; 12 x 75 glass tubes; GF/F 25 mm glass filters; PBS/0.1% tween 20 at 4°C (PBST); Aβ fibrils; radioactive compound; nonradioactive compound; Eppendorf repeat pipettor with tips; labels; forceps; and vacuum.

In this assay, each sample was run in triplicate. The "aged" Aβ fibril was first prepared approximately 8 days in advance by aging 1 ml aliquots of a 200 μM Aβ 1-40 peptide solution in 4%DMSO/PBS for 8 days at 37°C with rocking. Such "aged" Aβ peptide can be tested directly on cells or frozen at -80°C.

The 200 μM Aβ fibril was diluted in PBST to yield a 4 μM solution (320 μl in 16 ml PBST). 100μL aliquots of this solution were added per tube with the repeat pipettor.

The compound tested (*e.g.*, Aβ(16-30)-Fc, PPI-1019, or PPI-1621) was prepared at 2μM-200 fM dilutions. The compound tested (200 μL) was then added to the appropriate tube containing the Aβ fibril.

The radioactively labeled compound was prepared using standard radioactive safety protocols by making a dilution into a PBS/0.1% tween-20 solution such that there was a final concentration of 20,000 dpm per 100 μL. 100 μl aliqouots of the radioactively labeled compound were added per tube using the repeat pipettor. The samples were covered with parafilm and incubated at 37 °C inside plastic radioactivity bags overnight.

To filter the samples, the filters were pre-wetted in a small volume of PBST. Two Millipore multifiltration apparati were set with GF/F filters in each filtration slot following the instructions from the manufacturer. The samples were removed from the 37 °C incubator and each sample was filtered using a small volume (~5 ml) of cold PBST buffer. The sample tube was then washed with two additional 5 mL volumes of cold PBST buffer. The vacuum was allowed to pull to a semi dry filter for approximately 2 minutes after adding the last sample and the filter was transferred to a labeled tube for iodination counting. One minute counts were recorded, the data was plotted, and the Prism program (GraphPAD) was used to analyze the graph, according to the manufacturer's instructions.

The results from this experiment (set forth in Figure 9), demonstrate that the compounds tested (*e.g.*, PPI-1019, PPI-1621 and three different preparations of Aβ(16-30)-Fc) are effective inhibitors of Aβ aggregation.

**EXAMPLE 8: EXPRESSION OF A FUSION PROTEIN COMPRISING Aβ(16-30) FUSED TO THE N-TERMINUS OF THE HUMAN IGG1 FC DOMAIN**

The expression of the fusion protein comprising Aβ(16-30) fused to the N-terminus of a peptide consisting of the human IgG1 Fc domain and hinge region (Aβ(16-30)-hFc) domain was achieved using an expression construct generated in the pcDNA3.1 vector in which the neomycin resistance gene was replaced with a gene coding for dihydrofolate reductase (DHFR). Expression of the construct was driven by the CMV promoter. The construct further included the tPA signal sequence (amino acid sequence MDAMKRGLCCVLLLCGAVFVSP (SEQ ID NO:8)) fused to amino acids 16-30 of the human beta amyloid sequence (amino acid sequence KLVFFAEDVGSNKGA (SEQ ID NO:9)) fused to the N-terminus of the Fc and hinge region of human IgG1 heavy chain (amino acid sequence beginning EPKSCD...(SEQ ID NO:10)) followed by a BGH polyadenylation signal. The intact fusion protein is 272 amino acids and following processing of the signal sequence, the mature protein was 247 amino acids. Transient expression in Cos, 293 or 293T cells provided a fusion protein that was mainly present as a dimer in non-reduced PAGE analysis and, upon reduction, the monomers were detected.

**EXAMPLE 9*: IN VIVO* EVALUATION OF Aβ(16-30)-FC**

The ability of Aβ(16-30)-Fc to clear amyloid plaques in a mouse model of Alzheimer's disease was assessed. The fusion protein was administered to a mouse transgenic for both the Swedish mutation of amyloid precursor protein and presenilin M146L by direct infusion into the cerebral cortex in one hemisphere. The mouse was sacrificed and the amount of amyloid in brain sections was determined by Thioflavin S staining. As indicated in Figure 10, the plaque burden at the site of infusion was significantly decreased compared to the controlled hemisphere. Brain sections from a mouse that received a protein consisting of the mouse IgG1 Fc region but no amyloid binding sequence exhibited no difference in plaque burden between the two hemispheres.

**EXAMPLE 10: Construction and Analysis of a Synthetic Gene Encoding a Fusion Between Fragments of Human Amyloid Precursor Protein and a Fragment of Human Immunoglobulin G1 Heavy Chain in Mammalian Cell Expression Vectors**

A cDNA fragment of human IgGI encoding the Fc region of the protein, corresponding to amino acids 242-473 of human IgGI heavy chain (sequence accession #CAA75030), was synthesized using RT-PCR. In the process of synthesizing this fragment, a conservative EcoRV restriction endonuclease site was created within the Fc region to facilitate subsequent cloning steps. A second piece of synthetic DNA corresponding to the leader sequence and propeptide from tissue plasminogen activator (tPA) was synthesized and fused to the Fc fragment using PCR. The tPA/Fc fusion was cloned into a derivative of the pcDNA3.1 expression vector in which the neomycin resistance gene was replaced with dihydrofolate reductase. The synthetic gene was designed such that a unique BamHI site flanks the 5'end, and the unique introduced EcoRV site within the Fc region can be replaced with a corresponding BamHI/EcoRV fragment to make additional Fc fusion proteins.

A piece of synthetic DNA encoding amino acids 16-30 of the human beta amyloid peptide was generated using PCR and cloned into the tPA/Fc fusion vector. The propeptide region was deleted from this vector using PCR, yielding the vector ptΔpro16-30hFc. The coding region of the synthetic gene is shown in Figure 11 and SEQ ID NO:11. The amino acid sequence of the encoded protein and annotated functional elements are shown in figure 12 and SEQ ID NO:12.

The vector was transfected into COS and 293T cells using the FuGENE6 reagent (Boehringer Mannheim), and conditioned medium of transiently expressed protein was harvested 48-72 hours later. Alternatively, the vector was transfected in CHO cells and stable cell lines expressing the protein were generated using methotrexate-mediated gene amplification, or co-transfected into 293 cells with a second plasmid containing the neomycin resistance marker, and selected for resistance to G418.

Cell transfection and protein analysis of human constructs

293T cells were plated in 6 well dishes in Dulbecco's Modified Eagles Medium (Gibco-BRL) supplemented with 10% fetal bovine serum (Sigma) and 4 mM L-glutamine and transfected with DNA encoding amino acids 16-30 of β-amyloid fused to the human IgG1 Fc region when the cells reached approximately 70% confluency. The transfection reagent was prepared by adding 3 μl of FuGene (Roche) followed by 1 μg plasmid DNA to 50 μl serum-free medium. After incubating for 15 minutes at room temperature, the transfection reagent was added to the medium bathing the cells. The dishes were swirled gently to distribute the reagent. After 24 hours, the medium was removed and the cells were washed once in Dulbecco's Modified Eagles Medium/F12 (Gibco-BRL) supplemented with 4 mM L-glutamine, 0.8 mM L-serine, 0.3 mM L-asparagine, 10 μg/ml insulin, 1.5 μM ferrous sulfate, 100 nM hydrocortisone, 10 mM putrescine, and 28 nM sodium selenite then incubated in 2 ml of the same medium. After 24 hours, the conditioned medium was collected. An aliquot of the medium was added to 4X gel loading buffer (In Vitrogen). Cells were lysed in gel loading buffer and collected. Samples were heated to 100°C for 2 minutes and resolved on a 10% SDS-polyacrylamide gel and transferred to polyvinyliine defluoride membrane (Millipore). Membranes were blocked for 1 hour in 5% nonfat dry milk containing 0.05% tween-20 in phosphate-buffered saline (PBS) and incubated for one hour with horse radish peroxidase-conjugated anti-human antibody raised in sheep (Amersham) diluted 1/7000 in-5% nonfat dry milk in PBS with 0.05% tween-20. Blots were visualized by enhanced chemiluminescence using a kit from Roche. Proteins were similarly analyzed for their incorporation of the β-amyloid sequences by reacting membranes after blocking with 1/1000 dilution of biotinylated anti-β-amyloid amino acids 17-24 (Signet) in 5% nonfat dry milk in PBS with 0.05% tween-20. The membranes were then washed with PBS with 0.05% tween-20. Following washing, the membranes were incubated with 1/10,000 streptavidin conjugated to horse radish peroxidase (Pierce) in 5% nonfat dry milk in PBS with 0.05% tween-20. The blots were then washed with PBS with 0.05% tween-20 followed by PBS and then visualized by enhanced chemiluminescence using a kit from Roche.

**Equivalents**

Those skilled in the art will recognize, or be able to ascertain using no more than routine experimentation, many equivalents to the specific embodiments of the invention described herein. Such equivalents are intended to be encompassed by the following claims.

## Claims

**1.** A compound comprising the formula I-L-P, wherein:

I is an immunoglobulin heavy chain constant region or fragment thereof that retains the ability to bind an Fc receptor;
L is a linker group or a direct bond; and

P is a peptide capable of binding an amyloidogenic protein.

**2.** The compound of claim 1, wherein I comprises the amino acid sequence set forth in SEQ ID N0:1 or an amino acid sequence having at least 80% identity with the amino acid sequence set forth in SEQ ID N0:1.

**3.** The compound of claim 1, wherein I is an IgG heavy chain constant region or fragment thereof.

**4.** The compound of claim 1, wherein L is a direct bond, or a linker group.

**5.** The compound of claim 1, wherein P is a peptide capable of binding β-amyloid, or an amyloidogenic protein selected from the group consisting of transthyretin (TTR), prion protein (PrP), islet amyloid polypeptide (IAPP), atrial natriuretic factor (ANF), kappa light chain, lambda light chain, amyloid A, procalcitonin, cystatin C, β2 microglobulin, ApoA-I, gelsolin, calcitonin, fibrinogen and lysozyme.

**6.** The compound of claim 1, wherein P comprises 4, 5, 6, 7, 8, 9, 10, 11, 12, 13, 14, 15, 16, 17, 18, 19 or 20 amino acids, or 40 amino acids, preferably 30 amino acids, more preferably 20 amino acids.

**7.** The compound of claim 1, wherein P comprises at least one non-naturally occurring amino acid.

**8.** The compound of claim 1, wherein P comprises at least one D amino acid.

**9.** The compound of claim 5, wherein P comprises a subregion of an amyloidogenic protein selected from the group consisting of transthyretin (TTR), prion protein (PrP), islet amyliod polypeptide (IAPP), atrial natriuretic factor (ANF), kappa light chain, lambda light chain, amyloid A, procalcitonin, cystantin C, β2 microglobulin, ApoA-I, gelsolin, calcitonin, fibrinogen and lysozyme, or a subregion of a natural β-amyloid peptide.

**10.** The compound of claim 5, wherein P is a peptide comprised entirely of D-amino acids and having at least three amino acid residues independently selected from the group consisting of a D-leucine structure, a D-phenylalanine structure, a D-valine structure, a D-tyrosine structure, a D-iodotyrosine structure and a D-alanine structure.

**11.** The compound of the claim 5, wherein P is a peptide comprising the structure

$$(Y\text{-}Xaa_1\text{-}Xaa_2\text{-}Xaa_3\text{-}Xaa_4\text{-}Z)$$

wherein $Xaa_1$, $Xaa_2$, $Xaa_3$ and $Xaa_4$ are each D-amino acid structures and at least two of $Xaa_1$, $Xaa_2$, $Xaa_3$ and $Xaa_4$ are, independently, selected from the group consisting of a D-leucine structure, a D-phenylalanine structure and a D-valine structure;
Y, which may or may not be present, is a structure having the formula $(Xaa)_a$, wherein Xaa is any D-amino acid structure and a is an integer from 1 to 15; and
Z, which may or may not be present, is a structure having the formula $(Xaa)_b$, wherein Xaa is any D-amino acid structure and b is an integer from 1 to 15.

**12.** The compound of claim 5, wherein P is a peptide selected from the group consisting of: D-Leu-D-Val-D-Phe-D-Phe, D-Leu-D-Val-D-Phe-phenethylamide, D-Leu-D-Val-D-Tyr-D-Phe, D-Leu-D-Val-D-IodoTyr-D-Phe, D-Leu-D-Val-Phe-D-Tyr, D-Leu-D-Val-D-Phe-D-IodoTyr, D-Leu-D-Val-D-Phe-D-Ala, D-Leu-D-Val-D-Phe-D-Phe-D-Leu, D-Leu-D-Val-D-Phe-D-Phe-D-Ala, D-Ala-D-Val-D-Phe-D-Phe-D-Leu, D-Leu-D-Val-D-Tyr-D-Phe-D-Ala, D-Leu-D-Val-D-IodoTyr-D-Phe-D-Ala, D-Leu-D-Val-D-Phe-D-Tyr-D-Ala, D-Leu-D-Val-D-Phe-D-IodoTyr-D-Ala, D-Phe-D-Phe-D-Val-D-Leu, D-Ala-D-Phe-D-Phe-D-Val, D-Ala-D-Phe-D-Phe-D-Val-D-Leu, D-Ala-D-Phe-D-Phe-D-Leu-D-Leu, D-Leu-D-Phe-D-Phe-D-Val-D-Leu, D-Phe-D-Phe-D-Phe-D-Val-D-Leu, D-Phe-D-Phe-D-Phe-D-Leu-D-Val, D-Phe-D-Phe-D-Phe-D-Phe-D-Leu, D-Ala-D-Phe-D-Phe-D-Phe-D-Leu, Aβ (16-30), Aβ (10-25), Aβ (1-29), Aβ (1-40), and Aβ (1-42).

**13.** A dimer of the compound of claim 1.

**14.** A pharmaceutical composition comprising a therapeutically effective amount of the compound of claim 1 and a pharmaceutically acceptable carrier.

**15.** Use of a compound of claim 1 for the preparation of a medicament for clearing an amyloidogenic protein from a subject.

**16.** Use of a therapeutically effective amount of the compound of claim 1 for the preparation of a medicament for treating a subject suffering from an amyloidogenic disorder.

**17.** Use according to claim 16, wherein the amyloidogenic disorder is Alzheimer' s disease, or a spongiform encephalopathy.

**18.** A nucleic acid molecule comprising a nucleotide sequence encoding a fusion protein, said fusion protein comprising an immunoglobulin heavy chain constant region, or fragment thereof, that retains the ability to bind an Fc receptor and an amino acid sequence capable of binding to an amyloidogenic protein.

**19.** The nucleic acid molecule of claim 18, wherein the amyloidogenic protein is selected from the group consisting of β-amyloid, transthyretin (TTR), prion protein (PrP), islet amyloid polypeptide (IAPP), atrial natriuretic factor (ANF), kappa light chain, lambda light chain, amyloid A, procalcitonin, cystatin C, β2 microglobulin, ApoA-I, gelsolin, calcitonin, fibrinogen, lysozyme, Huntington, and α-synuclein.

**20.** The nucleic acid molecule of claim 18, wherein the immunoglobulin heavy chain constant region is an IgG heavy chain constant region or fragment thereof.

**21.** The nucleic acid molecule of claim 20, wherein the IgG is a human, canine, bovine, porcine, murine, ovine or rat IgG.

**22.** The nucleic acid molecule of claim 18, wherein the immunoglobulin heavy chain constant region is an IgM, IgA, IgD or IgE heavy chain constant region or fragment thereof.

**23.** The nucleic acid molecule of claim 21, wherein the human IgG is IgG1, IgG2, IgG3 or IgG4.

**24.** The nucleic acid molecule of claim 18, wherein the immunoglobulin heavy chain constant region comprises a functionally active CH2 domain.

**25.** The nucleic acid molecule of claim 18, wherein the amino acid sequence capable of binding to the amyloidogenic protein comprises $A\beta_{1-42}$ or a fragment thereof.

**26.** The nucleic aid molecule of claim 25, wherein the amino acid sequence capable of binding to the amyloidogenic protein comprises at least four, or at least five contiguous amino acid residues from the amino acid sequence of $A\beta_{1-42.}$

**27.** The nucleic acid molecule of claim 25, wherein the amino acid sequence capable of binding to the amyloidogenic protein comprises about 4-15, preferably about 5-10 contiguous amino acid residues from the amino acid sequence $A\beta_{1-42}$.

**28.** The nucleic acid molecule of claim 25, wherein the amino acid sequence capable of binding to the amyloidogenic protein comprises a sequence selected from the group consisting of Leu-Val-Phe-Phe, Leu-Val-Phe-Phe-Ala (SEQ ID NO:3), Leu-Val-Phe-Phe-Leu, Aβ (16-30), Aβ (10-25), Aβ (1-29), Aβ (1-40), and Aβ (1-42).

**29.** The nucleic acid molecule of claim 18, wherein the fusion protein further comprises a linker group of at least one amino acid residue, said linker group linking the immunoglobulin heavy chain constant region and the amino acid sequence capable of binding to an amyloidogenic protein.

**30.** The nucleic acid molecule of claim 29, wherein the linker group comprises about 1-20, preferably 1-10, more preferably 1-5 amino acid residues.

**31.** The nucleic acid molecule of claim 29, wherein the linker group comprises the sequence $-(Gly)_n-$, wherein n is an integer of about 1-10.

**32.** A vector comprising the nucleic acid molecule of any one of claims 17-31.

**33.** A recombinant cell comprising the nucleic acid molecule of any one of claims 17-31.

**34.** The recombinant cell of claim 33, wherein said cell is a mammalian cell.

**35.** The recombinant cell of claim 34, wherein said cell is a CHO cell or a COS cell.

**36.** A method of producing a polypeptide comprising culturing the recombinant cell of claim 33 in an appropriate culture medium to, thereby, produce the polypeptide.

**37.** The polypeptide comprising an immunoglobulin heavy chain constant region, or fragment thereof, that retains the ability to bind an Fc receptor and an amino acid sequence capable of binding to an amyloidogenic protein.

**38.** The polypeptide of claim 37, wherein the amyloidogenic protein is selected from the group consisting of β-amyloid, transthyretin (TTR), prion protein (prP9, islet amyloid polypeptide (IAPP), atrial natriuretic factor (ANF), kappa light chain, lambda light chain, amyloid A, procalcitonin, cystatin C, β2 microglobulin, ApoA-I, gelsolin, calcitonin, fibrinogen, lysozyme, Huntington, and α-synuclein.

**39.** The polypeptide of claim 37 wherein the immunoglobulin heavy chain constant region is an IgG heavy chain constant region or fragment thereof.

**40.** The polypeptide of claim 37 wherein the IgG is a human, canine, bovine, porcine, murine, ovine or rat IgG.

**41.** The polypeptide of claim 37, wherein the immunoglobulin heavy chain constant region is an IgM, IgA, IgD or IgE heavy chain constant region or fragment thereof.

**42.** The polypeptide of claim 40, wherein the human IgG is IgG1, IgG2, IgG3 or IgG4.

**43.** The polypeptide of claim 37, wherein the immunoglobulin heavy chain constant region comprises a functionally active CH2 domain.

**44.** The polypeptide of claim 38, wherein the amyloidogenic protein comprises $A\beta_{1-42}$ or a fragment thereof.

**45.** The polypeptide of claim 44, wherein the amyloidogenic protein comprises at least four, or at least five contiguous amino acid residues from the amino acid sequence of $A\beta_{1-42}$.

**46.** The polypeptide of claim 44, wherein the amyloidogenic protein comprises about 4-15, preferably about 5-10 contiguous amino acid residues from the amino acid sequence of $A\beta_{1-42}$.

**47.** The polypeptide of claim 44, wherein the amyloidogenic protein comprises a sequence selected from the group consisting of Leu-Val-Phe-Phe, Leu-Val-Phe-Phe-Ala (SEQ ID NO:3), Leu-Val-Phe-Phe-Leu, Aβ (16-30), Aβ (10-25), Aβ (1-29), Aβ (1-40), and Aβ (1-42).

**48.** The polypeptide of claim 37, wherein the fusion protein further comprises a linker group of at least one amino acid residue, said linker group linking the immunoglobulin heavy chain constant region and the amino acid sequence capable of binding to an amyloidogenic protein.

**49.** The polypeptide of claim 48, wherein the linker group comprises about 1-20, preferably about 1-10, more preferably about 1-5 amino acid residues.

**50.** The polypeptide of claim 48, wherein the linker group comprises the sequence $-(Gly)_n-$, wherein n is an integer of about 1-10.

**51.** A method of preparing a therapeutic agent comprising the formula I-L-P', wherein I is an immunoglobulin heavy chain constant region or fragment thereof that retains the ability to bind an Fc receptor; L is a linker group or a direct bond; and P' is a peptide capable of binding an amyloidogenic protein, the method comprising:

1) screening a peptide library to identify one or more peptides which bind to the amyloidogenic protein;
2) determining the amino acid sequence of at least one peptide which binds to the amyloidogenic protein; and
3) producing a therapeutic agent comprising a peptide having the amino acid sequence identified in step (2), an immunoglobulin heavy chain constant region or fragment thereof that retains the ability to bind an Fc receptor, and a linker group or a direct bond.

**52.** The method of claim 51, wherein the peptide library comprises L-amino acid peptides, or D-amino acid peptides.

**53.** A therapeutic agent prepared by the method of claim 51.

**54.** A pharmaceutical composition comprising the therapeutic agent of claim 53.

**Patentansprüche**

**1.** Verbindung, umfassend die Formel I-L-P, worin:

I eine konstante Region einer Immunglobulin-Schwere-Kette oder ein Fragment davon, welches die Fähigkeit beibehält, an Fc-Rezeptor zu binden, ist
L eine Linkergruppe oder eine direkte Bindung ist, und
P ein Peptid ist, welches an amyloidogenes Protein binden kann.

**2.** Verbindung nach Anspruch 1, wobei I die in SEQ ID NO: 1 angegebene Aminosäuresequenz oder eine Aminosäuresequenz mit mindestens 80% Identität zu der in SEQ ID NO: 1 angegebenen Aminosäuresequenz umfasst.

**3.** Verbindung nach Anspruch 1, wobei I eine konstante Region einer schweren Kette IgG oder ein Fragment davon ist.

**4.** Verbindung nach Anspruch 1, wobei L eine direkte Bindung oder eine Linkergruppe ist.

**5.** Verbindung nach Anspruch 1, wobei P ein Peptid ist, das an β-Amyloid binden kann, oder ein amyloidogenes Protein, ausgewählt aus der Gruppe, bestehend aus Transthyretin (TTR), Prion-Protein (PrP), Insel-Amyloid-Polypeptid (IAPP), atrialem natriuretischem Faktor (ANF), leichter Kette Kappa, leichter Kette Lambda, Amyloid A, Procalcitonin, Cystatin C, β2 Mikroglobulin, ApoA-I, Gelsolin, Calcitonin, Fibrinogen und Lysozym.

**6.** Verbindung nach Anspruch 1, wobei P 4, 5, 6, 7, 8, 9, 10, 11, 12, 13, 14, 15, 16, 17, 18, 19 oder 20 Aminosäuren, oder 40 Aminosäuren, bevorzugt 30 Aminosäuren, stärker bevorzugt 20 Aminosäuren umfasst.

**7.** Verbindung nach Anspruch 1, wobei P mindestens eine nicht natürlich vorkommende Aminosäure umfasst.

**8.** Verbindung nach Anspruch 1, wobei P mindestens eine D-Aminosäure umfasst.

**9.** Verbindung nach Anspruch 5, wobei P eine Subregion eines amyloidogenen Proteins, ausgewählt aus der Gruppe, bestehend aus Transthyretin (TTR), Prion-Protein (PrP), Insel-Amyloid-Polypeptid (IAPP), atrialem natriuretischem Faktor (ANF), leichter Kette Kappa, leichter Kette Lambda, Amyloid A, Procalcitonin, Cystatin C, β2 Mikroglobulin, ApoA-1, Gelsolin, Calcitonin, Fibrinogen und Lysozym, oder eine Subregion eines natürlichen β-Amyloid-Peptids umfasst.

**10.** Verbindung nach Anspruch 5, wobei P ein Peptid ist, welches vollständig aus D-Aminosäuren zusammengesetzt ist und mindestens drei Aminosäurereste aufweist, die unabhängig voneinander ausgewählt sind aus der Gruppe, bestehend aus einer D-Leucin-Struktur, einer D-Phenylalanin-Struktur, einer D-Valin-Struktur, einer D-Tyrosin-Struktur, einer D-lodtyrosin-Struktur und einer D-Alanin-Struktur.

**11.** Verbindung nach Anspruch 5, wobei P ein Peptid ist, umfassend die Struktur

$$(Y\text{-}Xaa_1\text{-}Xaa_2\text{-}Xaa_3\text{-}Xaa_4\text{-}Z)$$

worin $Xaa_1$, $Xaa_2$, $Xaa_3$ und $Xaa_4$ jeweils D-Aminosäure-Strukturen sind und mindestens zwei aus $Xaa_1$, $Xaa_2$, $Xaa_3$ und $Xaa_4$ unabhängig voneinander aus der Gruppe, bestehend aus einer D-Leucin-Struktur, einer D-Phenylalanin-Struktur und einer D-Valin-Struktur, ausgewählt sind,
Y, das vorhanden sein kann oder auch nicht, eine Struktur mit der Formel $(Xaa)_a$ ist, worin Xaa eine beliebige D-Aminosäure-Struktur ist und a eine ganze Zahl von 1 bis 15 ist, und
Z, das vorhanden sein kann oder auch nicht, eine Struktur mit der Formel $(Xaa)_b$ ist, worin Xaa eine beliebige D-Aminosäure-Struktur ist und b eine ganze Zahl von 1 bis 15 ist.

**12.** Verbindung nach Anspruch 5, wobei P ein Peptid ist, ausgewählt aus der Gruppe, bestehend aus: D-Leu-D-Val-D-Phe-D-Phe, D-Leu-D-Val-D-Phe-D-Phenethylamid, D-Leu-D-Val-D-Tyr-D-Phe, D-Leu-D-Val-D-IodTyr-D-Phe, D-Leu-D-Val-D-Phe-D-Tyr, D-Leu-D-Val-D-Phe-D-IodTyr, D-Leu-D-Val-D-Phe-D-Ala, D-Leu-D-Val-D-Phe-D-Phe-D-Leu, D-Leu-D-Val-D-Phe-D-Phe-D-Ala, D-Ala-D-Val-D-Phe-D-Phe-D-Leu, D-Leu-D-Val-D-Tyr-D-Phe-D-Ala, D-Leu-D-Val-D-IodTyr-D-Phe-D-Ala, D-Leu-D-Val-D-Phe-D-Tyr-D-Ala, D-Leu-D-Val-D-Phe-D-IodTyr-D-Ala, D-Phe-D-Phe-D-Val-D-Leu, D-Ala-D-Phe-D-Phe-D-Val, D-Ala-D-Phe-D-Phe-D-Val-D-Leu-, D-Ala-D-Phe-D-Phe-D-Leu-D-Leu-, D-Leu-D-Phe-D-Phe-D-Val-D-Leu-, D-Phe-D-Phe-D-Phe-D-Val-D-Leu-, D-Phe-D-Phe-D-Phe-D-Leu-D-Val, D-Phe-D-Phe-D-Phe-D-Phe-D-Leu-, D-Ala-D-Phe-D-Phe-D-Phe-D-Leu-, Aβ (16-30), Aβ (10-25), Aβ (1-29), Aβ (1-40) und Aβ (1-42).

**13.** Dimer der Verbindung nach Anspruch 1.

**14.** Pharmazeutische Zusammensetzung, umfassend eine therapeutisch wirksame Menge der Verbindung nach Anspruch 1 und einen pharmazeutisch akzeptablen Träger.

**15.** Verwendung einer Verbindung nach Anspruch 1 zur Herstellung eines Medikaments zur Clearance eines amyloidogenen Proteins aus einem Subjekt.

**16.** Verwendung einer therapeutisch wirksamen Menge der Verbindung nach Anspruch 1 zur Herstellung eines Medikaments für die Behandlung eines Subjekts, das an einer amyloidogenen Störung leidet.

**17.** Verwendung nach Anspruch 16, wobei die amyloidogene Störung Alzheimer-Krankheit oder eine schwammförmige Enzephalopathie ist.

**18.** Nukleinsäuremolekül, umfassend eine Nukleotidsequenz, die für ein Fusionsprotein kodiert, wobei das Fusionsprotein die konstante Region einer Immunglobulin-Schwere-Kette oder ein Fragment davon, welches die Fähigkeit beibehält, an Fc-Rezeptor zu binden, und eine Aminosäuresequenz, welche an ein amyloidogenes Protein binden kann, umfasst.

**19.** Nukleinsäuremolekül nach Anspruch 18, wobei das amyloidogene Protein ausgewählt ist aus der Gruppe, bestehend aus β-Amyloid, Transthyretin (TTR), Prion-Protein (PrP), Insel-Amyloid-Polypeptid (IAPP), atrialem natriuretischem Faktor (ANF), leichter Kette Kappa, leichter Kette Lambda, Amyloid A, Procalcitonin, Cystatin C, β2 Mikroglobulin, ApoA-I, Gelsolin, Calcitonin, Fibrinogen, Lysozym, Huntington und α-Synuclein.

**20.** Nukleinsäuremolekül nach Anspruch 18, wobei die konstante Region einer Immunglobulin-Schwere-Kette eine konstante Region einer schweren Kette IgG oder ein Fragment davon ist.

**21.** Nukleinsäuremolekül nach Anspruch 20, wobei das IgG ein IgG aus Mensch, Hund, Rind, Schwein, Maus, Schaf oder Ratte ist.

**22.** Nukleinsäuremolekül nach Anspruch 18, wobei die konstante Region einer Immunglobulin-Schwere-Kette eine konstante Region einer schweren Kette IgM, IgA, IgD oder IgE oder ein Fragment davon ist.

**23.** Nukleinsäuremolekül nach Anspruch 21, wobei das IgG aus Mensch IgG1, IgG2, IgG3 oder IgG4 ist.

**24.** Nukleinsäuremolekül nach Anspruch 18, wobei die die konstante Region einer Immunglobulin-Schwere-Kette eine funktionell aktive CH2-Domäne umfasst.

**25.** Nukleinsäuremolekül nach Anspruch 18, wobei die Aminosäuresequenz, welche an das amyloidogene Protein binden kann, $A\beta_{1-42}$ oder ein Fragment davon umfasst.

**26.** Nukleinsäuremolekül nach Anspruch 25, wobei die Aminosäuresequenz, welche an das amyloidogene Protein binden kann, mindestens vier oder mindestens fünf aufeinander folgende Aminosäurereste aus der Aminosäuresequenz von $A\beta_{1-42}$ umfasst.

**27.** Nukleinsäuremolekül nach Anspruch 25, wobei die Aminosäuresequenz, welche an das amyloidogene Protein binden kann, etwa 4-15, bevorzugt etwa 5-10 aufeinander folgende Aminosäurereste aus der Aminosäuresequenz von $A\beta_{1-42}$ umfasst.

**28.** Nukleinsäuremolekül nach Anspruch 25, wobei die Aminosäuresequenz, welche an das amyloidogene Protein binden kann, eine Sequenz umfasst, ausgewählt aus der Gruppe, bestehend aus Leu-Val-Phe-Phe, Leu-Val-Phe-Phe-Ala (SEQ ID NO: 3), Leu-Val-Phe-Phe-Leu, Aβ (16-30), Aβ (10-25), Aβ (1-29), Aβ (1-40) und Aβ (1-42).

**29.** Nukleinsäuremolekül nach Anspruch 18, wobei das Fusionsprotein weiterhin eine Linkergruppe aus mindestens einem Aminosäurerest umfasst, wobei die Linkergruppe die konstante Region einer Immunglobulin-Schwere-Kette und die Aminosäuresequenz, welche an ein amyloidogenes Protein binden kann, verbindet.

**30.** Nukleinsäuremolekül nach Anspruch 29, wobei die Linkergruppe etwa 1-20, bevorzugt 1-10, stärker bevorzugt 1-5 Aminosäurereste umfasst.

**31.** Nukleinsäuremolekül nach Anspruch 29, wobei die Linkergruppe die Sequenz - $(Gly)_n$- umfasst, wobei n eine ganze Zahl von etwa 1-10 ist.

**32.** Vektor, umfassend das Nukleinsäuremolekül nach einem der Ansprüche 17-31.

**33.** Rekombinante Zelle, umfassend das Nukleinsäuremolekül nach einem der Ansprüche 17-31.

**34.** Rekombinante Zelle nach Anspruch 33, wobei die Zelle eine Säugerzelle ist.

**35.** Rekombinante Zelle nach Anspruch 34, wobei die Zelle eine CHO-Zelle oder eine COS-Zelle ist.

**36.** Verfahren zur Herstellung eines Polypeptids, umfassend das Kultivieren der rekombinanten Zelle nach Anspruch 33 in einem geeigneten Kulturmedium, um **dadurch** das Polypeptid zu produzieren.

**37.** Polypeptid, umfassend eine konstante Region einer Immunglobulin-Schwere-Kette oder ein Fragment davon, welches die Fähigkeit beibehält, an Fc-Rezeptor zu binden, und eine Aminosäuresequenz, welche an ein amyloidogenes Protein binden kann.

**38.** Polypeptid nach Anspruch 37, wobei das amyloidogene Protein ausgewählt ist aus der Gruppe, bestehend aus β-Amyloid, Transthyretin (TTR), Prion-Protein (PrP), Insel-Amyloid-Polypeptid (IAPP), atrialem natriuretischem Faktor (ANF), leichter Kette Kappa, leichter Kette Lambda, Amyloid A, Procalcitonin, Cystatin C, β2 Mikroglobulin, ApoA-I, Gelsolin, Calcitonin, Fibrinogen, Lysozym, Huntington und α-Synuclein.

**39.** Polypeptid nach Anspruch 37, wobei die konstante Region einer Immunglobulin-Schwere-Kette eine konstante Region einer schweren Kette IgG oder ein Fragment davon ist.

**40.** Polypeptid nach Anspruch 37, wobei das IgG ein IgG aus Mensch, Hund, Rind, Schwein, Maus, Schaf oder Ratte ist.

**41.** Polypeptid nach Anspruch 37, wobei die konstante Region einer Immunglobulin-Schwere-Kette eine konstante Region einer schweren Kette IgM, IgA, IgD oder IgE oder ein Fragment davon ist.

**42.** Polypeptid nach Anspruch 40, wobei das IgG aus Mensch IgG1, IgG2, IgG3 oder IgG4 ist.

**43.** Polypeptid nach Anspruch 37, wobei die konstante Region einer Immunglobulin-Schwere-Kette eine funktionell aktive CH2-Domäne umfasst.

**44.** Polypeptid nach Anspruch 38, wobei das amyloidogene Protein $A\beta_{1-42}$ oder ein Fragment davon umfasst.

**45.** Polypeptid nach Anspruch 44, wobei das amyloidogene Protein mindestens vier oder mindestens fünf aufeinander folgende Aminosäurereste aus der Aminosäuresequenz von $A\beta_{1-42}$ umfasst.

**46.** Polypeptid nach Anspruch 44, wobei das amyloidogene Protein etwa 4-15, bevorzugt etwa 5-10 aufeinander folgende Aminosäurereste aus der Aminosäuresequenz von $A\beta_{1-42}$ umfasst.

**47.** Polypeptid nach Anspruch 44, wobei das amyloidogene Protein eine Sequenz umfasst, ausgewählt aus der Gruppe, bestehend aus Leu-Val-Phe-Phe, Leu-Val-Phe-Phe-Ala (SEQ ID NO: 3), Leu-Val-Phe-Phe-Leu, Aβ (16-30), Aβ (10-25), Aβ (1-29), Aβ (1-40) und Aβ (1-42).

**48.** Polypeptid nach Anspruch 37, wobei das Fusionsprotein weiterhin eine Linkergruppe aus mindestens einem Aminosäurerest umfasst, wobei die Linkergruppe die konstante Region einer Immunglobulin-Schwere-Kette und die Aminosäuresequenz, welche an ein amyloidogenes Protein binden kann, verbindet.

**49.** Polypeptid nach Anspruch 48, wobei die Linkergruppe etwa 1-20, bevorzugt etwa 1-10, stärker bevorzugt etwa 1-5 Aminosäurereste umfasst.

**50.** Polypeptid nach Anspruch 48, wobei die Linkergruppe die Sequenz $-(Gly)_n-$ umfasst, wobei n eine ganze Zahl von etwa 1-10 ist.

**51.** Verfahren zur Herstellung eines therapeutischen Mittels, umfassend die Formel I-L-P, worin I die konstante Region einer Immunglobulin-Schwere-Kette oder ein Fragment davon ist, welches die Fähigkeit beibehält, an Fc-Rezeptor zu binden, L eine Linkergruppe oder eine direkte Bindung ist, und P ein Peptid ist, welches an ein amyloidogenes Protein binden kann, wobei das Verfahren umfasst:

1) Screenen einer Peptidbibliothek, um ein oder mehrere Peptide zu identifizieren, welche an das amyloidogene Protein binden,
2) Bestimmen der Aminosäuresequenz von mindestens einem Peptid, welches an das amyloidogene Protein bindet, und
3) Herstellen eines therapeutischen Mittels, umfassend ein Peptid mit der in Schritt (2) identifizierten Aminosäuresequenz, eine konstante Region einer Immunglobulin-Schwere-Kette oder ein Fragment davon, welches die Fähigkeit beibehält, an Fc-Rezeptor zu binden, und eine Linkergruppe oder eine direkte Bindung.

**52.** Verfahren nach Anspruch 51, wobei die Peptidbibliothek L-Aminosäurepeptide oder D-Aminosäurepeptide umfasst.

**53.** Therapeutisches Mittel, hergestellt durch das Verfahren nach Anspruch 51.

**54.** Pharmazeutische Zusammensetzung, umfassend das therapeutische Mittel nach Anspruch 53.

## Revendications

**1.** Composé comprenant la formule I-L-P, dans lequel :

I est une région constante de chaîne lourde d'immunoglobuline ou un fragment de celle-ci qui conserve la faculté de lier un récepteur Fc ;
L est un groupe liant ou un lien direct ; et
P est un peptide capable de lier une protéine amyloïdogénique.

**2.** Composé selon la revendication 1, dans lequel I comprend la séquence d'aminoacide présentée dans SEQ ID N0: 1 1 ou une séquence d'aminoacide ayant au moins 80 % d'identité avec la séquence d'aminoacide présentée dans SEQ ID NO:1.

**3.** Composé selon la revendication 1, dans lequel I est une région constante de chaîne lourde de IgG ou un fragment de celle-ci.

**4.** Composé selon la revendication 1, dans lequel L est un lien direct, ou un groupe liant.

**5.** Composé selon la revendication 1, dans lequel P est un peptide capable de lier un β-amyloïde, ou une protéine amyloïdogénique sélectionnée parmi le groupe constitué de la transthyrétine (TTR), la protéine prion (PrP), l'amyline (IAPP), le facteur natriurétique auriculaire (ANF), une chaîne légère kappa, une chaîne légère lambda, l'amyloïde A, la procalcitonine, la cystatine C, la β2-microglobuline, le ApoA-I, la gelsoline, la calcitonine, le fibrinogène et le lysozyme.

**6.** Composé selon la revendication 1, dans lequel P comprend 4, 5, 6, 7, 8, 9, 10, 11, 12, 13, 14, 15, 16, 17, 18, 19 ou 20 aminoacides, ou 40 aminoacides, de préférence 30 aminoacides, de manière davantage préférée 20 aminoacides.

**7.** Composé selon la revendication 1, dans lequel P comprend au moins un acide produit non naturellement.

**8.** Composé selon la revendication 1, dans lequel P comprend au moins un aminoacide D.

**9.** Composé selon la revendication 5, dans lequel P comprend une sous-région d'une protéine amyloïdogénique sélectionnée parmi le groupe constitué de la transthyrétine (TTR), la protéine prion (PrP), l'amyline (IAPP), le facteur natriurétique auriculaire (ANF), une chaîne légère kappa, une chaîne légère lambda, l'amyloïde A, la procalcitonine, la cystatine C, la β2-microglobuline, le ApoA-I, la gelsoline, la calcitonine, le fibrinogène et le lysozyme, ou une sous-région d'un peptide β-amyloïde naturel.

**10.** Composé selon la revendication 5, dans lequel P est un peptide composé entièrement de D-aminoacides et ayant au moins trois restes d'aminoacides sélectionnés indépendamment du groupe constitué par une structure D-leucine, une structure D-phénylalanine, une structure D-valine, une structure D-tyrosine, une structure D-iodotyrosine et une structure D-alanine.

**11.** Composé selon la revendication 5, dans lequel P est un peptide comprenant la structure

$$(Y\text{-}Xaa_1\text{-}Xaa_2\text{-}Xaa_3\text{-}Xaa_4\text{-}Z)$$

dans laquelle $Xaa_1$, $Xaa_2$, $Xaa_3$ et $Xaa_4$ sont chacun des structures D-aminoacides et au moins deux de $Xaa_1$, $Xaa_2$, $Xaa_3$ et $Xaa_4$ sont, indépendamment, sélectionnés parmi le groupe constitué par une structure D-leucine, une structure D-phénylalanine et une structure D-valine ;
Y, qui peut être ou ne pas être présent, est une structure de formule $(Xaa)_a$, où Xaa est une structure quelconque D-aminoacide et a est un entier compris entre 1 et 15 ; et
Z, qui peut être ou ne pas être présent, est une structure de formule $(Xaa)_b$, où Xaa est une structure quelconque D-aminoacide et b est un entier compris entre 1 et 15.

**12.** Composé selon la revendication 5, dans lequel P est un peptide sélectionné parmi le groupe constitué par : D-Leu-D-Val-D-Phe-D-Phe,D-Leu-D-Val-D-Phe-phenéthylamide, D-Leu-D-Val-D-Tyr-D-Phe, D-Leu-D-Val-D-IodoTyr-D-Phe, D-Leu-D-Val-Phe-D-Tyr, D-Leu-D-Val-D-Phe-D-IodoTyr, D-Leu-D-Val-D-Phe-D-Ala, D-Leu-D-Val-D-Phe-D-Phe-D-Leu, D-Leu-D-Val-D-Tyr-D-Phe-D-Ala, D-Leu-D-Val-D-IodoTyr-D-Phe-D-Ala, D-Leu-D-Val-D-Phe-D-Tyr-D-Ala, D-Leu-D-Val-D-Phe-D-IodoTyr-D-Ala, D-Phe-D-Phe-D-Val-D-Leu, D-Ala-D-Phe-D-Phe-D-Val, D-Ala-D-Phe-D-Phe-D-Val-D-Leu, D-Ala-D-Phe-D-Phe-D-Leu-D-Leu, D-Leu-D-Phe-D-Phe-D-Val-D-Leu, D-Phe-D-Phe-D-Phe-D-Val-D-Leu, D-Phe-D-Phe-D-Phe-D-Leu-D-Val, D-Phe-D-Phe-D-Phe-D-Phe-D-Leu, D-Ala-D-Phe-D-Phe-D-Phe-D-Leu, Aβ (16-30), Aβ (10-25), Aβ (1-29), Aβ (1-40), et Aβ (1-42).

**13.** Un dimère du composé selon la revendication 1.

**14.** Composition pharmaceutique comprenant un volume thérapeutiquement efficace du composé selon la revendication 1 et un vecteur pharmaceutiquement acceptable.

**15.** Utilisation d'un composé selon la revendication 1 pour la préparation d'un médicament pour éliminer une protéine amyloïdogénique d'un sujet.

**16.** Utilisation d'un volume thérapeutiquement efficace du composé selon la revendication 1 pour la préparation d'un médicament visant à traiter un sujet souffrant d'un trouble amyloïdogénique.

**17.** Utilisation selon la revendication 16, dans laquelle le trouble amyloïdogénique est la maladie d'Alzheimer, ou une encéphalopathie spongiforme.

**18.** Molécule d'acide nucléique comprenant une séquence nucléotidique encodant une protéine hybride, ladite protéine hybride comprenant une région constante de chaîne lourde d'immunoglobuline, ou un fragment de celle-ci, qui conserve la faculté de lier un récepteur Fc et une séquence aminoacide capable de se lier à une protéine amyloïdogénique.

**19.** Molécule d'acide nucléique selon la revendication 18, dans laquelle la protéine amyloïdogénique est sélectionnée parmi le groupe constitué par la β-amyloïde, la transthyrétine (TTR), la protéine prion (PrP), l'amyline (IAPP), le facteur natriurétique auriculaire (ANF), une chaîne légère kappa, une chaîne légère lambda, l'amyloïde A, la procalcitonine, la cystatine C, la β2-microglobuline, le ApoA-I, la gelsoline, la calcitonine, le fibrinogène et le lysozyme, le Huntington et l'α-synucléine.

20. Molécule d'acide nucléique selon la revendication 18, dans laquelle la région constante de chaîne lourde d'immunoglobuline est une région constante de chaîne lourde d'IgG ou un fragment de celle-ci.

21. Molécule d'acide nucléique selon la revendication 20, dans laquelle l'IgG est une IgG humaine, canine, bovine, porcine, murine, ovine ou de rat.

22. Molécule d'acide nucléique selon la revendication 18, dans laquelle la région constante de chaîne lourde d'immunoglobuline est une région constante de chaîne lourde d'IgM, d'IgA, d'IgD ou d'IgE ou un fragment de celle-ci.

23. Molécule d'acide nucléique selon la revendication 21, dans laquelle l'IgG humaine est l'IgG1, l'IgG2, l'IgG3 ou l'IgG4.

24. Molécule d'acide nucléique selon la revendication 18, dans laquelle la région constante de chaîne lourde d'immunoglobuline comprend un domaine CH2 fonctionnellement actif.

25. Molécule d'acide nucléique selon la revendication 18, dans laquelle la séquence aminoacide capable de se lier à la protéine amyloïdogénique comprend $A\beta_{1-42}$ ou un fragment de celui-ci.

26. Molécule d'acide nucléique selon la revendication 25, dans laquelle la séquence aminoacide capable de se lier à la protéine amyloïdogénique comprend au moins quatre, ou au moins cinq résidus aminoacides contigus de la séquence aminoacide de $A\beta_{1-42}$.

27. Molécule d'acide nucléique selon la revendication 25, dans laquelle la séquence aminoacide capable de se lier à la protéine amyloïdogénique comprend environ 4 à 15, de préférence 5 à 10 résidus aminoacides contigus de la séquence aminoacide de $A\beta_{1-42}$.

28. Molécule d'acide nucléique selon la revendication 25, dans laquelle la séquence aminoacide capable de se lier à la protéine amyloïdogénique comprend une séquence sélectionnée parmi le groupe constitué par Leu-Val-Phe-Phe, Leu-Val-Phe-Phe-Ala (SEQ ID NO:3), Leu-Val-Phe-Phe-Leu, Aβ (16-30), Aβ (10-25), Aβ (1-29), Aβ (1- 40), et Aβ (1-42).

29. Molécule d'acide nucléique selon la revendication 18, dans laquelle la protéine hybride comprend en outre un groupe liant d'au moins un résidu aminoacide, ledit groupe liant la région constante de la chaîne lourde d'immunoglobuline et la séquence aminoacide capable de se lier à une protéine amyloïdogénique.

30. Molécule d'acide nucléique selon la revendication 29, dans laquelle le groupe liant comprend environ 1-20, de préférence 1-10, de manière davantage préférée 1-5 résidus aminoacides.

31. Molécule d'acide nucléique selon la revendication 29, dans laquelle le groupe liant comprend la séquence $-(Gly)_n-$, où n est un entier d'environ 1 à 10.

32. Vecteur comprenant la molécule d'acide nucléique selon l'une quelconque des revendications 17-31.

33. Cellule recombinante comprenant la molécule d'acide nucléique selon l'une quelconque des revendications 17-31.

34. Cellule recombinante selon la revendication 33, dans laquelle ladite cellule est une cellule de mammifère.

35. Cellule recombinante selon la revendication 34, dans laquelle ladite cellule est une cellule CHO ou une cellule COS.

36. Procédé de production d'un polypeptide consistant à cultiver la cellule recombinante selon la revendication 33 dans un milieu de culture approprié pour, ainsi, produire le polypeptide.

37. Polypeptide comprenant une région constante de chaîne lourde d'immunoglobuline, ou un fragment de celle-ci, qui conserve la faculté de se lier à un récepteur Fc et une séquence aminoacide capable de se lier à une protéine amyloïdogénique.

**38.** Polypeptide selon la revendication 37, dans lequel la protéine amyloïdogénique est sélectionnée parmi le groupe constitué par la β-amyloïde, la transthyrétine (TTR), la protéine prion (PrP), l'amyline (IAPP), le facteur natriurétique auriculaire (ANF), une chaîne légère kappa, une chaîne légère lambda, l'amyloïde A, la procalcitonine, la cystatine C, la β2-microglobuline, le ApoA-I, la gelsoline, la calcitonine, le fibrinogène et le lysozyme, le Huntington et l'α-synucléine.

**39.** Polypeptide selon la revendication 37, dans lequel la région constante de chaîne lourde d'immunoglobuline est une région constante de chaîne lourde d' IgG ou un fragment de celle-ci.

**40.** Polypeptide selon la revendication 37, dans lequel l'IgG est une IgG humaine, canine, bovine, porcine, murine, ovine ou de rat.

**41.** Polypeptide selon la revendication 37, dans lequel la région constante de chaîne lourde d'immunoglobuline est une région constante de chaîne lourde d'IgM, d'IgA, d'IgD ou d'IgE ou un fragment de celle-ci.

**42.** Polypeptide selon la revendication 37, dans lequel l'IgG humaine est l' IgG1, l'IgG2, l'IgG3 ou l'IgG4.

**43.** Polypeptide selon la revendication 37, dans lequel la région constante de chaîne lourde, d'immunoglobuline comprend un domaine CH2 fonctionnellement actif.

**44.** Polypeptide selon la revendication 37, dans lequel la protéine amyloïdogénique comprend $A\beta_{1-42}$ ou un fragment de celui-ci.

**45.** Polypeptide selon la revendication 44, dans lequel la protéine amyloïdogénique comprend au moins quatre, ou au moins cinq résidus aminoacides contigus de la séquence aminoacide de $A\beta_{1-42}$.

**46.** Polypeptide selon la revendication 44, dans lequel la protéine amyloïdogénique comprend environ 4 à 15, de préférence 5 à 10 résidus aminoacides contigus de la séquence aminoacide de $A\beta_{1-42}$.

**47.** Polypeptide selon la revendication 44, dans lequel la protéine amyloïdogénique comprend une séquence sélectionnée parmi le groupe constitué par Leu-Val-Phe-Phe, Leu-Val-Phe-Phe-Ala (SEQ ID NO:3), Leu-Val-Phe-Phe-Leu, Aβ (16-30), Aβ (10-25), Aβ (1-29), Aβ (1- 40), et Aβ (1-42).

**48.** Polypeptide selon la revendication 37, dans lequel la protéine hybride comprend en outre un groupe liant d'au moins un résidu aminoacide, ledit groupe liant la région constante de la chaîne lourde d'immunoglobuline et la séquence aminoacide capable de se lier à une protéine amyloïdogénique.

**49.** Polypeptide selon la revendication 48, dans lequel le groupe liant comprend environ 1-20, de préférence 1-10, de manière davantage préférée 1-5 résidus aminoacides.

**50.** Polypeptide selon la revendication 48, dans lequel le groupe liant comprend la séquence $-(Gly)_n-$, où n est un entier d'environ 1 à 10.

**51.** Procédé de préparation d'un agent thérapeutique comprenant la formule I-L-P, dans lequel I est une région constante de chaîne lourde d'immunoglobuline ou un fragment de celle-ci qui conserve la faculté de lier un récepteur Fc ; L est un groupe liant ou un lien direct ; et P est un peptide capable de lier une protéine amyloïdogénique, comprenant les étapes consistant à :

1) analyser une bibliothèque de peptides pour identifier un ou plusieurs peptides qui se lient à la protéine amyloïdogénique ;
2) déterminer la séquence aminoacide d'au moins un peptide qui se lie à la protéine amyloïdogénique ; et
3) produire un agent thérapeutique comprenant un peptide ayant la séquence aminoacide identifiée à l'étape 2, une région constante de chaîne lourde d'immunoglobuline ou un fragment de celle-ci qui conserve la faculté de lier un récepteur Fc et un groupe liant ou un lien direct.

**52.** Procédé selon la revendication 51, dans lequel la bibliothèque de peptides comprend des peptides de L-aminoacides, ou des peptides de D-aminoacides.

**53.** Agent thérapeutique préparé par le procédé selon la revendication 51.

**54.** Composition pharmaceutique comprenant l'agent thérapeutique selon la revendication 53.

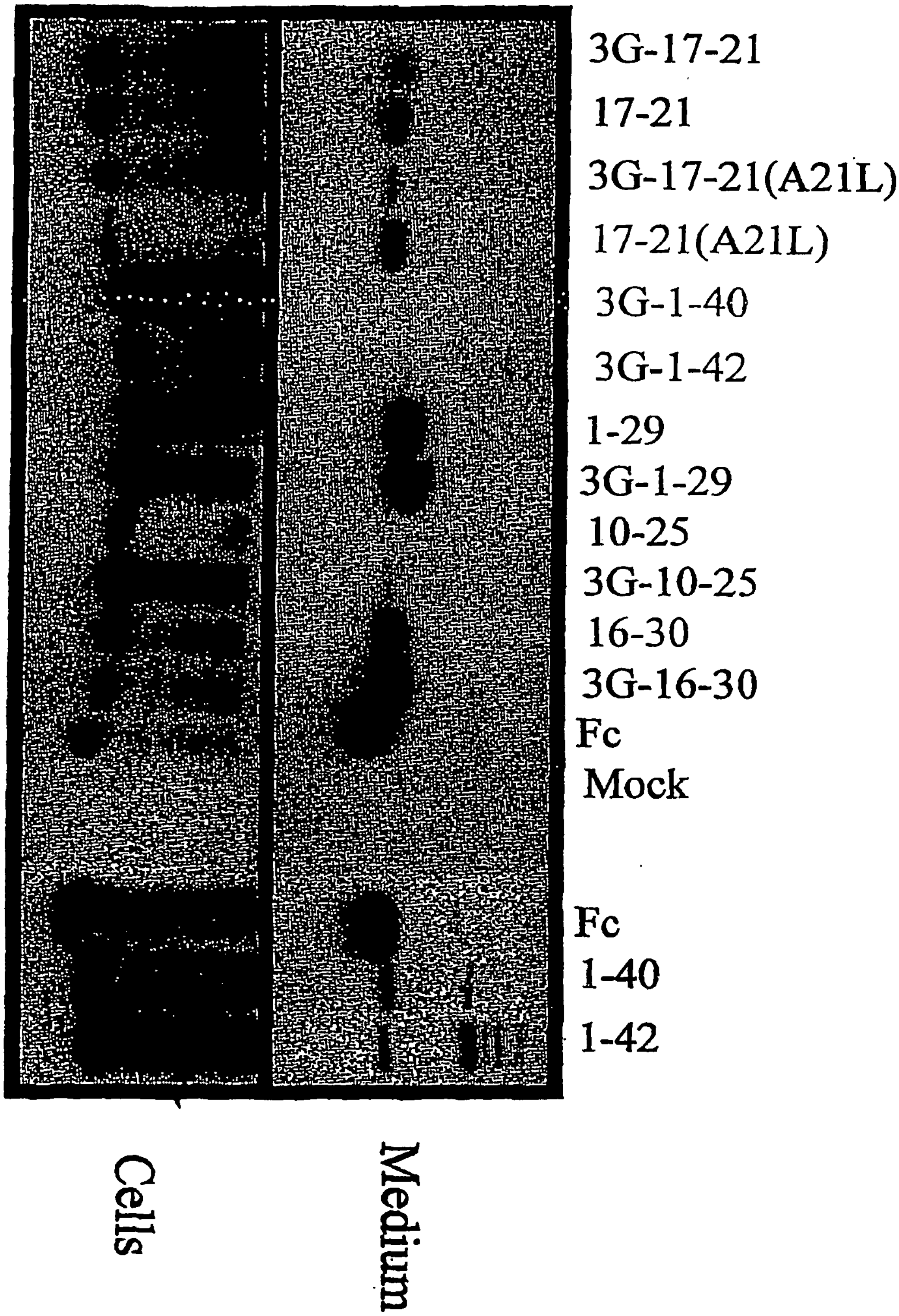
3G-17-21
17-21
3G-17-21(A21L)
17-21(A21L)
3G-1-40
3G-1-42
1-29
3G-1-29
10-25
3G-10-25
16-30
3G-16-30
Fc
Mock
Fc
1-40
1-42
Cells
Medium

**FIGURE 1**

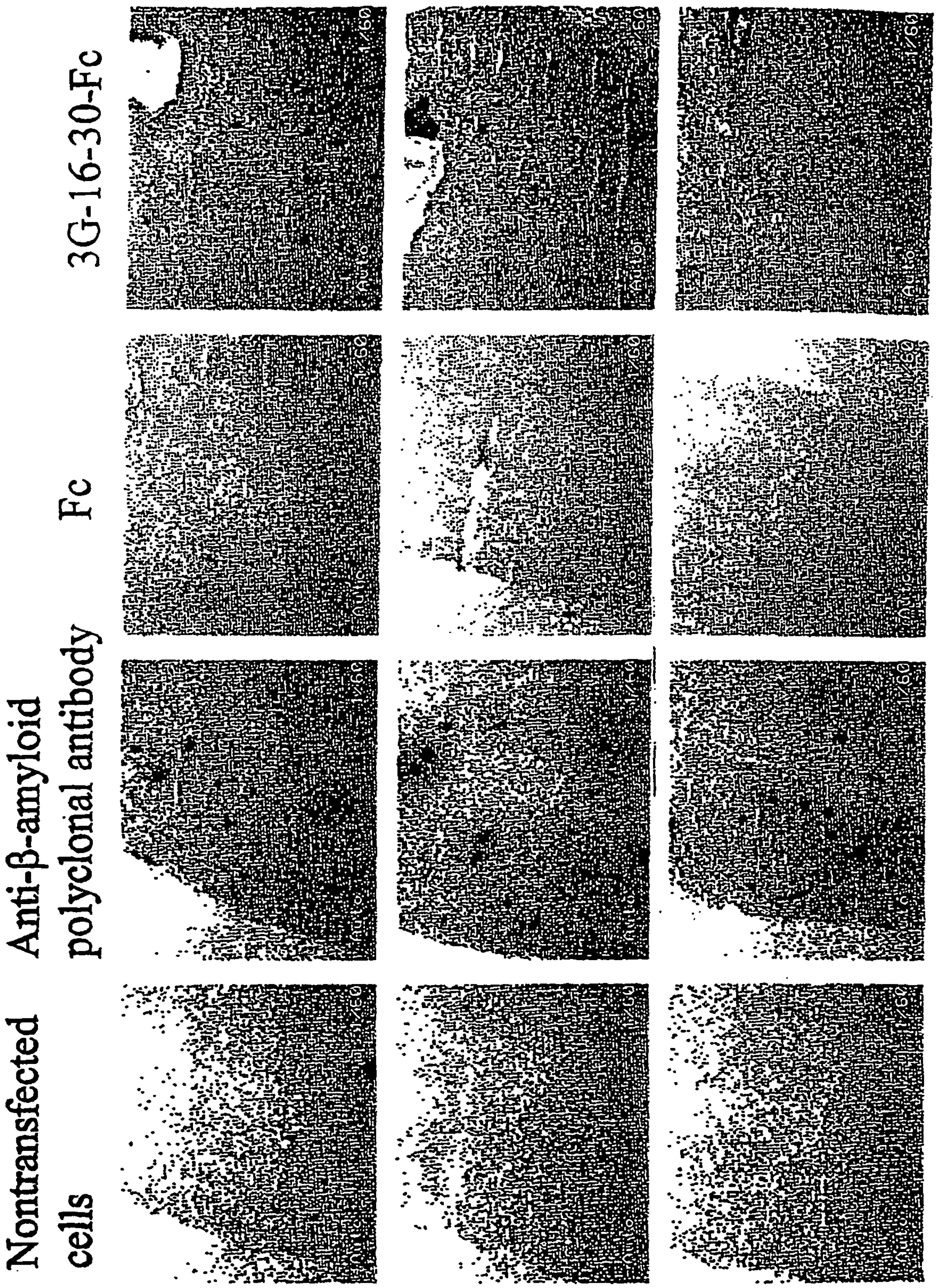
3G-16-30-Fc
Fc
Anti-β-amyloid
polyclonal antibody
Nontransfected
cells

**FIGURE 2**

BssHII-Spe-BamHI converter:

```
               BssHII          SpeI  BamHI
     DI215      CGCGCTTCAGAAGAACTAGTG
                    GAAGTCTTCTTGATCACCTAG DI216
             A  R  F  R  R  T  S  A  S
```

# FIGURE 3

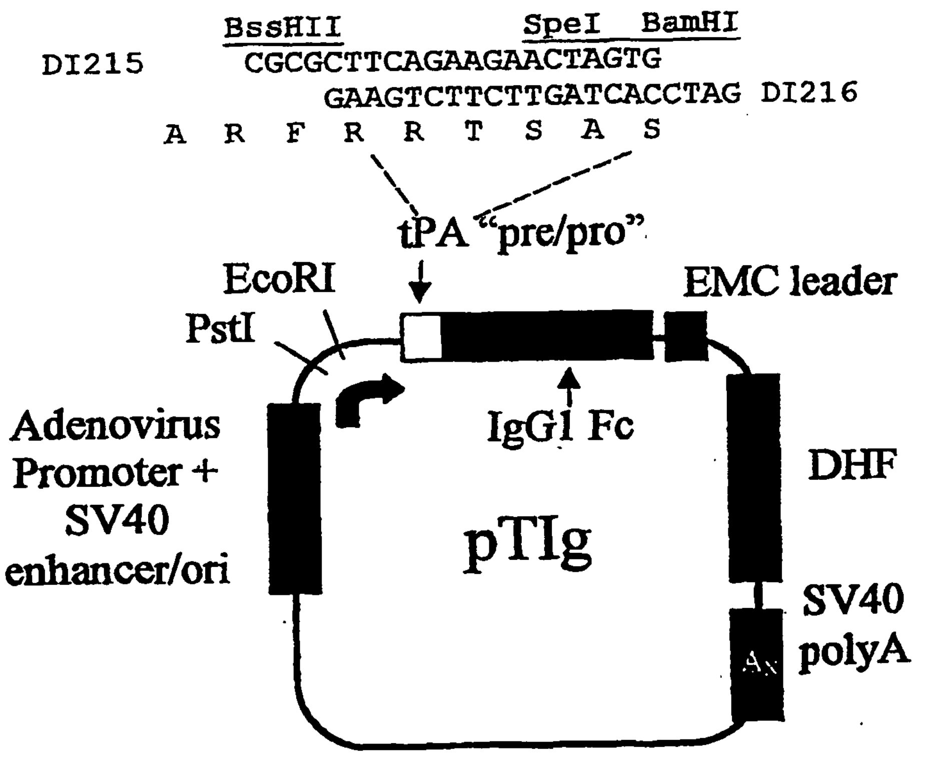


# FIGURE 4

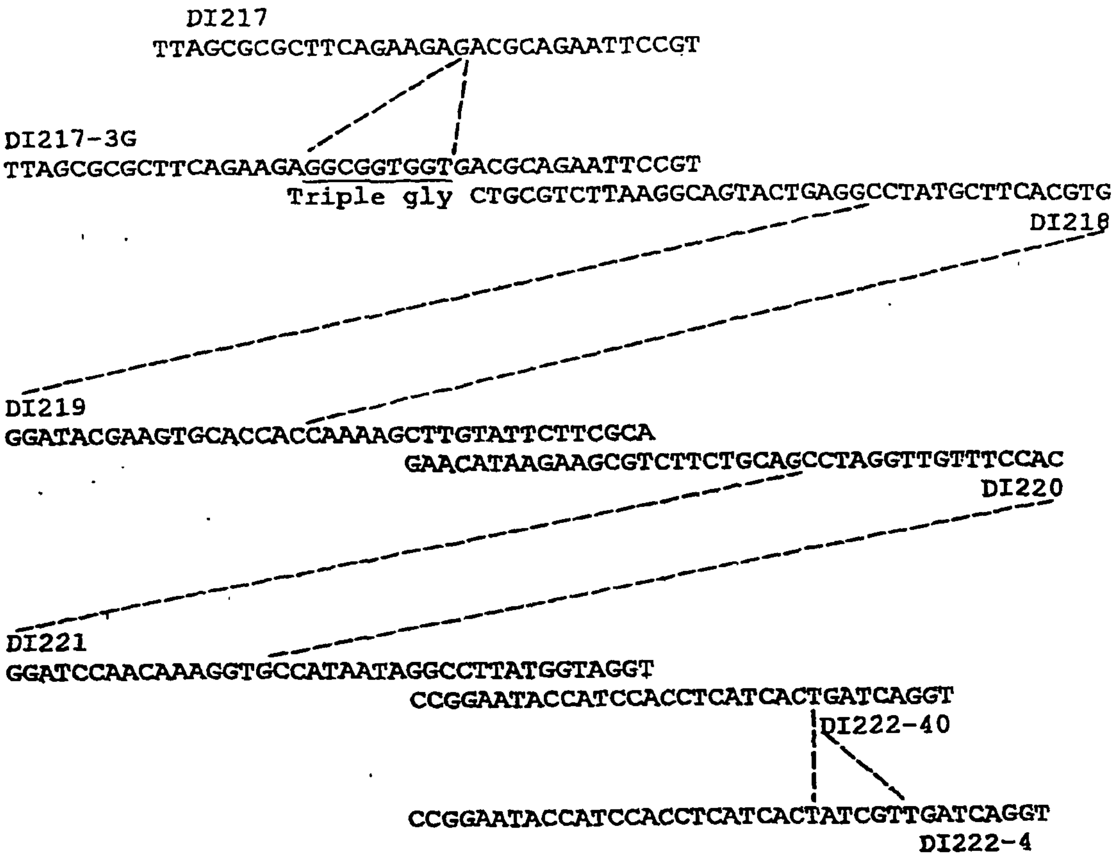
DI217
TTAGCGCGCTTCAGAAGAGACGCAGAATTCCGT
DI217-3G
TTAGCGCGCTTCAGAAGAGGCGGTGGTGACGCAGAATTCCGT
Triple gly
CTGCGTCTTAAGGCAGTACTGAGGCCTATGCTTCACGTG
DI218
DI219
GGATACGAAGTGCACCACCAAAAGCTTGTATTCTTCGCA
GAACATAAGAAGCGTCTTCTGCAGCCTAGGTTGTTTCCAC
DI220
DI221
GGATCCAACAAAGGTGCCATAATAGGCCTTATGGTAGGT
CCGGAATACCATCCACCTCATCACTGATCAGGT
DI222-40
CCGGAATACCATCCACCTCATCACTATCGTTGATCAGGT
DI222-4

# FIGURE 5

```
 1  2  3  4  5  6  7  8  9  10 11 12 13 14 15 16 17 18 19 20 21
 D  A  E  F  R  H  D  S  G  Y  E  V  H  H  Q  K  L  V  F  F  A
GACGCAGAATTCCGTCATGACTCCGGATACGAAGTGCACCACCAAAAGCTTGTATTCTTCGCA
      EcoRI    BspHI   BspEI       ApaLI        HinDIII

 22 23 24 25 26 27 28 29 30 31 32 33 34 35 36 37 38 39 40 41 42
 E  D  V  G  S  N  K  G  A  I  I  G  L  M  V  G  G  V  V  I  A
GAAGACGTCGGATCCAACAAAGGTGCCATAATAGGCCTTATGGTAGGTGGAGTAGTGATAGCA
    AatII BamHI                   StuI
```

# FIGURE 6

**β-amyloid fragments made as IgG1 $F_c$ fusions:**

- LVFFA
- LVFFL
- 16-30
- 10-25
- 1-29
- 1-40
- 1-42

All cloned N-terminal or following triple gly

- also control construct of IgG1 $F_c$ (no β-amyloid sequence)

- Overlapping complimentary oligonucleotides for pentapeptides:

```
GGGLVFFA:
         5'
DI223    CGCGCTTCAGAAGAGGCGGTGGTCTTGTATTCTTCGCAA
             GAAGTCTTCTCCGCCACCAGAACATAAGAAGCGTTGATC DI224
         BssHII                                      SpeI 5'

LVFFA:

DI225    CGCGCTTCAGAAGACTTGTATTCTTCGCAA
             GAAGTCTTCTGAACATAAGAAGCGTTGATC DI226

GGGLVFFL:

DI227    CGCGCTTCAGAAGAGGCGGTGGTCTTGTATTCTTCCTTA
             GAAGTCTTCTCCGCCACCAGAACATAAGAAGGAATGATC DI228
```

# FIGURE 7A

LVFFL:

DI229 CGCGCTTCAGAAGACTTGTATTCTTCCTTA
GAAGTCTTCTGAACATAAGAAGGAATGATC DI230

- PCR primers for longer fragments

β-amyloid 1-29 oligos

For 5′ use oligos 217 and 217-3G

DI-231 TGGACTAGTACCTTTGTTGGATCCGAC

β-amyloid 10-25 oligos

DI-232 TTAGCGCGCTTCAGAAGATACGAAGTGCACCACCAA

DI-232-3G
TTAGCGCGCTTCAGAAGAGGCGGTGGTTACGAAGTGCACCACCAA

DI-233 TGGACTAGTTCCGACGTCTTCTGCGAA

β-amyloid 16-30 oligos

DI-234 TTAGCGCGCTTCAGAAGAAAGCTTGTATTCTTCGCA

DI-234-3G
TTAGCGCGCTTCAGAAGAGGCGGTGGTAAGCTTGTATTCTTCGCA

DI-235 TGGACTAGTGGCACCTTTGTTGGATCC

**FIGURE 7B**

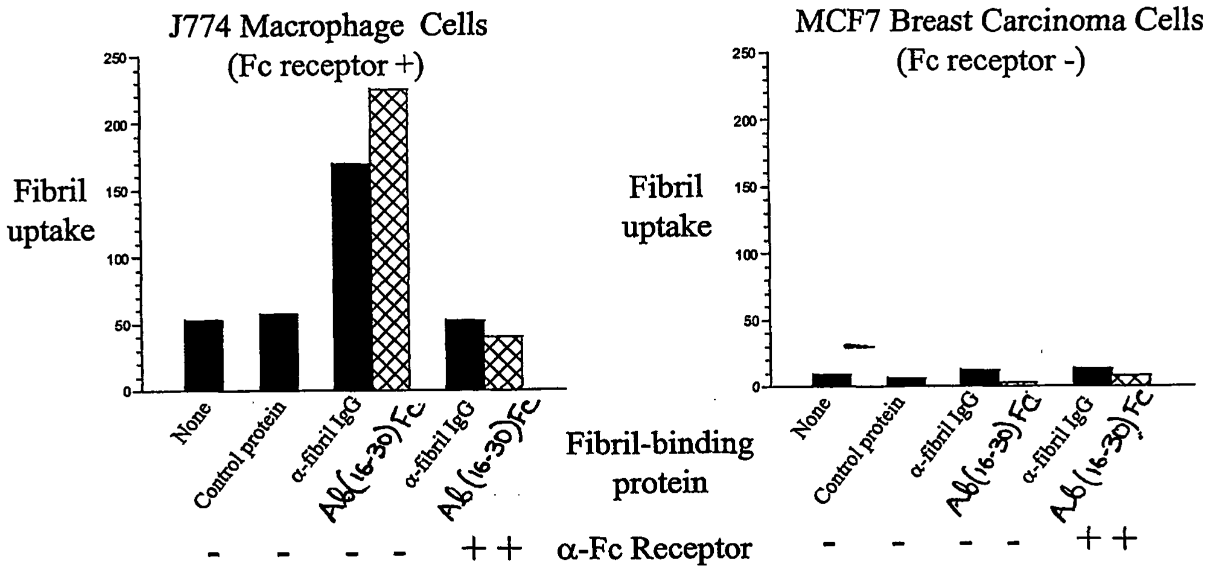

J774 Macrophage Cells
(Fc receptor +)
MCF7 Breast Carcinoma Cells
(Fc receptor -)
Fibril uptake
250
200
150
100
50
0
None
Control protein
α-fibril IgG
Aβ(16-30)Fc
α-fibril IgG
Aβ(16-30)Fc
Fibril-binding protein
- - - - + +
α-Fc Receptor
- - - - + +


# FIGURE 8

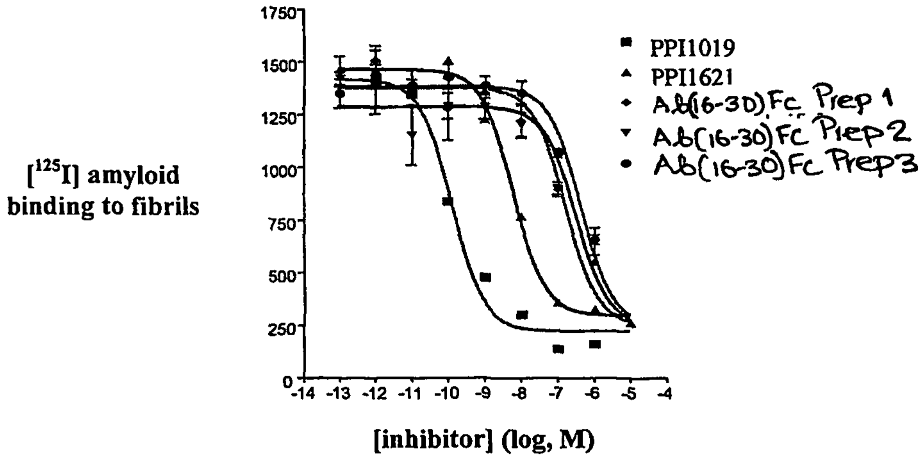
[125I] amyloid binding to fibrils
1750
1500
1250
1000
750
500
250
0
-14 -13 -12 -11 -10 -9 -8 -7 -6 -5 -4
[inhibitor] (log, M)
PPI1019
PPI1621
Aß(16-30)Fc Prep 1
Aß(16-30)Fc Prep 2
Aß(16-30)Fc Prep3

# FIGURE 9

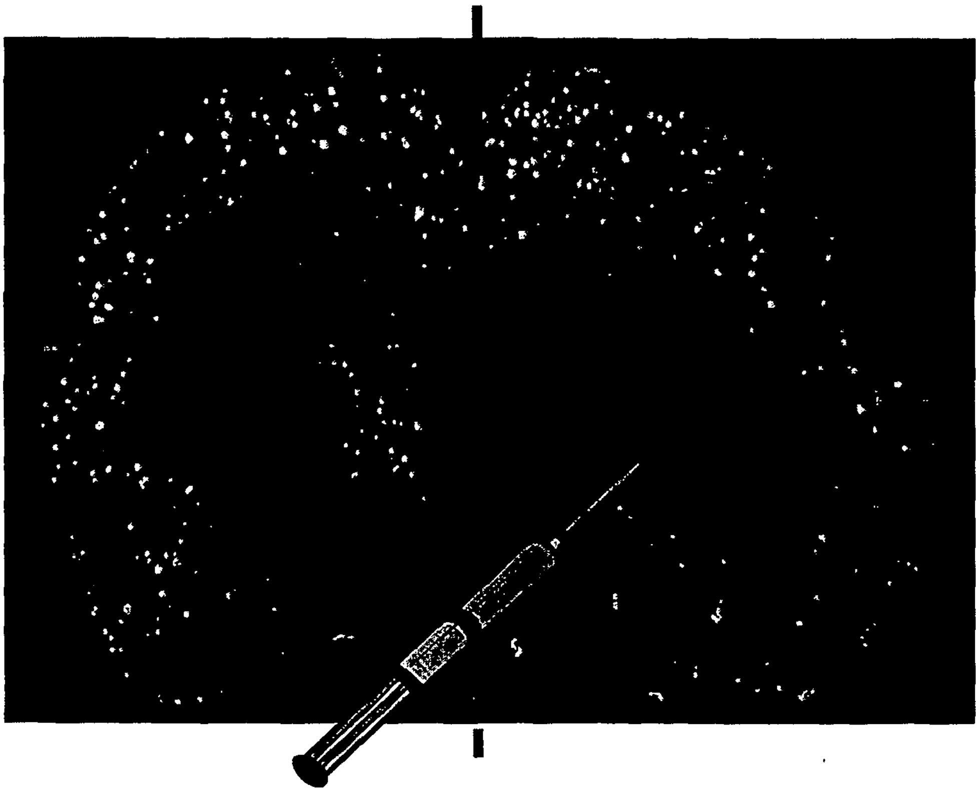

FIGURE10

ATGGATGCAATGAAGAGAGGGCTCTGCTGTGTGCTGCTGCTGTGTGGAGC
AGTCTTCGTTAAGCTTGTATTCTTCGCAGAAGACGTCGGATCGAACAAAG
GTGCCGAGCCCAAATCTTGTGACAAAACTCACACATGCCCACCGTGCCCA
GCACCTGAACTCCTGGGGGGACCGTCAGTCTTCCTCTTCCCCCCAAAACCC
AAGGACACCCTCATGATATCCCGGACCCCTGAGGTCACATGCGTGGTGGT
GGACGTGAGCCACGAAGACCCTGAGGTCAAGTTCAACTGGTACGTGGACG
GCGTGGAGGTGCATAATGCCAAGACAAAGCCGCGGGAGGAGCAGTACAA
CAGCACGTACCGGGTGGTCAGCGTCCTCACCGTCCTGCACCAGGACTGGC
TGAATGGCAAGGAGTACAAGTGCAAGGTCTCCAACAAAGCCCTCCCAGCC
CCCATCGAGAAAACCATCTCCAAAGCCAAAGGGCAGCCCCGAGAACCAC
AGGTGTACACCCTGCCCCCATCCCGGGATGAGCTGACCAAGAACCAGGTC
AGCCTGACCTGCCTGGTCAAAGGCTTCTATCCCAGCGACATCGCCGTGGA
GTGGGAGAGCAATGGGCAGCCGGAGAACAACTACAAGACCACGCCTCCC
GTGCTGGACTCCGACGGCTCCTTCTTCCTCTACAGCAAGCTCACCGTGGAC
AAGAGCAGGTGGCAGCAGGGGAACGTCTTCTCATGCTCCGTGATGCATGA
GGCTCTGCACAACCACTACACGCAGAAGAGCCTCTCCCTGTCTCCGGGTA
AATGA

**FIGURE 11**

tPA ——————▶ 16-30 beta amyloid —▶ human Fc ——————▶
MDAMKRGLCCVLLLCGAVFVKLVFFAEDVGSNKGAEPKSCDKTHTCPPCPAPE

——————————————————————▶
LLGGPSVFLFPPKPKDTLMISRTPEVTCVVVDVSHEDPEVKFNWYVDGVEVHNA

——————————————————————▶
KTKPREEQYNSTYRVVSVLTVLHQDWLNGKEYKCKVSNKALPAPIEKTISKAKG

——————————————————————▶
QPREPQVYTLPPSRDELTKNQVSLTCLVKGFYPSDIAVEWESNGQPENNYKTTPP

——————————————————————▶
VLDSDGSFFLYSKLTVDKSRWQQGNVFSCSVMHEALHNHYTQKSLSLSPGK.

# FIGURE 12